# EUROPEAN PATENT SPECIFICATION

(11) **EP 0 458 846 B1**
(45) Date of publication and mention of the grant of the patent: **02.10.1996**
(21) Application number: 90903321.9
(22) Date of filing: 16.02.1990
(51) Int. Cl.: C12N 9/42, C12N 15/56

(54) **A THERMOSTABLE (1,3-1,4)-BETA-GLUCANASE**
THERMOSTABILE (1,3-1,4)-BETA-GLUCANASE
(1,3-1,4)-BETA-GLUCANASE THERMOSTABLE

(30) Priority: 16.02.1989 DD 325800; 04.08.1989 DK 3848/89
(43) Date of publication of application: 04.12.1991
(73) Proprietor: CARLSBERG A/S, 1799 Copenhagen V (DK); Akademie der Wissenschaften der DDR, D-10117 Berlin (DE)
(72) Inventor: BORRISS, Rainer,Institut für Mikrobiologie, D-O-1017 Berlin (DE); HOFEMEISTER, Jürgen; Zentralinstitut für Genetik, DDR-4325 Gatersleben (DD); THOMSEN, Karl, Kristian, DK-4200 Slagelse (DK); OLSEN, Ole, DK-2300 Copenhagen S (DK); VON WETTSTEIN, Dietrich, DK-3500 Vaerlose (DK)
(74) Representative: Andersen, Henrik Rastrup
(86) International application number: DK9000044
(87) International publication number: WO9009436

(56) References cited:
- EP-A- 0 252 666
- Appl. Microbiol. Biotechnol., Vol. 24, 1987; HIROYUKI HONDA et al: "Cloning and expression in Escherichia coli of a Thermoanaerobacter cellulolyticus gene coding for heat-stable B-glucanase", see page 480 - page 48; figures 4A,B.
- CHEMICAL ABSTRACTS, Volume 95, No. 17, 26 October 1981, (Columbus, Ohio, US); J. PETRE et al: "Purification and properties of an endo-B-1,4:glucanase from Clostridium thermocellum", see, Abstract 145879q, & Biochimie 1981, 63 (7), 6296-39K
- CHEMICAL ABSTRACTS, Volume 110, No. 19, 8 May 1989, (Columbus, Ohio, US); O. TIKHOMIROV et al: "Endo-1,4-B-glucanases of the anaerobic bacterium Clostridium thermocellum st. No. 3 with high heat stability", see Abstract 168879g, & Prikl. Biokhim. Mikrobiol. 1989, 25(1), 48- 55K.
- CHEMICAL ABSTRACTS, Volume 108, No. 25, 20 June 1988, (Columbus, Ohio, US); W. H. SCHWARZ et al: "Isolation of a Clostridium themocellum gene encoding a thermostable B-1,3-glucanase (laminarinase)", see, Abstract 217067k, & Biotechnol. Lett. 1988, 10(4), 225-230K.
- CHEMICAL ABSTRACTS, Volume 109, No. 7, 15 August 1988, (Columbus, Ohio, US); A. A. KLYOSOV et al: "A Thermostable endo-1,4-B-glucanase from My=celiophthora thermophia", see Abstract 50624w, & Biotechnol. Lett. 1988, 10(5), 351-354K.
- CHEMICAL ABSTRACTS, Volume 107, No. 3, 20 July 1987, (Columbus, Ohio, US); J. HOFEMEISTER et al: "The B-glucanase gene from Bacillus amyloliquefaciens shows extensive homology with that of Bacillus subtilis", see, Abstract 18601j, & Gene 1986, 49(2), 177- 187K.

## Description

### FIELD OF INVENTION

The present invention relates to novel thermostable (1,3-1,4)-β-glucanases, the use of novel thermostable (1,3-1,4)-β-glucanases, DNA fragments encoding such glucanases, organisms expressing the DNA fragments and a method for producing the thermostable (1,3-1,4)-β-glucanases.

### TECHNICAL BACKGROUND

(1,3-1,4)-β-glucanases are used in the manufacture of different food products and animal feed and as subsidiary materials in biological research when it is necessary to cleave the β-glycosidic linkages in (1,3-1,4)-β-glucans. Especially in the brewing industry the use of such glucan hydrolyzing enzymes permits the application of larger proportions of raw grain in substitution for the use of malt, without this causing any trouble in the filtration due to high viscosity of the mash which may be caused by an increased amount of glucan compounds.

The mixed linked (1,3-1,4)-β-glucans constitute the major part of the endosperm cell walls of cereals like oat and barley. They may cause severe problems in the brewing industry such as reduced yield of extract and lowered rates of wort separation or beer filtration. Remaining β-glucans in the finished beer may lead to the formation of hazes and gelatinous precipitates (Godfrey, 1983). Barley (1,3-1,4)-β-glucanases (EC 3.2.1.73) are synthesized in the scutellum and the aleurone layer during the early stages of germination of seeds (McFadden et al., 1988). However, a large proportion of the malt β-glucanase is irreversibly heat inactivated during kilning and the remaining activity is rapidly destroyed during mashing (Loi et al., 1987).

It has long been known that the viscosity of the wort can be reduced by using β-glucanases from mesophilic *Bacillus* strains, e.g. from *Bacillus amyloliquefaciens* or *Bacillus subtilis.* A serious disadvantage with the known glucanases is their temperature sensitivity, which implies that they are only effective during the early phase of the mashing process. Later on when temperatures are above 65°C their activity is reduced substantially.

In an attempt to obtain a more thermostable glucanase, the gene from *Bacillus macerans* encoding glucanase was introduced into *Bacillus subtilis* in order to express the gene in this organism (DD Patent Application WP C12N/315 706 1). However, at 70°C this glucanase is also rapidly and irreversibly denatured. Another drawback to the known glucanases in relation to the brewing process is that these glucanases do not exert their full activity in the pH range from 4 to 5 which is the normal condition during mashing. For example the activity of the *Bacillus* β-glucanase at pH 4.6 is only 20% of that between 6 and 7. Furthermore, the stability is reduced when the glucanase is incubated at pH 4.

The best characterized bacterial (1,3-1,4)-β-glucanases are those from *Bacillus subtilis* and *B. amyloliquefaciens* where the genes encoding the enzymes have been cloned and sequenced (Boriss et al., 1985; Cantwell and McConnell, 1983: Hofemeister et al., 1986; Murphy et al., 1984). It has recently been shown that the β-glucanase from *B. macerans* is more thermostable than the *B. subtilis* and *B. amyloliquefaciens* enzymes (Borriss, 1981; Borriss and Schroeder, 1981). However, at temperatures exceeding 65°C and at pH values of 4.5 to 5.5, which is typical for industrial mashing, the *B. macerans* β-glucanase is rapidly inactivated. The *B. macerans* β-glucanase gene has been cloned (Borriss et al., 1988) and its nucleotide sequence determined (Borriss et al., in prep.). Comparison of the derived amino acid sequence of *B. macerans* β-glucanase with the derived sequences of *B. subtilis* and *B. amyloliquefaciens* β-glucanases reveals an overall homology of 70%.

During recent years a number of attempts have been made to construct improved forms of existing, biologically active proteins to make them better suited for industrial processes and to widen their range of application. Much interest has been focused on increasing the thermostability of enzymes. It has been proposed that the thermostability of enzymes may be enhanced by single amino acid substitutions that decrease the entropy of unfolding (Matthews et al., 1987). Several tentative rules for increasing the thermostability of proteins have been established (Argos et al., 1979; Imanaka et al., 1986; Querol and Parilla, 1987) but precise predictions for changes of function as a consequence of changes in structure remain elusive.

Several researchers have conducted experiments with *in vitro* recombination of homologous genes giving rise to hybrid proteins retaining the biological activity of the parental molecules. Streuli et al. (1981) as well as Weck and coworkers (1981) constructed hybrid human leukocyte interferon genes. Some of the hybrid interferons extended the host cell range for protection against Vesicular Stomatitis and Encephalomyocarditis virus. Thus the AD hybrids combining portions of interferons A and D elicited significantly higher antiviral activities than either parental molecule in mouse L-929 cells, human He-La cells and primary rabbit kidney cells. Heat stability, pH stability and antigenic specificity were the same for the hybrid and parental interferon molecules.

Danish Patent Application 3368/87 discloses the combination of DNA sequences from the *Bacillus licheniformis* and *Bacillus amyloliquefaciens* α-amylase genes in order to obtain a chimeric α-amylase enzyme for the liquefaction of starch, which did not have a negative effect on the maximum percentage by weight of dextrose obtainable by saccharification with a glycoamylase. This negative effect was reduced with the chimeric α-amylase and the thermostable properties were retained in comparison with the parent enzymes.

### DISCLOSURE OF THE INVENTION

The present invention relates to a thermostable (1,3-1,4)-β-glucanase which retains at least 50% of its activity after 10 minutes, preferably 15 minutes, more preferably 18 minutes of incubation in 10mM CaCl₂, 40mM Na-acetate at pH 6.0 and 70°C, the incubated solution having a concentration range from 0.3 to 1 mg (1,3-1,4)-β-glucanase per ml, the activity of the (1,3-1,4)-β-glucanase being understood as the ability of the enzyme to hydrolyze β-glycosidic linkages in (1,3-1,4)-β-glucans.

In the present context, the term "thermostable" relates to the ability of an enzyme to resist denaturing and to retain the enzymatic activity at high temperatures for a period of time sufficient for the enzyme to convert its substrate into the reaction products, in the present case to cleave β-glycosidic linkages in (1,3-1,4)-β-glucans to obtain reducing sugars. By high temperatures is meant temperatures above 60°C.

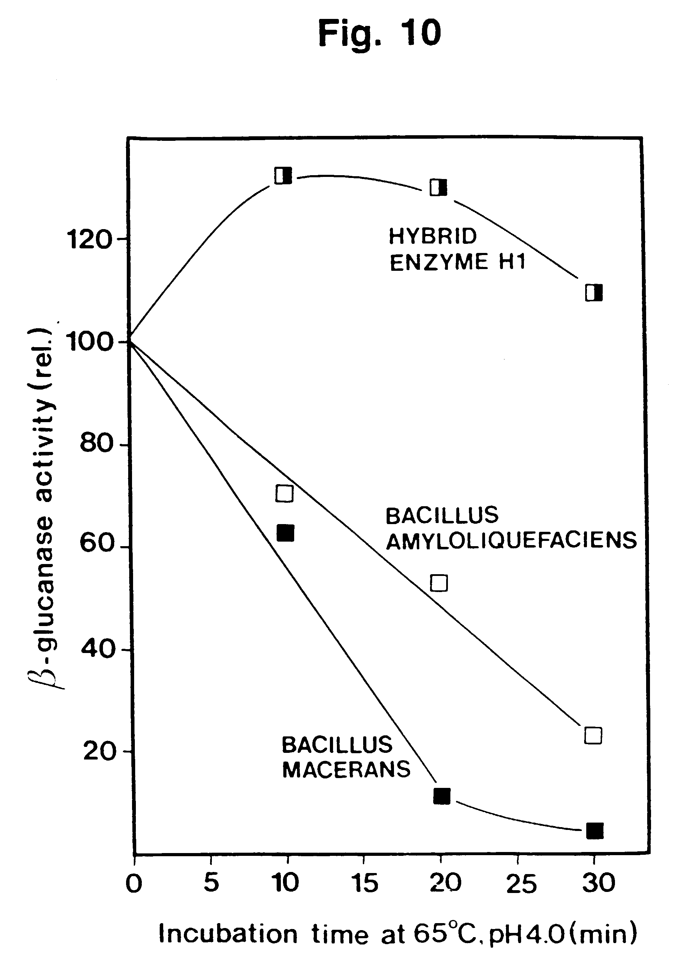

In another aspect, the present invention relates to a thermostable (1,3-1,4)-β-glucanase which after 10 minutes of incubation in crude cell extracts at 65°C and pH 4.0 has a relative β-glucanase activity of at least 100%, preferably at least 110%, more preferably at least 120%. An example of such a characteristic behaviour of the (1,3-1,4)-β-glucanase of the invention is shown in Fig. 10, where the relative β-glucanase activity of hybrid enzyme H1 is compared to the relative β-glucanase activities of *B. amyloliquefaciens* and *B. macerans* β-glucanases. From Fig. 10 it is clear that the relative β-glucanase activity of the H1 enzyme is about 130% after 10 minutes of incubation.

The present inventors have constructed hybrid genes encoding recombinant Bacillus(1,3-1,4)-β-glucanases, which are more thermostable than any (1,3-1,4)-β-glucanases known until now. The hybrid genes were constructed by reciprocal exchanges of the amino-terminal and carboxy-terminal parts of the β-glucanase encoding genes from *Bacillus amyloliquefaciens* and *Bacillus macerans* by using a common EcoRV endonuclease restriction site in the middle of the (1,3-1,4)-β-glucanase gene in *B. amyloliquefaciens* and *B. macerans,* respectively, as a fusion point or by construction of hybrid fusion genes using the polymerase chain reaction (PCR) according to Yon & Fried *(Nucleic Acid Research,* 1989, 17, 4895) and Horton et al. (Gene*,* 1989, 77, 61-68.

The β-glucanase hybrid enzyme 1 (H1) contains the 107 amino-terminal residues of mature *B. amyloliquefaciens* β-glucanase and the 107 carboxyl-terminal amino acid residues of *B. macerans* β-glucanase. A reciprocal β-glucanase hybrid enzyme 2 (H2) consists of the 105 amino-terminal parts from the *B. macerans* enzyme and the carboxyl-terminal 107 amino acids from *B. amyloliquefaciens.* The biochemical properties of the two hybrid enzymes differ significantly from each other as well as from both parental β-glucanases.

The hybrid enzymes H3, H4, H5, and H6 were constructed by using PCR. H3 contains the 16 amino-terminal amino acid residues of mature *B. amyloliquefaciens* (1,3-1,4)-β-glucanase and the 198 carboxy-terminal amino acid residues of *B. macerans* β-glucanase; H4 contains the 36 amino-terminal amino acid residues of mature *B. amyloliquefaciens* β-glucanase and the 178 carboxy-terminal residues of *B. macerans;* H5 contains the 78 amino-terminal amino acid residues of mature *B. amyloliquefaciens* (1,3-1,4)-β-glucanase and the 136 carboxy-terminal amino acid residues of mature *B. macerans* β-glucanase; and H6 contains the 152 amino-terminal residues of mature *B. amyloliquefaciens* β-glucanase and the 62 carboxy-terminal amino acid residues of mature *B. macerans* (1,3-1,4-)-β-glucanase.

Compared to the parental enzymes, the hybrid proteins exhibit novel biochemical properties such as different pH-optima, thermostability and differences in pH tolerance. The H1 protein is of special interest for the brewing industry since in this protein the tolerance to lower pH and a low pH optimum of enzymatic activity has been combined with a thermostability exceeding that of the *B. macerans* β-glucanase at high pH. The pH optimum and especially the pH tolerance has been shifted to more acidic conditions and the thermostability surpasses that of both parental enzymes over the entire tested pH range.

However, the properties of the thermostable (1,3-1,4)-β-glucanase of the invention also makes it interesting to use the enzyme for different purposes where it is desirable to obtain (1,3-1,4)-β-glucanase enzymatic activity at high temperature and possibly at low pH, e.g. in the manufacturing of coffee surrogates or feed pellets, especially for use in feeding poultry. Poultry are not able to degrade β-glucans in the feed and pelleted feed containing high amounts of β-glucans causes reduced feed/weight gain ratios and also digestive disorders.

Therefore, it is advantageous to degrade the β-glucans in the feed by adding (1,3-1,4)-β-glucanases to the feed. Production of the feed pellets takes place at high temperatures meaning that non-thermostable (1,3-1,4)-β-glucanases are rapidly degraded. However, this problem can now be solved by using the thermostable (1,3-1,4)-β-glucanase of the invention in the production.

Different strategies may be followed in the construction of a hybrid gene encoding a thermostable (1,3-1,4)-β-glucanase. Other restriction sites in the (1,3-1,4)-β-glucanase gene may be used; the nucleotide sequence of the (1,3-1,4)-β-glucanase gene may be digested by nuclease followed by the introduction of synthetic nucleotide sequences containing useful endonuclease restriction sites; or a total or partially synthetic gene may be constructed. Also, (1,3-1,4)-β-glucanase genes originating from other organisms than *B. amyloliquefaciens* and *B. macerans* may be used in order to obtain a hybrid gene encoding a thermostable (1,3-1,4)-β-glucanase.

Thus, in another aspect the present invention relates to a thermostable hybrid (1,3-1,4)-β-glucanase comprising an amino acid sequence with the formula

A - M

where A is a polypeptide consisting of 5-200 amino acids which are at least 75%, preferably at least 85%, more preferably at least 90% identical to the amino acid residues of the N-terminal part of the *Bacillus amyloliquefaciens* or *Bacillus macerans* (1,3-1,4)-β-glucanase as given in Table I and M is a polypeptide consisting of 5 to 200 amino acids which are at least 75%, preferably at least 85%, more preferably at least 90% identical to the amino acid residues of the carboxy-terminal part of the *Bacillus macerans* or *Bacillus amyloliquefaciens* (1,3-1,4)-β-glucanase as shown in Table I.

The term "identical to" refers to a comparison of the overall composition of amino acids in two polypeptides or parts thereof. In each polypeptide the number of individual amino acid residues as well as the total number of amino acid residues is determined. Then, the degree of identity is given by the ratio of the number of identical amino acid residues in the two polypeptides relative to the total number of amino acid residues in the polypeptide to be compared. Thus, in the present context, the degree of identity is determined as the percentage of the amino acids in the (1,3-1,4)-β-glucanase of the invention or part thereof, which are identical to the amino acids in another polypeptide or part thereof, in the present case the amino-terminal part of *B. amyloliquefaciens* or *B. macerans* (1,3-1,4)-β-glucanase as given in Table I and the carboxy-terminal part of *B. macerans* or *B. amyloliquefaciens* (1,3-1,4)-β-glucanase as given in Table I, relative to the total number of amino acid residues in the compared part of (1,3-1,4)-β-glucanase. It should be recognized that identity is not dependent on the position of amino acids in the polypeptides and is absolute in the sense that identity is not related to possible homologous functions of two amino acids with different chemical formula.

In the present context, the amino-terminal part of a polypeptide is understood as that part of the polypeptide in question or a portion thereof, measured in number of amino acids, which possesses a free α-NH₂ group at its end. The carboxy-terminal part of a polypeptide is understood as that part of the polypeptide in question or a portion thereof, measured in number of amino acids, which possesses a free α-COOH group at its end.

In one aspect of the invention, a signal peptide enabling the transport out of the cell may be linked to the amino-terminal end of the thermostable (1,3-1,4)-β-glucanase. The signal peptide may be one encoded by the relevant part of native (1,3-1,4)-β-glucanase genes in bacteria or other microorganisms such as yeast and fungi; it may be of synthetic origin, or a combination of these sources. The choice of signal peptide depends on the microorganism used for expression of the thermostable (1,3-1,4)-β-glucanase. Preferably, the signal peptide is a signal peptide homologous to the microorganism in question; e.g. when a yeast is used for expression the signal peptide should be homologous to yeast in order to enable transport of the thermostable (1,3-1,4)-β-glucanase out of the yeast cell. A suitable yeast signal peptide is the signal peptide from invertase which is known to be one of the few secreted proteins in yeast such as *Saccharomyces* species. However, also other signal peptides from yeast such as the signal peptide for α-factor and acid phosphatase may be used for transporting the thermostable (1,3-1,4)-β-glucanase out of the yeast cell.

In a preferred embodiment of the present invention the signal peptide is at least 75%, preferably at least 85%, more preferably at least 90% identical to the signal peptide of *Bacillus amyloliquefaciens* at the amino acid level as defined above.

In a still further aspect the invention relates to a thermostable (1,3-1,4)-β-glucanase which comprises the following amino acid sequence: 
Gln-Thr-Gly-Gly-Ser-Phe-Phe-Glu-Pro-Phe-Asn-Ser-Tyr-Asn-Ser-Gly-Leu-
Trp-Gln-Lys-Ala-Asp-Gly-Tyr-Ser-Asn-Gly-Asp-Met-Phe-Asn-Cys-Thr-Trp-
Arg-Ala-Asn-Asn-Val-Ser-Met-Thr-Ser-Leu-Gly-Glu-Met-Arg-Leu-Ala-Leu-
Thr-Ser-Pro-Ser-Tyr-Asn-Lys-Phe-Asp-Cys-Gly-Glu-Asn-Arg-Ser-Val-Gln-
Thr-Tyr-Gly-Tyr-Gly-Leu-Tyr-Glu-Val-Arg-Met-Lys-Pro-Ala-Lys-Asn-Thr-
Gly-Ile-Val-Ser-Ser-Phe-Phe-Thr-Tyr-Thr-Gly-Pro-Thr-Glu-Gly-Thr-Pro-
Trp-Asp-Glu-Ile-Asp-Ile-Glu-Phe-Leu-Gly-Lys-Asp-Thr-Thr-Lys-Val-Gln-
Phe-Asn-Tyr-Tyr-Thr-Asn-Gly-Val-Gly-Gly-His-Glu-Lys-Val-Ile-Ser-Leu-
Gly-Phe-Asp-Ala-Ser-Lys-Gly-Phe-His-Thr-Tyr-Ala-Phe-Asp-Trp-Gln-Pro-
Gly-Tyr-Ile-Lys-Trp-Tyr-Val-Asp-Gly-Val-Leu-Lys-His-Thr-Ala-Thr-Ala-
Asn-Ile-Pro-Ser-Thr-Pro-Gly-Lys-Ile-Met-Met-Asn-Leu-Trp-Asn-Gly-Thr-
Gly-Val-Asp-Asp-Trp-Leu-Gly-Ser-Tyr-Asn-Gly-Ala-Asn-Pro-Leu-Tyr-Ala-
Glu-Tyr-Asp-Trp-Val-Lys-Tyr-Thr-Ser-Asn
 or analogues thereof.

The invention also relates to a thermostable (1,3-1,4)-β-glucanase with the following amino acid sequence: 
Met-Lys-Arg-Val-Leu-Leu-Ile-Leu-Val-Thr-Gly-Leu-Phe-Met-Ser-Leu-Cys-
Gly-Ile-Thr-Ser-Ser-Val-Ser-Ala-Gln-Thr-Gly-Gly-Ser-Phe-Phe-Glu-Pro-
Phe-Asn-Ser-Tyr-Asn-Ser-Gly-Leu-Trp-Gln-Lys-Ala-Asp-Gly-Tyr-Ser-Asn-
Gly-Asp-Met-Phe-Asn-Cys-Thr-Trp-Arg-Ala-Asn-Asn-Val-Ser-Met-Thr-Ser-
Leu-Gly-Glu-Met-Arg-Leu-Ala-Leu-Thr-Ser-Pro-Ser-Tyr-Asn-Lys-Phe-Asp-
 
Cys-Gly-Glu-Asn-Arg-Ser-Val-Gln-Thr-Tyr-Gly-Tyr-Gly-Leu-Tyr-Glu-Val-
Arg-Met-Lys-Pro-Ala-Lys-Asn-Thr-Gly-Ile-Val-Ser-Ser-Phe-Phe-Thr-Tyr-
Thr-Gly-Pro-Thr-Glu-Gly-Thr-Pro-Trp-Asp-Glu-Ile-Asp-Ile-Glu-Phe-Leu-
Gly-Lys-Asp-Thr-Thr-Lys-Val-Gln-Phe-Asn-Tyr-Tyr-Thr-Asn-Gly-Val-Gly-
Gly-His-Glu-Lys-Val-Ile-Ser-Leu-Gly-Phe-Asp-Ala-Ser-Lys-Gly-Phe-His-
Thr-Tyr-Ala-Phe-Asp-Trp-Gln-Pro-Gly-Tyr-Ile-Lys-Trp-Tyr-Val-Asp-Gly-
Val-Leu-Lys-His-Thr-Ala-Thr-Ala-Asn-Ile-Pro-Ser-Thr-Pro-Gly-Lys-Ile-
Met-Met-Asn-Leu-Trp-Asn-Gly-Thr-Gly-Val-Asp-Asp-Trp-Leu-Gly-Ser-Tyr-
Asn-Gly-Ala-Asn-Pro-Leu-Tyr-Ala-Glu-Tyr-Asp-Trp-Val-Lys-Tyr-Thr-Ser-
Asn
 or analogues thereof.

The generally accepted abbreviation codes for amino acids are given in the following table:

| Amino acid | Abbreviation |
|---|---|
| Alanine | Ala |
| Arginine | Arg |
| Asparagine | Asn |
| Aspartic acid | Asp |
| Asparagine or aspartic acid | Asx |
| Cysteine | Cys |
| Glutamine | Gln |
| Glutamic acid | Glu |
| Glutamine or glutamic acid | Glx |
| Glycine | Gly |
| Histidine | His |
| Isoleucine | Ile |
| Leucine | Leu |
| Lysine | Lys |
| Methionine | Met |
| Phenylalanine | Phe |
| Proline | Pro |
| Serine | Ser |
| Threonine | Thr |
| Tryptophane | Trp |
| Tyrosine | Tyr |
| Valine | Val |

The term "analogue" is used in the present context to indicate an enzyme of a similar amino acid composition or sequence as the characteristic amino acid sequence derived from the (1,3-1,4)-β-glucanase of the invention, allowing for minor variations which do not have an adverse effect on the enzymatic activity and the thermostability of the analogue. The analogous polypeptide or protein may be derived from other microorganisms than *B. amyloliquefaciens* and *B. macerans* or may be partially or completely of synthetic origin.

The amino acids of the (1,3-1,4)-β-glucanase may optionally have been modified, e.g. by chemical, enzymatic or another type of treatment, which does not effect adversely the specific activity of the (1,3-1,4)-β-glucanase and its thermostability to any substantial extent.

In a further aspect, the invention relates to a DNA fragment comprising a nucleotide sequence encoding the thermostable hybrid (1,3-1,4)-β-glucanase as described above. The DNA fragment may be used in a method of preparing the (1,3-1,4)-β-glucanase by recombinant DNA techniques. The use of the DNA fragment of the invention in the production of a recombinant (1,3-1,4)-β-glucanase (e.g. by inserting the fragment in a suitable vector, transforming a suitable host microorganism with the vector, cultivating the microorganism so as to produce the (1,3-1,4)-β-glucanase and subsequently recovering the enzyme from the microorganisms) includes a number of advantages. It is thus possible to provide large amounts of the (1,3-1,4)-β-glucanase and the enzyme produced may be isolated in a substantially pure form, free from contaminating substances.

The (1,3-1,4)-β-glucanase of the invention may also be prepared by the well-known methods of liquid or solid phase peptide synthesis utilizing the successive coupling of the individual amino acids of the polypeptide sequence or the coupling of individual amino acids forming fragments of the polypeptide sequence which are subsequently coupled so as to result in the desired polypeptide. The solid phase peptide synthesis may e.g. be performed as described by Merrifield (1963). In solid phase synthesis, the amino acid sequence is constructed by coupling an initial amino acid to a solid support and then sequentially adding the other amino acids in the sequence by peptide bonding until the desired length has been obtained. The preparation of synthetic peptides for use for diagnostic purposes may be carried out essentially as described in Shinnick (1983).

In a particular aspect, the present invention relates to a DNA fragment substantially comprising the nucleotide sequence: 
30                                                    60
GAATTCAACG AAGAATCGCT GCACTATTAT CGATTCGTCA CCCACTTAAA GTTTTTCGAC
90                                                   120
CAGCGTCTTT TTAACGGCAC ACACATGGAA AGCCAGGACG ATTTTTTACT GGAGACAGTG
150                                                  180
AAAGAAAAGT ATCATCAGGC GTATAAATGC ACGAAGAATA TCCATACCTA CATTGAGAAA
210                                                  240
GAGTATGGGC ATAAGCTCAC CAGTGACGAG CTGCTGTATT TAACGATTCA CATAGAAAGG
270                                                  300
ATAGGATTGT TACGGATAAA GCAGGCAAAA GTAGTCAAAC AAGTATAATG AAAGCGCTTT
330                                                  360
CCTCGTATTA ATTGTTTCTT CCATTCATAT CCTATCTGTC TGTGCTGATG GTAGTTTAGG
390                                                  420
TTTGTATTTT TAACAGAAGG ATTATCATTA TTTCGACCGA TGTTCCCTTT GAAAAGGATC
450                                                  480
ATGTATGATC AATAAAGAAA GCGTGTTCAA AAAAGGGGGA ATGCTAACAT GAAACGAGTG
510                                                  540
TTGCTAATTC TTGTCACCGG ATTGTTTATG AGTTTGTGTG GGATCACTTC TAGTGTTTCG
570                                                  600
GCTCAAACAG GCGGATCGTT TTTTGAACCT TTTAACAGCT ATAACTCCGG GTTATGGCAA
630                                                  660
AAAGCTGATG GTTACTCAAA TGGAGATATG TTTAACTGCA CTTGGCGTGC TAATAACGTC
690                                                  720
TCTATGACGT CATTAGGTGA AATGCGTTTG GCGCTGACAA GTCCGTCTTA TAACAAGTTT
750                                                  780
GACTGCGGGG AAAACCGCTG GGTTCAAACA TATGGCTATG GACTTTATGA AGTCAGAATG
810                                                  840
AAACCGGCTA AAAACACAGG GATTGTTTCA TCGTTCTTCA CTTATACAGG TCCAACGGAG
870                                                  900
GGGACTCCTT GGGATGAGAT TGATATCGAA TTTCTAGGAA AAGACACGAC AAAAGTCCAG
930                                                  960
TTTAACTATT ATACCAATGG GGTTGGCGGT CATGAAAAGG TTATCTCTCT TGGCTTTGAT
 
990 1020
GCATCAAAGG GCTTCCATAC CTATGCTTTC GATTGGCAGC CAGGGTATAT TAAATGGTAT
1050 1080
GTAGACGGTG TTTTGAAACA TACCGCCACC GCGAATATTC CGAGTACGCC AGGCAAAATT
1110 1140
ATGATGAATC TATGGAACGG AACCGGAGTG GATGACTGGT TAGGTTCTTA TAATGGAGCG
1170 1200
AATCCGTTGT ACGCTGAATA TGACTGGGTA AAATATACGA GCAATTAATA TGATTGCAGC
1230
TGGGCATGAG CTTTTTAGTC CACTCCAGGC ATGCAAGCTT
 or an analogue or a subsequence thereof.

Each of the nucleotides of the above sequence is represented by the abbreviations generally used, i.e.
A represents adenine
T represents thymidine
G represents guanine
C represents cytosine

In the present context, the term "analogue" is intended to designate a DNA fragment which shows one or several modifications in the nucleotide sequence, the modifications being of such a character that the modified DNA fragment is capable of encoding a hybrid (1,3-1,4)-β-glucanase having temperature stability properties as defined above. The modifications include, e.g., base substitutions which do not affect the resulting amino acid sequence encoded by the DNA fragment, substitutions of single base pairs resulting in the encoding of functionally equivalent amino acids, deletions, and additions. In the present context, the term "subsequence" designates a DNA sequence which comprises part of the DNA sequence shown above or other DNA sequences of the invention and which has retained its capability of expressing a (1,3-1,4)-β-glucanase having temperature stability properties as defined above, including subsequences which have been analogized by modifications as explained above.

The DNA fragment encoding the (1,3-1,4)-β-glucanase or a part thereof may be subjected to mutagenization, e.g. by treatment with ultraviolet radiation, ionizing radiation or a chemical mutagen such as mitomycin C, 5-bromouracil, methylmethane sulphonate, hydroxylamine, nitrogen mustard or a nitrofuran so as to alter some of the properties of the gene product expressed from the mutagenized sequence substantially without effecting the enzymatic activity and the thermostability properties of the gene product. Especially, site-directed mutagenesis or directed mutagenesis is useful in order to improve the thermostability, pH optimum for enzymatic activity and other useful properties of the (1,3-1,4)-β-glucanase.

The DNA fragment of the invention may be one which has been modified by substitution, addition, insertion or deletion of one or more nucleotides in the sequence for the purpose of establishing a sequence which, when expressed in a suitable host organism, results in the production of a (1,3-1,4)-β-glucanase having the temperature stability properties as defined above.

Also, in a still further aspect, the present invention relates to a method for producing a thermostable (1,3-1,4)-β-glucanase comprising cultivating a microorganism in which a DNA fragment as described above has been introduced in such a way that the microorganism is capable of producing the thermostable (1,3-1,4)-β-glucanase, the cultivation being performed under conditions leading to production of the thermostable (1,3-1,4)-β-glucanase and recovering the (1,3-1,4)-β-glucanase from the culture.

Suitable expression vectors for the production of (1,3-1,4)-β-glucanase or a part thereof are vectors which upon transformation of a host organism are capable of replicating in the host organism. The vector may either be one which is capable of autonomous replication, such as a plasmid, or one which is replicated with the host chromosome, such as a bacteriophage. Examples of suitable vectors which have been widely employed are pBR322 and related vectors as well as pUC vectors and the like. Examples of suitable bacteriophages include M13 and λ. Examples of self-replicating yeast vectors are those vectors carrying that part of the yeast 2 µ DNA which is responsible for autonomous replication.

The organism harbouring the vector carrying the DNA fragment of the invention or part thereof may be any organism which is capable of expressing said DNA fragment. The organism is preferably a microorganism such as a yeast or a bacterium. Yeasts such as *Saccharomyces* species possess some inherent properties which may be of great advantage in the production of extracellular proteins. Normally, yeasts only secrete very few proteins to the medium wherein they are cultured. It is, therefore, relatively easy to isolate and purify a yeast produced recombinant protein if this protein can be secreted from the yeast cell. In order to obtain secretion of the thermostable (1,3-1,4)-β-glucanase a signal peptide must be linked to the N-terminal end of the enzyme. The yeast-produced enzyme invertase is one of the few yeast proteins which are secreted from the yeast cell and it is therefore anticipated that the signal peptide from yeast invatase is suitable for enabling transport out of the yeast cell of the thermostable (1,3-1,4)-β-glucanase. However, gram-positive microorganisms as well as gram-negative bacteria may be employed as host organisms. Especially, a gram-negative bacterium such as *E. coli* is useful, but also gram-positive bacteria such as *B. subtilis* and other types of microorganisms such as fungi or other microorganisms conventionally used to produce recombinant DNA products may be used.

When a microorganism is used for expressing the DNA fragment of the invention, the cultivation conditions will typically depend on the type of microorganism employed, and a person skilled in the art will know which cultivation method to choose and how to optimize this method.

In a still further aspect the invention relates to a plant capable of expressing the DNA fragment as described above. It may be advantageous to construct a plant which is able to express in its grains and germlings a thermostable (1,3-1,4)-β-glucanase as this can eliminate the need for adding the enzyme to, e.g. the mash during the brewing process. Preferably, the plant is oat, barley, rye, wheat, rice or maize or any other plant used in the production of beer, coffee surrogates, feed or other manufacturing processes where the degradation of β-glucans by (1,3-1,4)-β-glucanases is required. A plant with an increased (1,3-1,4)-β-glucanase activity as compared to the plant in its natural form is, e.g. advantageous as a raw material for the production of beer because an increased β-glucanase activity will lead to a decreased amount of β-glucans in the wort which makes the filtration easier and improves the quality of the final product. Accordingly, the present invention relates to a genetic construct useful for producing a thermostable (1,3-1,4)-β-glucanase as defined above, i.e. a (1,3-1,4)-β-glucanase encoded by a DNA fragment as described above which construct comprises 1) a regulatory sequence functionally connected to 2) a DNA fragment as defined above encoding the (1,3-1,4)-β-glucanase, possibly including a nucleotide sequence encoding a signal peptide and 3) a transcription termination DNA sequence, functionally connected to the DNA fragment of 2).

The genetic construct useful for producing a (1,3-1,4)-β-glucanase of the invention, or a part thereof, is preferably used in the construction of a plant having an increased β-glucanase activity as compared to a plant not containing the genetic construct. However, this need not be the case since a plant expressing the DNA fragment of the invention may have a lower β-glucanase activity but a β-glucanase activity which is retained at high temperatures. When constructing a plant producing a temperature tolerant β-glucanase and possibly having an increased β-glucanase activity relative to the non-modified plant, the genetic construct should be active in a tissue or cell in which the (1,3-1,4)-β-glucanase is required for the desired activity or from which the β-glucanase may be transported into the place of activity. The genetic construct may be inserted in connection with or instead of another (1,3-1,4)-β-glucanase gene and may be inserted under the control of the regulatory sequence of a plant gene so that no additional regulatory sequence is required. However, in certain plants such as maize, rice and wheat no (1,3-1,4)-β-glucanase gene is present and it will therefore, in order to obtain expression of the inserted β-glucanase gene in the plant, be necessary to introduce regulatory sequences to control the expression of the inserted β-glucanase gene or, alternatively, employ other regulatory sequences in the plant to control expression. The introduction of DNA into a plant cell may e.g. be carried out by direct injection of DNA into naked protoplasts or by firing DNA-coated particles into the cell or protoplast.

The regulatory sequence contained in the above defined genetic constructs is preferably a plant promoter such as a constitutive or regulatable plant promoter. When the genetic construct is to be used in a genetically modified plant, the promoter is preferably a promoter active in a plant which may be the CaMV promoter, the NOS promoter or the (1,3-1,4)-β-glucanase promoter. The examples of promoters are illustrative, other sequences can fulfil the same need. The transcription termination sequence of the genetic construct is a nucleotide sequence capable of terminating the transcription of a DNA fragment of gene and providing a polyadenylation signal and is preferably derived from a plant, i.e. being a plant transcription termination sequence.

The genetic construct may further be provided with a marker which allows for the selection of the genetic construct in a plant cell into which it has been transferred. Various markers exist which may be used in plant cells, particularly markers which provide for antibiotic resistance. These markers include resistance to G418, hygromycin, bleomycin, kanamycin and gentamycin.

In recent years, considerable effort has been focused on developing useful methods for constructing novel plants or plant cells having specific and desirable properties, and a number of such methods based on recombinant DNA technology and suitable plant transformation systems are now available. It is contemplated that plants of the invention, e.g. plants having the properties described above, may be constructed by use of such methods.

The basic principle in the construction of genetically modified plants is to insert genetic information in the plant genome so as to obtain a stable maintenance of the inserted genetic material. Several techniques exist for inserting the genetic information, the two main principles being direct introduction of the genetic information and introduction of the genetic information by use of a bacterial vector system. Thus, in another aspect, the present invention relates to introduce a gene plus a genetic construct as defined above into the genome of a plant such as oat, barley, rye, wheat, rice or maize.

When plant cells with new genetic information are constructed, these cells may be grown and maintained in accordance with well-known tissue culturing methods such as culturing the cells in a suitable culture medium supplied with the necessary growth factors such as amino acids, plant hormones, vitamins etc.

As mentioned previously, it is especially advantageous to use the thermostable (1,3-1,4)-β-glucanase of the invention in the production of beer. During the mashing process, β-glucans are extracted from the malt leading to an increased viscosity of the mash. In order to reduce the amount of β-glucans in the mash and the wort, the thermostable (1,3-1,4)-β-glucanase should be added during the mashing process. In some breweries, the mashing process is carried out as a stepwise process where the temperature is gradually raised up to a maximum temperature as high as about 76°C. In other breweries, the mashing process is carried out at a fixed temperature, typically in an interval from 50 to 65°C. The (1,3-1,4)-β-glucanase present in the barley becomes inactivated at about 55°C and commercially available products such as Cereflo® (a composite enzyme product comprising a (1,3-1,4)-β-glucanase available from Novo-Nordisk A/S, Denmark) becomes inactivated at about 67°C. Due to the high thermostability and high specific activity of the (1,3-1,4)-β-glucanase of the present invention, addition of a much lower amount of this enzyme is sufficient to achieve degradation of the β-glucans in the mash. While it is necessary to add 4 mg of Cereflo® pr. kg of malt in order to degrade the β-glucans present in a typical mash, it is anticipated that a much lower amount of the thermostable (1,3-1,4)-β-glucanase of the present invention is sufficient to achieve the same result. An amount of the thermostable (1,3-1,4)-β-glucanase as low as 20 µg/kg malt or even lower is believed to be sufficient to degrade the β-glucans in a typical mash.

Thus, the invention further relates to a method of degrading (1,3-1,4)-β-glucans in a substrate, which method comprises subjecting the substrate to the action of an effective amount of a thermostable (1,3-1,4)-β-glucanase as described above for an appropriate period of time at a temperature of 65°C or higher, the amount of (1,3-1,4)-β-glucanase being at the most 200 µg, preferably at the most 100 µg, more preferably at the most 50 µg, still more preferably at the most 20 µg, and most preferably at the most 15 µg pr. kg of substrate.

The β-glucan containing substrate may be in solid or liquid form and may comprise different types of raw grains such as oat or barley, or parts and mixtures thereof. The (1,3-1,4)-β-glucanase enzyme may be added in purified form, or as part of a mixture containing other enzymes or subsidiary materials, the enzyme preferably being solubilized. Also, the enzyme may be contained in the substrate by being incorporated in the plant by genetic engineering.

The substrate comprising (1,3-1,4)-β-glucans may also be mixed with a second substrate containing a thermostable (1,3-1,4)-β-glucanase, the second substrate originating from maize, rice or wheat. Maize, rice and wheat does not naturally produce (1,3-1,4)-β-glucanase but can be changed by genetic engineering techniques to incorporate and express a gene encoding a thermostable (1,3-1,4)-β-glucanase.

The invention will now be further described with reference to the accompanying drawings and the following Examples.

### LEGEND TO FIGURES

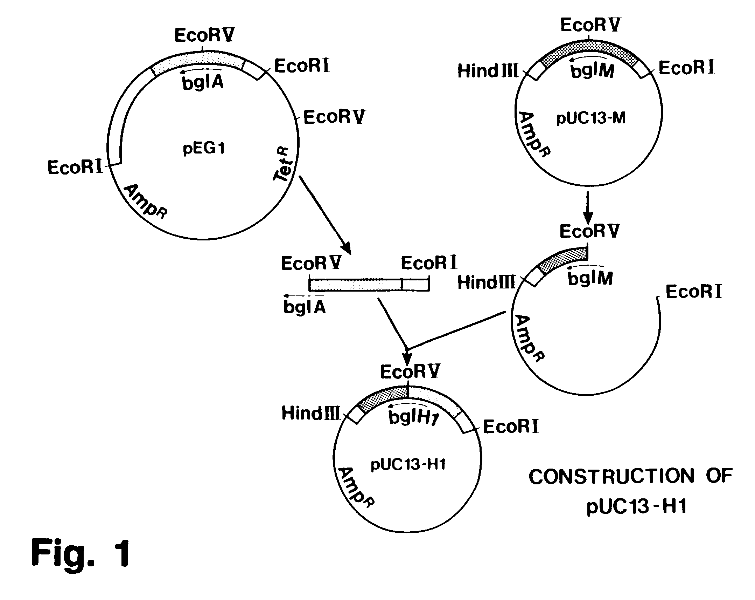

*Figure 1.* Construction of an *E. coli* expression and secretion vector containing the hybrid gene bgl-H1. bgl-A: (1,3-1,4)-β-glucanase gene from *B. amyloliquefaciens.* bgl-M: (1,3-1,4)-β-glucanase gene from *B. macerans.* For details, see Example 1.

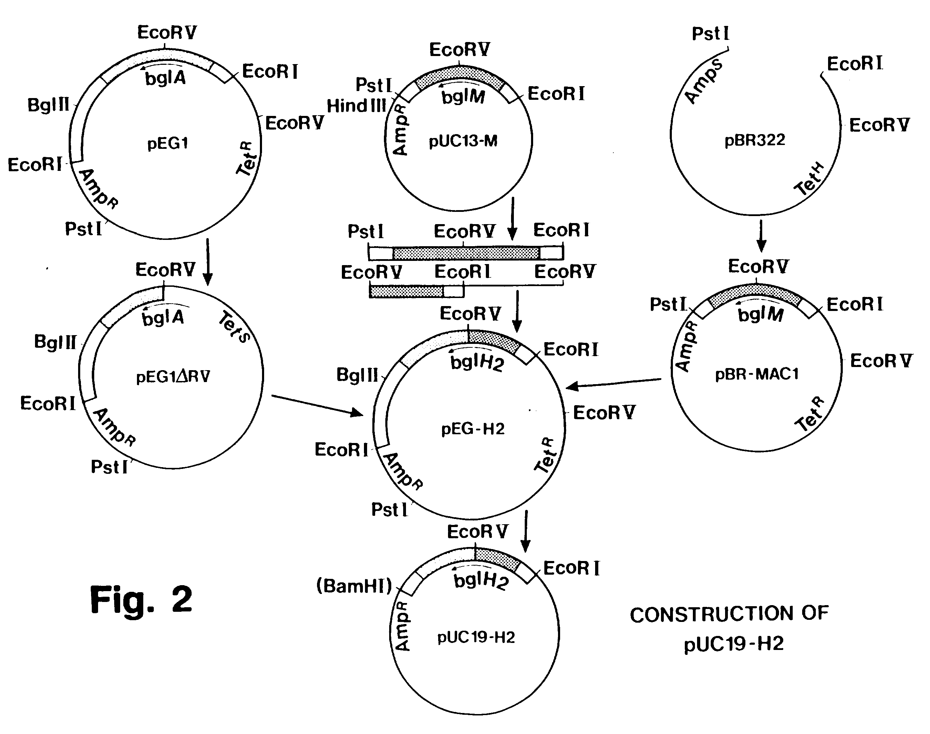

*Figure 2.* Construction of an *E. coli* expression and secretion vector containing the hybrid gene bgl-H2. bgl-A: (1,3-1,4)-β-glucanase gene from *B. amyloliquefaciens.* bgl-M: (1,3-1,4)-β-glucanase gene from *B. macerans.* For details, see Example 1.

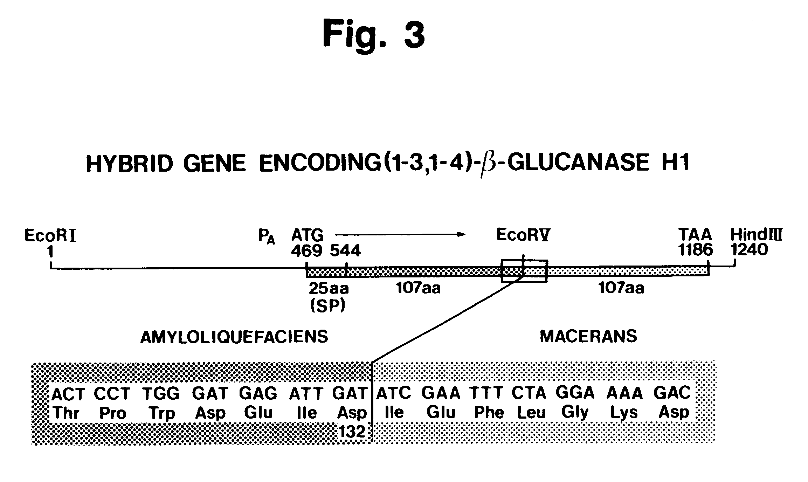

*Figure 3.* Diagram of the bgl-H1 gene and details of the fusion region. SP: signal peptide.

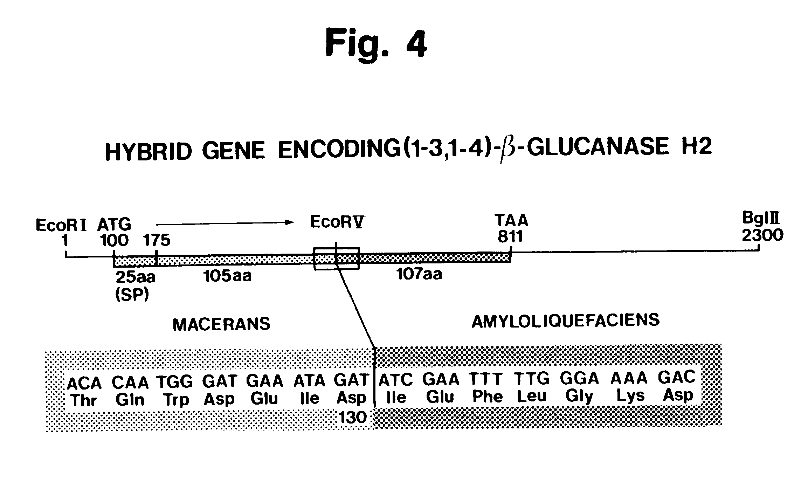

*Figure 4.* Diagram of the bgl-H2 gene and details of the fusion region. SP: signal peptide.

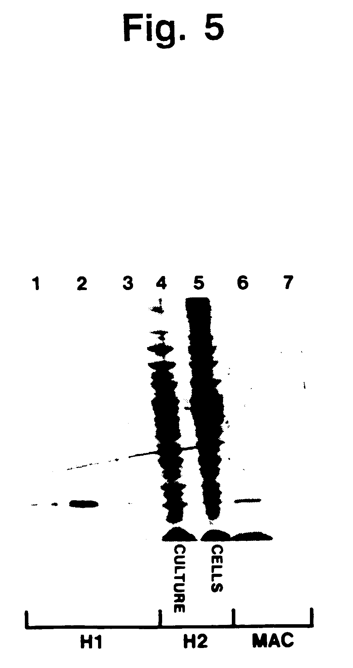

*Figure 5.* SDS-PAGE of samples containing hybrid β-glucanases and *B. macerans* β-glucanase. Lanes 1-3: 2 µg, 5 µg, and 1 µg purified β-glucanase H1. Lanes 4: sample containing 50 µg supernatant protein and lane 5: 100 µg cell extract of *E. coli* cells transformed by pUC-H2. Lane 6: 2 µg of partially purified *B. macerans* β-glucanase. Lane 7: 1 µg of purified *B. macerans* β-glucanase.

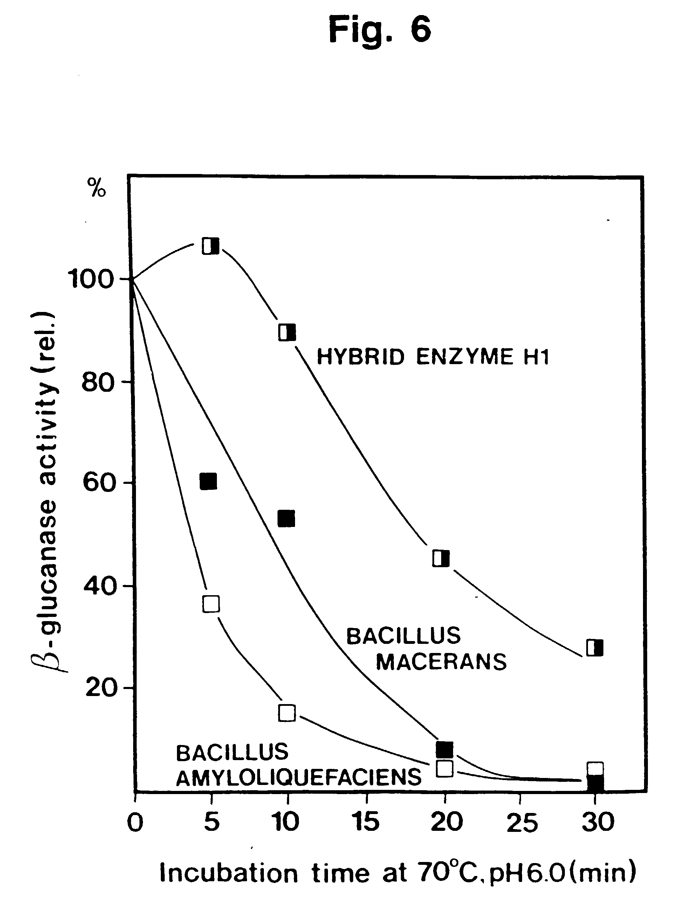

*Figure 6.* Activity of *Bacillus* hybrid β-glucanase H1 and parental enzymes in crude extracts from transgenic *E. coli* cells after incubation for various lengths of time at 70°C, pH 6.0. Activity is expressed as per cent of the activity at time 0.

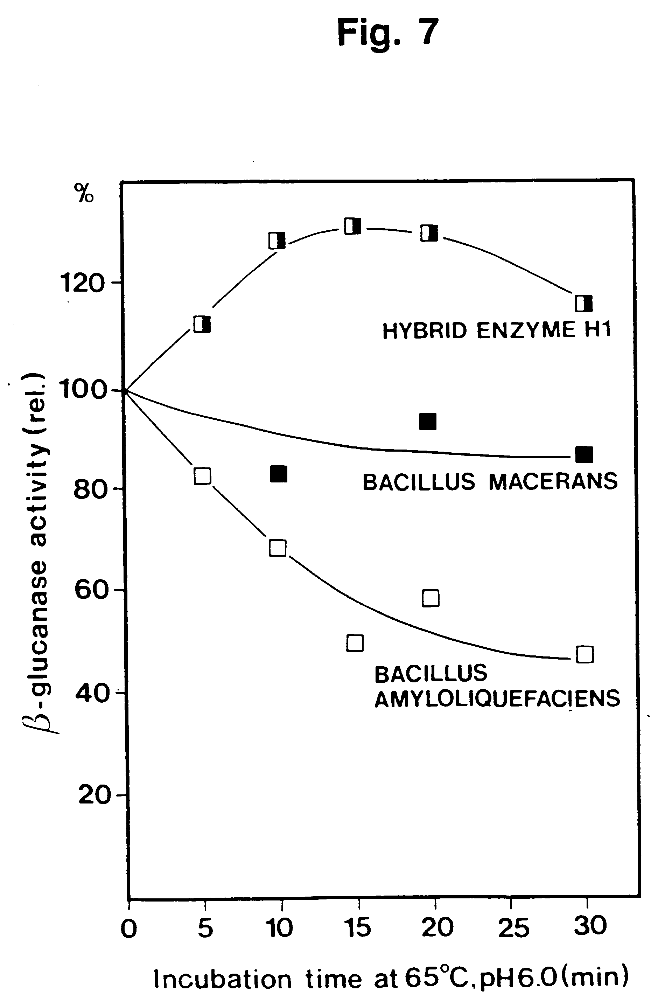

*Figure 7.* Activity of hybrid *Bacillus* β-glucanase H1 and parental enzymes in crude extracts (see Materials and Methods) from transgenic *E. coli* cells after incubation for various lengths of time at 65°C, pH 6.0. Activity is expressed as per cent of the activity at time 0.

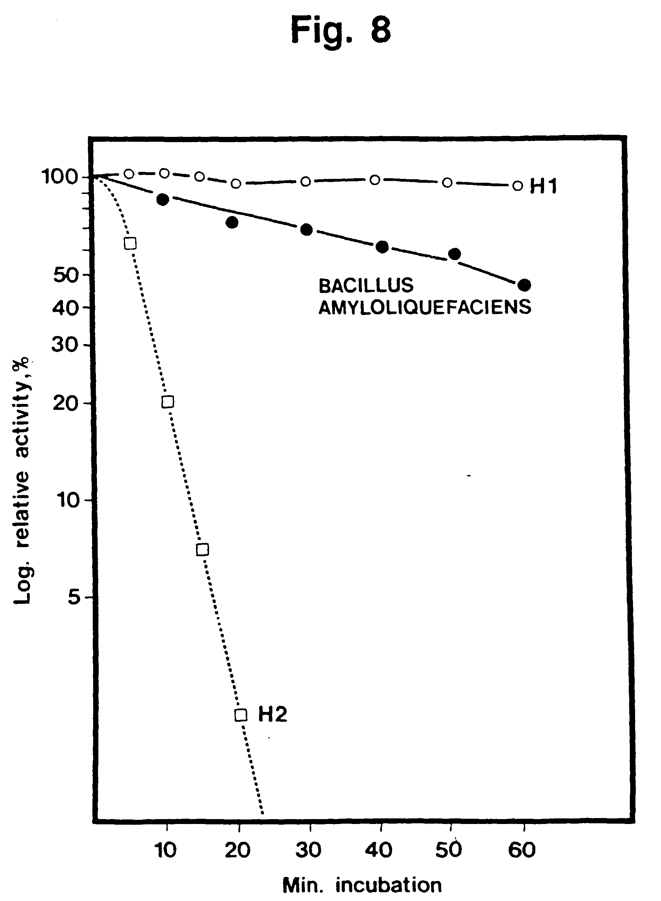

*Figure 8.* Time course of thermoinactivation of *Bacillus* hybrid β-glucanases H1 and H2 at 65°C, pH 5.5 in comparison with the β-glucanase of *B. amyloliquefaciens.* The purified amyloliquefaciens and H1-enzymes were dissolved at a concentration of 1 µg•ml"¹ in 40 mM Na-acetate, pH 5.5, 10 mM CaCl₂ and 50 pg•ml⁻¹ bovine serum albumin. The H2-enzyme preparation was dissolved at a protein concentration of 0.75 mg•ml⁻¹ in an identical buffer. Samples were withdrawn periodically and assayed for residual β-glucanase activity.

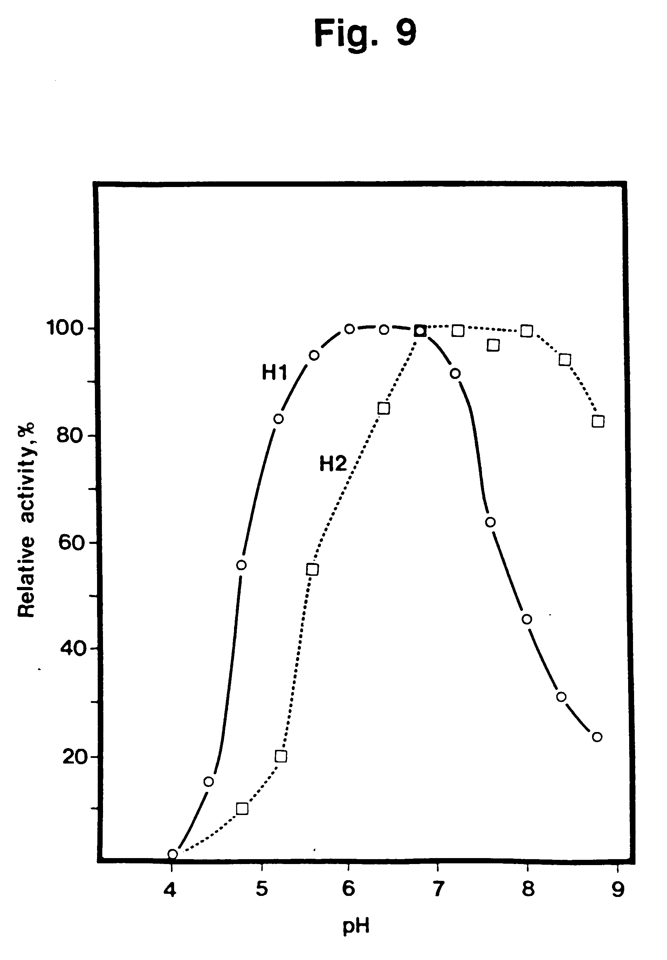

*Figure 9.* The pH dependence of the activity of *Bacillus* hybrid βglucanases H1 and H2. The reactions were carried out with 1-6 µg H1 β-glucanase and 7-70 µg H2 β-glucanase preparation in the following buffers: 40 mM Na-acetate, pH 3.6-5.6; 40 mM K/Na phosphate, pH 6-8 and 40 mM Tris-Hcl, pH 8.4-8.8. Activity was determined with the Biocon assay using azo-barley β-glucan as substrate (McCleary, 1988).

*Figure 10.* Activity of *Bacillus* hybrid β-glucanase H1 and parental enzymes in crude extracts from transgenic *E. coli* cells after incubation for various lengths of time at 65°C, pH 4.0. Activity is expressed as per cent of the activity at time 0.

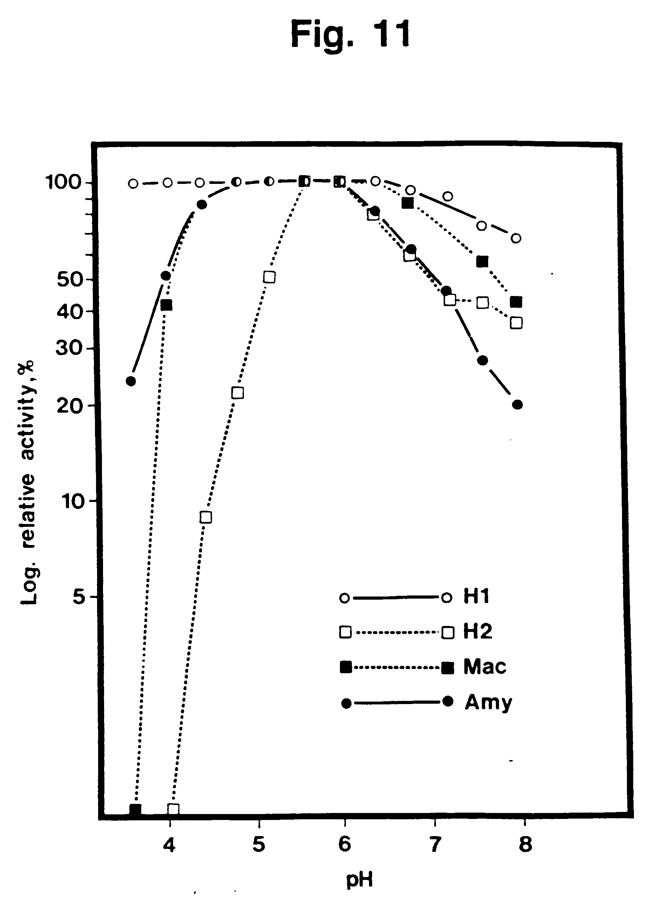

*Figure 11.* The pH dependence of stability of *Bacillus* β-glucanase at 55°C. 2 µg of hybrid β-glucanase H1, 375 µg protein of hybrid β-glucanase H2 preparation, 2 µg of *B. macerans* β-glucanase or 10 µg of *B. amyloliquefaciens* β-glucanase were tested with 10 mM CaCl and 50 µg.ml⁻¹ bovine serum albumin in 40 mM Na-acetate buffer adjusted to the indicated pH values in the range of 3.6 to 5.6 or in 40 mM K/Na phosphate buffer adjusted to the indicated pH values in the range 6.0 to 8.0. After incubation for 1 h at 55°C the residual activity was measured with method A (see Materials and Methods).

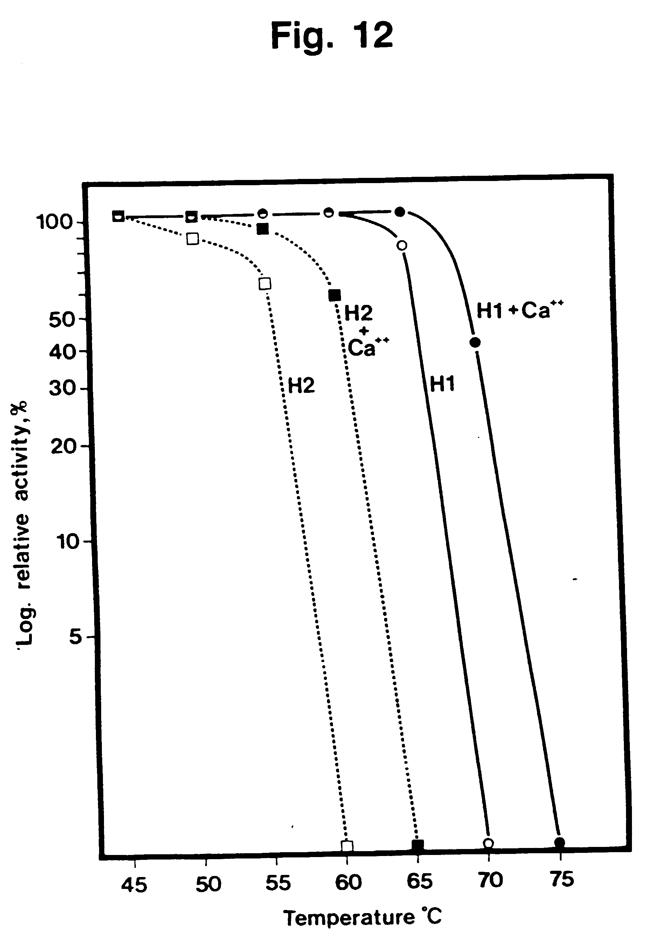

*Figure 12.* Improvement of thermal stability of *Bacillus* hybrid β-glucanases in the presence of CaCl₂. 0.1 µg *Bacillus* hybrid enzyme H1 or 750 µg hybrid enzyme H2 preparation was dissolved in 1 ml 40 mM Na-acetate buffer pH 5.5 with or without 50 mM CaCl₂ and supplemented with 50 µg•ml⁻¹ bovine serum albumin. After incubation for 30 min. at the indicated temperatures the residual activity was determined.

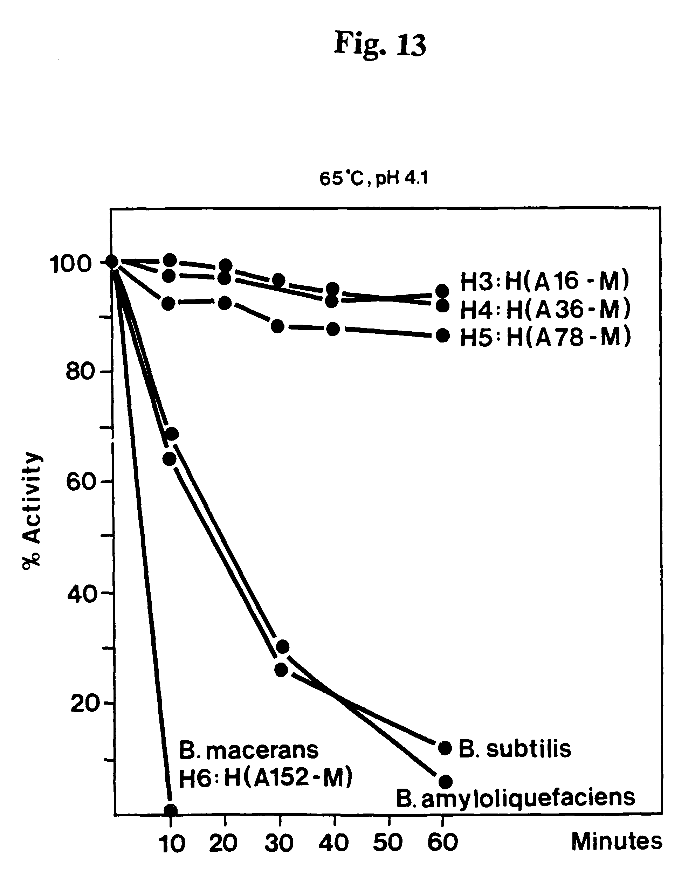

*Figure 13.* Time course of thermoinactivation of purified *Bacillus* hybrid β-glucanases H3, H4, H5 and H6 at 65°C, pH 4.1 in comparison with purified native β-glucanases of *B. amyloliquefaciens, B. macerans,* and *B. subtilis.* The enzymes were dissolved at a concentration of 0.1 mg•ml⁻¹ in 40 mM Na-acetate buffer, pH 4.1, 10 mM CaCl₂. Samples were withdrawn periodically and assayed for residual β-glucanase activity.

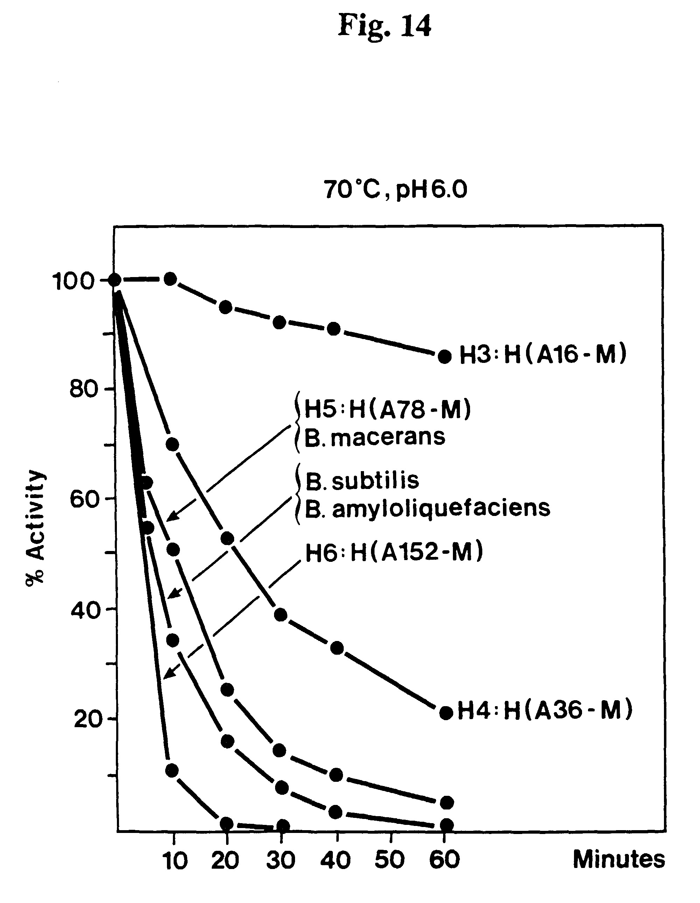

*Figure 14.* Time course of thermoinactivation of purified *Bacillus* hybrid β-glucanases H3, H4, H5 and H6 at 70°C, pH 6.0 in comparison with purified native β-glucanases of *B. amyloliquefaciens, B. macerans,* and *B. subtilis.* The enzymes were dissolved at a concentration of 0.1 mg•ml⁻¹ in 40 mM Na-acetate buffer, pH 5.5, 10 mM CaCl₂. Samples were withdrawn periodically and assayed for residual β-glucanase activity.

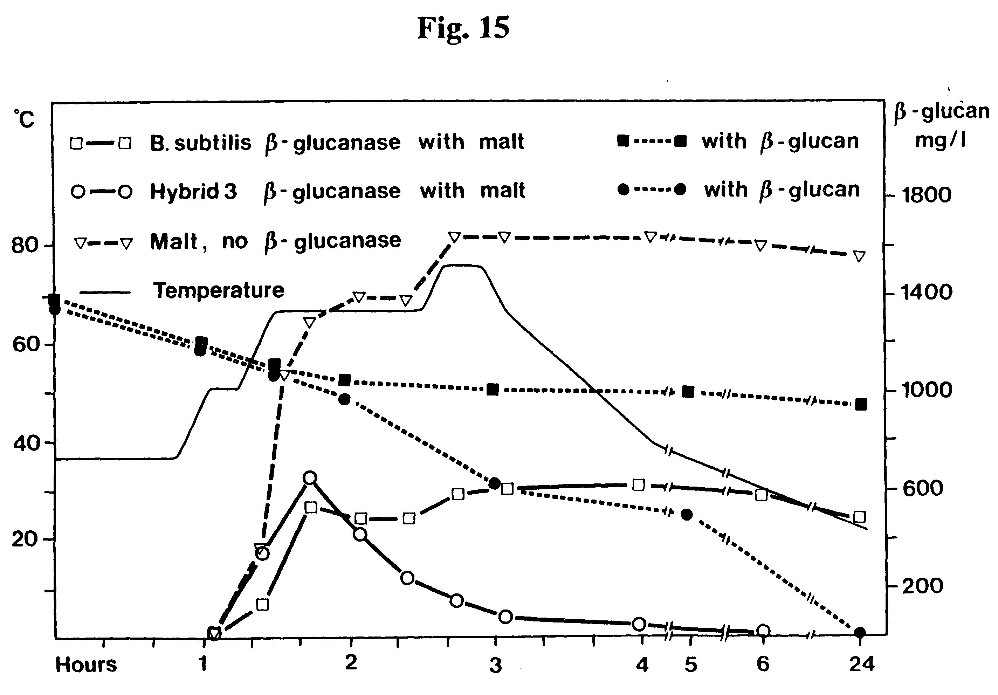

*Figure 15.* The concentration of residual malt β-glucans during mashing of mixtures consisting of 50 g finely ground malt and 200 ml water and containing 5 µg H3 hybrid β-glucanase, 5 µg *B. subtilis* β-glucanase and no β-glucanase, respectively, and of residual β-glucan in solutions consisting of 50 ml β-glucan solutions (1.5 mg/ml) in 100 ml Na-acetate buffer, pH 5.5, 5 mM CaCl₂ to which 250 ng *B. subtilis* β-glucanase and H3 hybrid glucanase, respectively, were added. Samples were drawn periodically and assayed for residual β-glucans.

### MATERIALS AND METHODS

### Strains, plasmids and growth media

*E. coli* DH5α cells: F⁻, *endA1, hsd R17* (rₖ⁻, mₖ⁺), *supE44, thi1, λ*^{*-*}, *recA1, gyrA96, relA1, ø80dlacZ, ΔM15* (Hanahan, 1985) were used for propagation of plasmids and for expression of β-glucanase genes. The vectors comprised pBR322 (Bolivar et al., 1977) and pUC19 (Vanish-Perron et al., 1985). The recombinant plasmid pEG1 (Borriss et al., 1985) carries an insert with the *B. amyloliquefaciens* β-glucanase gene and pUC13-M carries a DNA insert with the β-glucanase gene from *B. macerans* which is identical to the insert of plasmid pUC19/34 (Borriss et al., 1988). Media and growth conditions were as described previously (Borriss et al., 1988).

### Enzymes and chemicals

Radioactive nucleotides were from New England Nuclear, Boston, Massachusetts, USA. Restriction endonucleases, calf intestinal phosphatase and T4-DNA ligase were from Boehringer Mannheim, Mannheim, W. Germany. Modified T7-DNA polymerase (Sequenase™) was from United States Biochemical Corporation, Cleveland, Ohio, USA. A Geneclean™ kit was from BIO 101 Inc., La Jolla, California, USA. Barley β-glucan as well as a β-glucanase assay kit was purchased from Biocon, Boronia, Victoria, Australia. Lichenan was prepared from *Cetraria islandica* as described previously (Borriss, 1981).

### Transformation

*E. coli* cells were grown and prepared for transformation as described by Lederberg and Cohen (1974) and the competent cells were stored frozen as described by Thomsen (1983).

### DNA purification

Plasmid DNA was prepared from *E. coli* by the method of Hattori and Sakaki (1986). Specific DNA fragments generated by restriction endonuclease digestion were separated by agarose gel electrophoresis and purified from the gel matrix using a Geneclean™ kit according to the manufacturer's recommendations.

### DNA sequence determination

Modified T7-DNA polymerase (Sequenase™) was used for nucleotide sequence determination around splice junctions of hybrid-β-glucanase genes. The reactions were performed as described by Zhang et al. (1988).

### Enzyme purification and analysis

For determination of thermostability of the hybrid enzyme H1 and parental enzymes *E. coli* cells harbouring the plasmid pUC13-H1, the plasmids pEG1 and pUC13-M were grown in tryptone-yeast medium (10 g tryptone, 5 g yeast extract, 5 g NaCl per 1) at 37°C for 16 to 20 hours. The cells were lysed by sonication (MSE sonifier) and after clearing of the lysate by centrifugation, β-glucanase stability was analyzed by incubation of the reaction mixture containing an aliquot of clarified lysate for various lengths of time at 65°C or 70°C followed by determination of residual β-glucanase activity.

Purification of β-glucanase from cell extracts as described in Borriss et al. (1988) has been used for the parental enzymes and hybrid enzyme H1. Due to low yield of H2 β-glucanase this enzyme was not purified to homogeneity. Ammonium sulphate precipitation of crude cell extracts enriched this β-glucanase to a specific activity of 10.4 U/mg (10.4 µmole glucose mg⁻¹•min⁻¹). Protein concentration was determined according to Bradford (1976) using bovine serum albumin as standard. Enzyme preparations were analyzed by SDS-PAGE (Laemmli, 1970).

### β-Glucanase assays

*Method A:* The reaction mixture consisted of 1 ml 0.5% (w/v) lichenan or barley β-glucan in 40 mM Na-acetate buffer, pH 6.0, with or without 10 mM CaCl₂. The reaction was initiated by addition of 0.1 ml enzyme solution and incubation was at 37°C for 20 min. The reaction was stopped by addition of 0.5 ml 3,5-dinitrosalicylic acid and the amount of reducing sugars were measured using the reagent formulation outlined by Miller (1959). Specific activity is expressed as µmole glucose released per min. and mg of protein.

*Method B:* Alternatively, azo-barley β-glucan was used as substrate for analysis of β-glucanase activity (McCleary, 1988). The buffers employed were: 40 mM sodium acetate, pH 3.6-5.6; 40 mM potassium-sodium phosphate, pH 6-8; 40 mM Tris-HCl, pH 8.4-8.8.

### Plate assay

*E. coli* cells were incubated on solid medium containing 0.2% (w/v) lichenan. Staining with 0.2% (w/v) Congo red reveals a clearing zone around colonies expressing β-glucanase.

### Containment

All experiments involving recombinant DNA were carried out under BL1 laboratory conditions and waste containing biological material was autoclaved.

### EXAMPLE 1

### CONSTRUCTION OF PLASMIDS pUC13-H1 AND pUC19-H2 CARRYING HYBRID β-GLUCANASE GENES

The *B. amyloliquefaciens* and *B. macerans* β-glucanase genes, and proteins, are highly homologous. In the center of the genes is a unique EcoRV restriction site which was used as fusion point in the construction of hybrid β-glucanase genes.

### Construction of pUC13-H1 (Fig. 1 )

An EcoRV fragment which contains the 5'-flanking region and the amino-terminal half coding region of the *B. amyloliquefaciens* β-glucanase gene was isolated from plasmid pEG1 (Boriss et al., 1985) and ligated with the large EcoRV-EcoRI fragment from pUC13-M encoding the carboxyl-terminal half of the *B. macerans* enzymes thus generating plasmid pUC13-H1 carrying the hybrid gene bgl-H1. *E. coli* DH5α cells transformed with pUC13-H1 are resistant to ampicillin.

### Construction of pUC19-H2 (Fig. 2)

For construction of the reciprocal recombinant gene, the *B. macerans* β-glucanase gene was excised as an EcoRI-PstI fragment from plasmid pUC13-M and recloned in pBR322 giving rise to plasmid pBR-MAC1 from which the small EcoRV fragment was purified and fused to the large EcoRV fragment from plasmid pEG1. With the insert in the correct orientation the plasmid is designated pEG-H2 and *E. coli* cells transformed with the plasmid were selected on medium containing tetracycline. The hybrid gene was excised from pEG-H2 as an EcoRI-BglII fragment and recloned in EcoRI-BamHI digested pUC19 to give plasmid pUC19-H2.

### EXAMPLE 2

### THE STRUCTURE OF HYBRID β-GLUCANASE GENES bgl-H1 AND bglH2

The fragment for the expression of the bgl-H1 recombinant gene is shown in Fig. 3. The construct contains 469 bp of the flanking region, 75 bp encoding the signal peptide, and 321 bp encoding the amino-terminal half of the *B. amyloliquefaciens* β-glucanase. This 865 bp DNA stretch is fused in frame to the carboxyl-terminal half coding region as well as 54 bp of the 3'-flanking region of the β-glucanase gene from *B. macerans.*

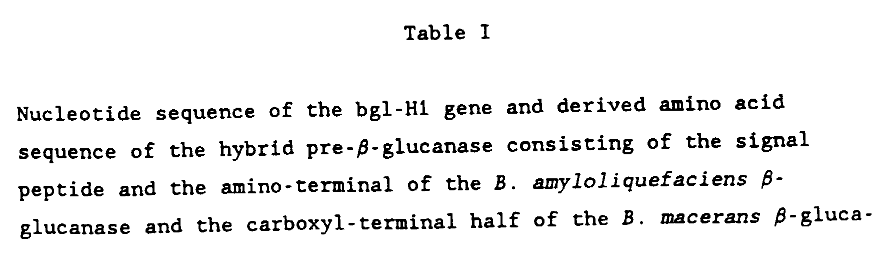

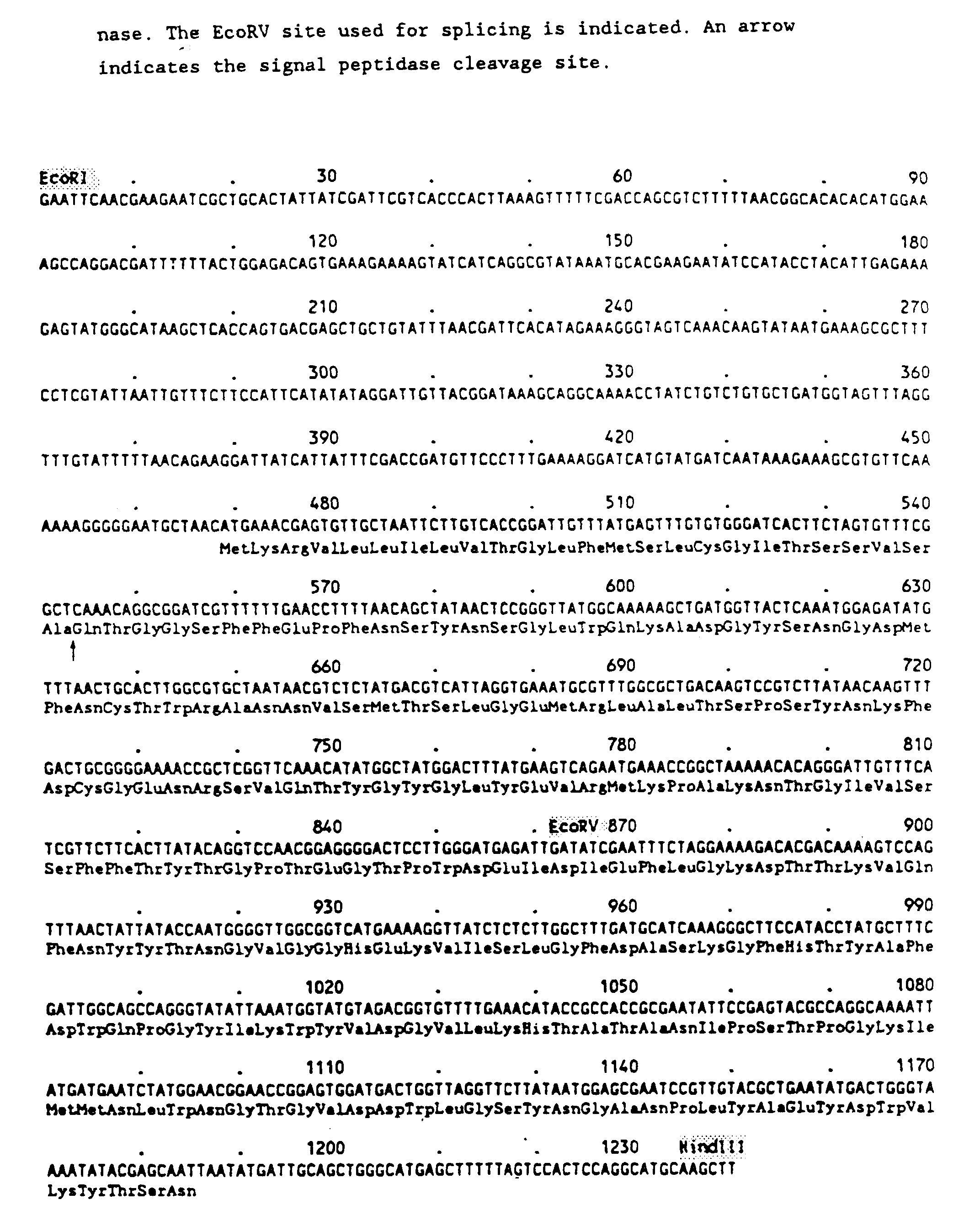

The other recombinant gene, bgl-H2, (Fig. 4) consists of 99 bp of the 5'-flanking region, 75 bp encoding the signal peptide and 315 bp encoding the amino-terminal half of the *B. macerans* β-glucanase. This 489 bp fragment is fused in frame to a 321 bp DNA segment encoding the carboxyl-terminal half of *B. amyloliquefaciens* β-glucanase and approximately 1.5 Kb 3'-flanking region.

Plasmid constructions were analyzed by restriction enzyme digests, DNA sequence determination around splice junctions, or both.

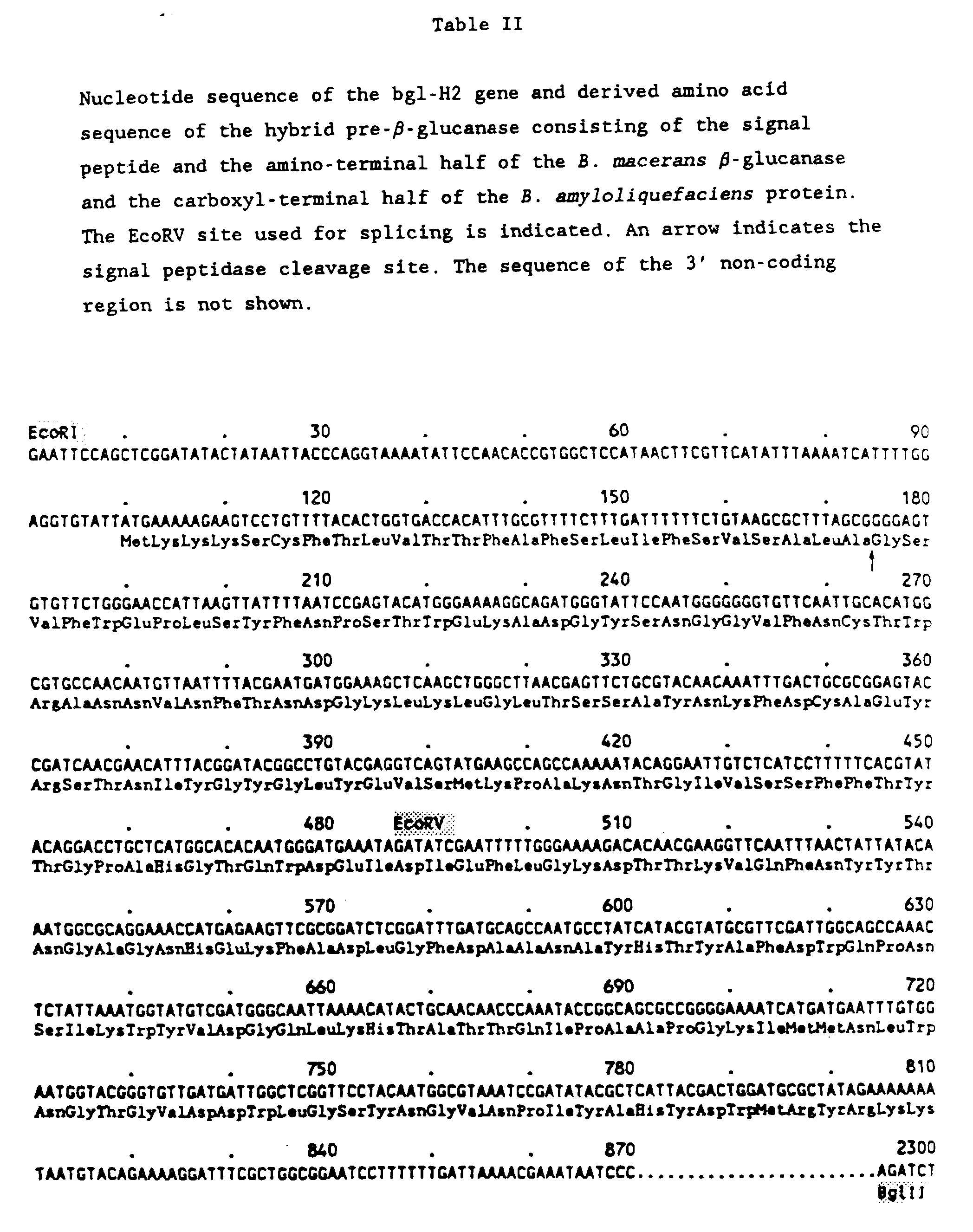

### EXAMPLE 3

### ANALYSIS OF HYBRID GENE PRODUCTS ENCODED BY pUC13-H1 AND pUC19-H2

*E. coli* DH5α cells were transformed with pUC13-H1 or pUC19-H2, respectively and transformed hybrid β-glucanase genes were expressed in these *E. coli* cells. The hybrid β-glucanase H1 was purified according to the procedure used for *B. macerans* β-glucanase (Borriss et al., 1988). By SDS-PAGE it was confirmed that the β-glucanase migrated as one Coomassie blue staining band (Fig. 5). The yield of hybrid enzyme H2 was only 1% of that obtained of H1 and too low to produce a chromatographically pure preparation (Table III).

**TABLE III**

| **Expression of β-glucanase in** *E. coli* **cells transformed with pUC13-H1 and pUC19-H2, respectively** | | |
|---|---|---|
| β-glucanase activity (µmole glucose ml culture⁻¹•min⁻¹) | | |
| Plasmid | Cells | Supernatant |
| pUC13-H1 | 67.5 | 7.0 |
| pUC19-H2 | 0.06 | n.d. |
| n.d. = not detectable | | |

Cells were grown in tryptone-yeast medium with intensive shaking for 20 h at 37°C. After centrifugation (5000 x g, 10 min.), the supernatant was used directly for assay of enzyme activity. The pellet was washed, resuspended in 40 mM acetate, pH 6 and sonicated on ice 4 x 20 sec. with a Branson Sonifier and clarified by centrifugation.

The specific activity of β-glucanase H1 was determined to be 3700 µmole glucose mg⁻¹•min.⁻¹ which is comparable to the specific activity of β-glucanases from *Bacillus* IMET B376 (1330 µmole glucose mg⁻¹•min⁻¹) (Borriss et al., 1985) and from *B. macerans* (5030 µmole glucose mg⁻¹•min⁻¹). For characterization of the bgl-H2 gene product an enriched extract with a specific activity of 10.4 µmole glucose mg⁻¹•min⁻¹ was used (Table IV)

**TABLE IV**

| **Kinetic parameters of hybrid and parental β-glucanases** | | | | |
|---|---|---|---|---|
| | β-glucanase | | | |
| Substrate | Hybrid 1 | Hybrid 2 | *Macerans* | *Amyloliquefaciens* |
| | Relative Vₘₐₓ | | | |
| Glucan | 1 | 1 | 1 | 1 |
| Lichenan | 0.77 | 0.88 | 0.73 | 1.1 |
| | | | | |

| | Kₘ (mg/ml) | | | |
|---|---|---|---|---|
| Glucan | 1.25 | 1.67 | 0.83 | 1.25 |
| Lichenan | 1.05 | 1.54 | 0.67 | 1.67 |
| | | | | |

| | Specific activity (µmole glucose mg⁻¹•min⁻¹) | | | |
|---|---|---|---|---|
| | 3722 | 10.4 (1) | 5030 | 1330 (2) |

| | | | | |
|---|---|---|---|---|
| (1) enriched cell extract | | | | |
| (2) Borriss and Zemek, 1981 | | | | |

### Substrate specificity

Hybrid enzymes H1 and H2 degraded barley (1,3-1,4)-β-glucan as well as lichenan and the Vₘₐₓ values determined with both substrates did not differ significantly (Table IV). The Kₘ values for both hybrid proteins were determined using either barley β-glucan or lichenan as substrate.

### Kinetics of thermoinactivation of hybrid β-glucanases

The thermostability of hybrid β-glucanases in comparison with the parental enzymes from *B. amyloliquefaciens* and *B. macerans* was studied by measuring the time course of thermoinactivation of β-glucanase in samples of cleared lysates of *E. coli* DH5α cells transformed with plasmids pUC13-H1, pEG-H2, pEG1 and pUC13-M encoding H1, H2, *B. amyloliquefaciens* and *B. macerans* recombinant β-glucanase, respectively.

The *E. coli* strain transformed with pUC13-H1 was deposited with Deutscha Sammlung von Mikroorganismen under the Accession No. DSM 5461. The *E. coli* strain transformed with pEG-H2 was deposited with Deutsche Sammlung von Mikroorganismen under the Accession No. DSM 5460. The *E. coli* strain transformed with pEG1 was deposited with Deutsche Sammlung von Mikroorganismen under the Accession No. DSM 5459 and the *E. coli* strain transformed with pUC13-M was deposited with Deutsche Sammlung von Mikroorganismen under the Accession No. DSM 5462.

The samples (usually in the concentration range 0.3-1 mg protein/ml) were incubated in 10 mM CaCl₂, 40 mM Na-acetate, pH 6.0 at 70°C and samples were removed periodically for determination of residual β-glucanase activity (Fig. 6). The results of this analysis revealed that the half-life of H1 β-glucanase is significantly higher (50% inactivation in 18.5 min.) than half-lives of the parental enzymes from *B. amyloliquefaciens* (4 min.) and *B. macerans* (9 min.). The H2 β-glucanase underwent thermoinactivation with a half-life less than 2 min. and is thus more heat-labile than the parental enzymes. When the analysis was carried out at 65°C (Fig. 7) the hybrid enzyme H1 was stable for more than 30 min. while the half-life of the enzyme from *B. amyloliquefaciens* was about 25 min. and that of *B. macerans* intermediate between the two. Purified H1 enzyme was stable for more than 1 hour when analyzed at 65°C, pH 6.0, whereas partially purified H2 enzyme was irreversibly thermoinactivated within 20-25 min. (Fig. 8). A time course for the inactivation of purified enzyme from *B. amyloliquefaciens* is shown as reference. Consistently, the hybrid enzyme H1 was significantly activated when tested after 5 min. at 65 to 70°C (Figs. 6-8).

### Effect of pH on enzymatic activity and stability of hybrid β-glucanases

The pH range for optimal enzymatic activity of hybrid β-glucanase H1 was pH 5.6 to 6.6, while that for hybrid enzyme H2 was pH 7.0 to 8.0 (Fig. 9). For comparison, the pH optimum range for enzymatic activity of the β-glucanases from *B. amyloliquefaciens* and *B. macerans* was from pH 6.0 to 7.0 and from pH 6.0 to 7.5, respectively (results not shown). Fig. 9 also shows that the hybrid enzyme H1 retains 50% of its activity at pH 4.8 and that H2 retains 50% of its activity at pH 5.6. The corresponding values for the parental enzymes are pH 5.2 *(B. amyloliquefaciens)* and pH 5.5 *(B. macerans).*

Another characteristic is enzyme stability as a function of pH. When the time course of thermoinactivation of the β-glucanases in crude extracts was followed at pH 4.0 and a temperature of 65°C the hybrid enzyme H1 was stable for more than 30 min. while the β-glucanase from *B. amyloliquefaciens* had a half-life of 20 min. and that of *M. macerans* of only 12 min. (Fig. 10). This feature was examined for hybrid and parental β-glucanases by incubation at 55°C for 1 h in the range pH 3 to 9, followed by determination of residual enzymatic activity (Fig. 11). It appears that β-glucanase H1 is stable from below pH 3.6 up to 7.0, while β-glucanase H2 has a very narrow pH range of stability between pH 5.6 to 6.0. Both parental β-glucanases are unstable below pH 4.8 and above pH 6.0 (*B. amyloliquefaciens*) or pH 6.5 (*B. macerans*)*.*

### The effects of Ca⁺⁺ on thermostability

The effect of Ca⁺⁺ on the stability of hybrid β-glucanases was analyzed in a 30 min. assay at pH 5.5 and temperatures ranging from 45°C to 75°C. From the results of this analysis, shown in Fig. 12, the temperature for 50% inactivation in a 30 min. assay can be deduced. It appears clearly that Ca⁺⁺ ions have a stabilizing effect on both hybrid enzymes. The temperatures for 50% inactivation increase about 5°C for both hybrid β-glucanases in the presence of 10 mM Ca⁺⁺. The same stabilizing effect of Ca⁺⁺ ions is also found for the two parental enzymes.

### EXAMPLE 4

### CONSTRUCTION OF HYBRID β-GLUCANASES H3, H4, H5, AND H6

Four (1,3-1,4)-β-glucanases were produced by constructing hybrid fusion genes encoding the glucanases using a polymerase chain reaction technique according to the procedure described by Yon & Fried (1989), *Nucleic Acid Res.,* 17, 4895, and Horton et al. (1989) *Gene,* 77, 61-68. The fusion genes comprise DNA sequences from the *B. amyloliquefaciens* BE20/78 β-glucanase gene and from the *B. macerans* E 138 β-glucanase gene. The fusion genes were inserted in the plasmid pTZ19R (Mead et al., 1986, *Protein Engineering, 1,* 67-74). The four resulting recombinant plasmids were designated pTZ19R-H3, pTZ19R-H4, pTZ19R-H5, and pTZ19R-H6, respectively. The plasmids were used for transformation of *E. coli* DH5α. The host cells were grown in minimal medium to stationary phase to obtain expression of the β-glucanase genes. The resulting hybrid enzymes were designated H3, H4, H5, and H6, respectively. The H3 enzyme has the formula A16 - M indicating that it is a hybrid enzyme comprising 16 amino acids from the N-terminal part of mature *B. amyloliquefaciens* (1,3-1,4)-β-glucanase which have replaced the corresponding 16 amino acids from the N-terminal part of the mature *B. macerans* (1,3-1,4)-β-glucanase. The hybrid enzymes H4-H6 were constructed in a similar manner by replacing 36, 78, and 152 amino acids, respectively of the N-terminal part of mature *B. macerans* (1,3-1,4)-β-glucanase with the corresponding number of amino acids from the N-terminal part of the *B. amyloliquefaciens* β-glucanase. Thus all of the four constructed hybrid enzymes have an N-terminal end originating from the *B. amyloliquefaciens* (1,3-1,4)-β-glucanase. Furthermore, all hybrid enzymes are synthesized with the transient signal peptide of *B. amyloliquefaciens* β-glucanase.

The *E. coli* strain carrying pTZ19R-H3 encoding the hybrid enzyme H3 has been deposited with Deutsche Sammlung von Mikroorganismen under the Accession No. DSM 5790, the *E. coli* strain transformed with pTZ19R-H4 encoding the hybrid enzyme H4 was deposited with Deutsche Sammlung von Mikroorganismen under the Accession No. DSM 5791, the *E. coli* strain transformed with pTZ19R-H5 encoding the hybrid enzyme H5 was deposited with Deutsche Sammlung von Mikroorganismen under the Accession No. DSM 5792, and the *E. coli* strain carrying the pTZ19R-H6 plasmid encoding the hybrid enzyme H6 was deposited with Deutsche Sammlung von Mikroorganismen under the Accession No. DSM 5793. All the above four strains were deposited on 9 February, 1990.

### EXAMPLE 5

### PURIFICATION AND CHARACTERIZATION OF HYBRID β-GLUCANASES H3, H4, H5, AND H6

The hybrid enzymes encoded by the hybrid fusion genes were characterized by determining their temperature optimum, pH optimum, specific activity, and thermostability. These characteristics were compared with the corresponding characteristics of the hybrid enzyme H1 as described hereinbefore and of native *Bacillus* β-glucanases as produced by the following *Bacillus* spp.: *B. amyloliquefaciens* BE20/78, *B. macerens* E138, and a *B. subtilis* sp. *E. coli* cells transformed with plasmids carrying the genes encoding said *B. amyloliquefaciens* β-glucanase (pEG1) and *B. macerans* β-glucanase (pUC13-M), respectively were grown in minimal medium to stationary phase to obtain expression of the β-glucanase gene products.

One to five litres of culture medium resulting from the growth of the above transformed *E. coli* cells expressing the hybrid β-glucanases H3, H4, H5, and H6 and the native *B. amyloliquefaciens* and *B. macerans* β-glucanases were cleared by centrifugation and filtration through an 0.8 µm filter. The cleared supernatants were concentrated to 100 ml by ultrafiltration. Following diafiltration against 20 mM Na-acetate, pH 5.0, 5 mM CaCl₂, the crude supernatant was applied to a CM-sepharose cation exchange column. After washing, the column was eluted with 50 mM sodium acetate, pH 5.0, 50 mM NaCl, 5 mM CaCl₂. The β-glucanase obtained by this purification scheme was essentially pure, but usually the fractions containing β-glucanase activity were pooled and concentrated to 2-5 ml and subjected to molecular sieve chromatography on a Sephacryl S200 HR column (2.5 x 60 cm) in 20 mM sodium acetate, pH 5.0, 5 mM CaCl₂. The β-glucanase peak fractions were used for analysis of the thermostability of pure enzymes. The yield of pure β-glucanase was in the range of 0.5-25 mg per litre culture medium.

The native *B.subtilis* enzyme was a commercial (1,3-1,4)-β-glucanase product (Cereflo® 200L, Novo-Nordisk A/S, Bagsværd, Denmark). As a first step of purification this product was concentrated five-fold followed by diafiltration against 50 mM Tris-HCl, pH 8 and anion exchange chromatography (Whatman DE 53). Unbound protein was concentrated and the buffer was changed to 20 mM sodium acetate, pH 5.0, 5 mM CaCl₂ by diafiltration. Further purification was obtained by cation exchange chromatography on CM52 (Whatman). Fractions containing β-glucanase activity were pooled, concentrated and subjected to molecular sieve chromatography on Sephacryl S200 HR. Approximately 100 mg pure *B. subtilis* β-glucanase was obtained from 1 litre of Cereflo® 200L.

### Temperature optima for H3-H6

The above pure preparations of the four test enzymes and of the four reference enzymes containing 100 µg purified enzyme per ml were diluted to contain an amount of enzyme which under the assay conditions resulted in measurable values (0.5 - 1.5 µg per ml). The reaction mixtures consisted of 1 ml substrate (0.5 mg/ml lichenan) in 100 mM Na-acetate buffer, pH 6.0 supplemented with 50 µg/ml bovine serum albumin and 0.1 ml of the appropriately diluted enzyme preparations. The reaction mixtures were incubated for 10 minutes at the following temperatures: 25, 37, 50, 55, 60, 65, 70, 75, 80, and 85°C and the reaction was stopped by the addition of 0.5 ml 3,5-dinitrosalicylic acid. The optimum temperatures and the temperature ranges within which at least 90% of the optimum activity was exerted were determined and the results are shown in the below table:

**TABLE V**

| **Temperature optima for H3-H6, H1, and native** *Bacillus* **β-glucanases** | | |
|---|---|---|
| β-glucanase | Temperature optimun, °C | Temperature range with ≥90% of optimum activity, °C |
| H3 | 65 | 55-75 |
| H4 | 70 | 55-70 |
| H5 | 65 | 55-70 |
| H6 | 55 | 50-65 |
| H1 | 55 | 50-65 |
| *B. subtilis* | 55 | 50-65 |
| *B. macerans* | 65 | 60-70 |
| *B. amyloliquefaciens* | 55 | 50-65 |

Additionally, the H3 enzyme had a residual activity at 80°C of 75% of the optimum activity and at 85°C the corresponding residual activity was 20%.

Among the tested hybrid enzymes, H4 had a higher temperature optimum than any of the native enzymes (70°C) and the H3 enzyme showed the broadest temperature range within which 90% or more of the optimum activity was retained (55 - 75°C). This enzyme also had the highest temperature limit for at least 90% activity relative to its optimum activity.

### pH optima for H3-H6

In these experiments the enzymatic activity of the same enzyme preparations as described above including the four reference enzymes was assayed at different pH-values ranging from 3.6 to 8.0. In the assay, Azo-barley β-glucan was used as the substrate. 200 µl of substrate solution was mixed with 200 µl 100 mM buffer solutions having appropriate pH-values and containing 10 - 100 ng of the purified enzyme preparations. Within the pH range 3.6 to 6.0 Na-acetate buffers were used and within the range of 6.1 to 8.0 Tris-acetate were used. The assay time was 10 minutes. The pH optimum and the pH interval within which at least 90% of the optimum activity was present were determined for each enzyme preparation. Furthermore, the activity of the enzymes at pH 5.0 relative to the activity at the optimum pH was calculated. These assay results are summarized in the table below:

**TABLE VI**

| **pH optima for H3-H6, H1, and native** *Bacillus* **β-glucanases** | | | | |
|---|---|---|---|---|
| β-glucanase | pH optimum | pH range with ≥90% of optimum activity | Activity of pH 5.0 relative to to optimum pH 5.5 | |
| H3 | 7.0 | 6.5-7.0 | 10% | 63% |
| H4 | 6.5 | 5.9-7.6 | 15% | 70% |
| H5 | 7.0 | 5.9-7.6 | 30% | 88% |
| H6 | 6.5 | 5.9-6.5 | 50% | 80% |
| H1 | 5.9 | 5.5-6.5 | 55% | 86% |
| *B. subtilis* | 6.5 | 5.9-6.5 | 19% | 75% |
| *B. macerans* | 7.6 | 5.9-7.6 | 8% | 56% |
| *B. amyloliquefaciens* | 6.5 | 5.9-7.0 | 30% | 69% |

The pH optima for the four test enzymes were in the range of 6.5 - 7.0. At pH 5.0 the enzymes H5 and H6 showed activities relative to these at their pH optima which exceeded the corresponding values for all of the three native *Bacillus* enzymes indicating a somewhat lower sensitivity to non-optimum pH conditions. The lower pH limit for retaining at least 90% activity was 5.9 for all test enzymes which is similar to what was found for the native enzymes. The results of this experiment therefore indicates that by constructing hybrid enzymes comprising polypeptides from the *B. amyloliquefaciens* and *B. macerans* (1,3-1,4)-β-glucanases a higher tolerance to acidic conditions can be obtained.

### The specific activity of H3-H6

The specific activity of the H3-H6 β-glucanases were determined essentially as described hereinbefore using the preparations of purified enzymes at a concentration of 100 µg β-glucanase protein per ml of 20 mM Na-acetate buffer, pH 6.0 supplemented with 5 mM CaCl₂. The reaction mixtures were incubated at 25 and 50°C, respectively for 20 minutes after which the specific activity in terms of µmoles glucose released per mg of enzyme per minute was determined. The results are shown in the table below.

**TABLE V**

| **Specific activities of H3-H6, H1, and native** *Bacillus* **β-glucanases (µmole glucose mg purified enzyme**^{**-1**}**•min**^{**-1**}**)** | | |
|---|---|---|
| β-glucanase | Specific activity at | |
| | 25°C | 50°C |
| H3 | 790 | 1700 |
| H4 | 850 | 2600 |
| H5 | 615 | 1890 |
| H6 | 2040 | 3750 |
| H1 | 2130 | 3690 |
| *B. subtilis* | 1420 | 2600 |
| *B. macerans* | 350 | 1180 |
| *B. amyloliquefaciens* | 1320 | 2490 |

From the results it appears that at 25°C the hybrid enzyme H6 has a specific activity which is significantly higher than any of the parental *Bacillus* enzymes and of the *B. subtilis* β-glucanase. Generally, the specific activities at 50°C were 1.5 - 3 times higher than the values at 25°C. Also at this temperature the specific activity of H6 exceeded that of the parental enzymes as well as that of the native *Bacillus subtilis* β-glucanase. The hybrid enzyme H1 also exhibited a high specific activity at both temperatures of the same magnitude as the H6 enzyme.

### The thermostability of hybrid β-glucanases H3-H6

The thermostabilities of the purified hybrid enzymes were determined in comparison with those of the purified native enzymes from the previously mentioned *Bacillus* spp. essentially according to the procedure described in Example 3. The enzyme activities were tested at 65°C, pH 4.1 and at 70°C, pH 6.0. The concentrations of enzymes were 100 µg/ml of the assay buffer. Samples of the reaction mixtures were collected at time intervals indicated in Figures 13 and 14 in which the results are summarized. It appears that the hybrid β-glucanases H3, H4 and H5 in comparison with the native enzymes showed an extremely high stability at 65°C and at pH 4.1. More than 90% of the initial enzyme activity of H3 and H4 and 85% of H5 remained after 60 minutes. At 70°C and pH 6.0 the residual enzyme activity of H3 after 60 minutes was 85%. In contrast hereto, the residual activity of the native enzymes after 60 minutes at 65°C and pH 4.1 was only about 10% for the *B. amyloliquefaciens* and the *B. subtilis* (1,3-1,4)-β-glucanase whereas the *B. macerans* β-glucanase was completely inactivated after 10 minutes. At 70°C and pH 6.0 less than 10% activity of the *B. subtilis,* the *B. macerans* and the *B. amyloliquefaciens* enzymes remained after 60 minutes of incubation.

### EXAMPLE 6

### THE EFFECT OF H3 HYBRID(1,3-1,4)-β-GLUCANASE ON THE HYDROLYSIS OF BARLEY β-GLUCAN DURING MASHING

An experiment was carried out in which the efficiency of the H3 hybrid enzyme to degrade barley β-glucan during a mashing process was compared to the efficiency of a commercial β-glucanase product. The mashing mixture consisted of 50 g finely ground malt to which 200 ml of prewarmed (37°C) tap water was added. To this substrate mixture 5 µg of the purified preparation of H3 enzyme was added under thorough mixing. As controls two similar mashing mixtures were prepared to one of which an amount of the commercial β-glucanase product Cereflo® 200L (Novo-Nordisk A/S) containing 5 µg *Bacillus subtilis* β-glucanase was added. The last mashing mixture served as a negative control without any addition of β-glucanase. The thus prepared mixtures were left at 37°C for about 50 minutes to initiate mashing whereafter they were heated according to the temperature curve indicated in Fig. 15 until 175 minutes. During the period from 65 to 185 minutes samples were drawn from the mixtures at the intervals indicated in Fig. 15. Subsequent to this period of mashing further samples were drawn after 4, 6, and 24 hours.

The drawn samples were immediately cooled in ice and centrifuged at 40°C after which the supernatants were transferred to fresh tubes and incubated for 15 minutes in boiling water to inactivate the enzymes. The samples were then assayed for residual β-glucan using calcofluor complex formation and flow injection analysis according to the procedure described by Jørgensen (*Carlsberg Res. Commun.,* 1988, 53, 277-285 and 287-296.

The results of the mashing experiments are summarized in Fig. 15 which shows the amounts of residual β-glucans in the above mashing mixtures. It appears clearly that the addition of β-glucanases resulted in significantly lower amounts of β-glucans in the mixtures as compared to the negative control. Whereas the commercial β-glucanase product ceased to hydrolyze further β-glucan when the temperature exceeded about 67°C the hybrid enzyme H3 added at the same concentration continuously degraded β-glucans during the whole incubation period irrespective of the temperature conditions. The H3 enzyme was so active that the amount of residual β-glucans after the termination of the mashing process was less than 100 mg per litre as compared to about 1600 mg in the negative control mixture and about 600 mg per litre of the mixture with the commercial *B. subtilis* β-glucanase. After 24 hours of mashing and standing essentially no detectable residual β-glucans were found in the mixture to which the H3 hybrid enzyme has been added.

During the same experiment the amounts of residual β-glucans were analyzed in solutions of pure β-glucans to which was added 250 µg of H3 hybrid β-glucanase and *B. subtilis* β-glucanase, respectively. The solutions consisted of 50 ml β-glucan (1.5 mg/ml) in 100 mM Na-acetate, pH 5.5, 5 mM CaCl₂. The solutions were prewarmed to 37°C before addition of the enzymes.

### REFERENCES

Argos, P., M.G. Rossmann, V.M. Grau, H. Zuber & J.D. Tratschin (1979): Thermal stability and protein structure. *Biochemistry 18,* 5698-5703.
Bolivar, F., R.L. Rodriguez, P.J. Greene, M.C. Betlach, H.L. Heyneker, H.W. Boye (1977): Construction and characterization of new cloning vehicle. II. A multipurpose cloning system. *Gene 2,* 95-113.
Borriss, R. (1981): Purification and characterization of an extracellular beta-glucanase from *Bacillus* IMET B376. Z. *Alg. Mikrobiologie 21,* 7-17.
Borriss, R. & K.L. Schroeder (1981): β-1,3-1,4-glucanase in sporeforming microorganisms. V. The efficiency of β-glucanase in reducing the viscosity of wort. *Zbl. Bakt. II Abt. 136,* 330-340.
Borriss, R., H. Bäumlein & J. Hofemeister (1985): Expression in *Escherichia coli* of a cloned β-glucanase gene from *Bacillus amyloliquefaciens. Appl. Microbiol. Biotechnol. 22,* 63-71.
Borriss, R., R. Manteuffel & J. Hofemeister (1988): Molecular cloning of a gene coding for thermostable beta-glucanase from *Bacillus macerans. J. Basic Microbiol. 28,* 3-10.
Bradford, M.M (1976): A rapid and sensitive method for the quantitation of microgram quantities of protein utilizing the principle of protein dye binding. *Anal. Biochem. 72,* 248-254.
Cantwell, B.A. & D.J. McConnell (1983): Molecular cloning and expression of a *Bacillus subtilis* β-glucanase gene in *Escherichia coli. Gene 23,* 211-219.
DD Patent Application WP c12N/315 706 1.
Godfrey, T. (1983): On comparison of key characteristics of industrial enzymes by type and source. Godfrey, T. & J. Reichelt (eds) Industrial Enzymology. MacMillan, London, p. 466.
Hanahan, D. (1985): Techniques for transformation of *E. coli.* In: *DNA Cloning, vol. 1.* A practical approach. D.M. Glover ed., IRL Press, Oxford, pp. 109-135.
Hattori, M. & Y. Sakaki (1986): Dideoxy sequencing method using denatured plasmid templates. *Anal. Chem. 152,* 232-238.
Hofemeister, J., A. Kurtz, R. Borriss & J. Knowles (1986): The β-glucanase gene from *Bacillus amyloliquefaciens* shows extensive homology with that of *Bacillus subtilis. Gene 49,* 177-187.
Horton, R.M., H.D. Hunt, S.N. Ho, J.K. Pullen & L.R. Pease (1989): Engineering hybrid genes without the use of restriction enzymes: Gene splicing by overlap extension. *Gene 77,* 61-68.
Imanaka, T., M. Shibazaki & M. Takagi (1986): A new way of enhancing the thermostability of proteases. *Nature 324,* 695-697.
Jørgensen, K.G. (1988): Quantification of high molecular weight (1→3)(1→4)-β-D-glucan using calcofluor complex formation and flow injection analysis. I. Analytical principle and its standardization. *Carlsberg Res. Commun. 53,* 277-285.
Jørgensen, K.G. (1988): Quantification of high molecular weight (1→3)(1→4)-β-D-glucan using calcofluor complex formation and flow injection analysis. II. Determination of total β-glucan content of barley and malt. *Carlsberg Res. Commun. 53,* 287-296.
Laemmli, U.K. (1970): Cleavage of structural proteins during the assembly of the head of bacteriophage T4. *Nature 227,* 680-685.
Lederberg, E.M. & S.N. Cohen (1974): Transformation of *Salmonella typhimurium* by plasmid deoxyribonucleic acid. *J. Bacteriol. 119,* 1072-1074.
Loi, L., P.A. Barton & G.B. Fincher (1987): Survival of barley (1→3,1→4)-β-glucanase isoenzymes during kilning and mashing. J. *Cereal Sci.5,* 45-50.
Matthews, B.W., H. Nicholson & W.J. Becktel (1987): Enhanced protein thermostability from site-directed mutations that decrease the entropy of unfolding. *Proc. Natl. Acad. Sci. 84,* 6663-6667.
McCleary, B.V. (1988): Soluble, dye-labeled polysaccharides for the assay of endohydrolases. *Methods Enzymol. 160,* 74-86.
McFadden, G.I., B. Ahluwalia, A.E. Clarke & G.B. Fincher (1988): Expression sites and developmental regulation of genes encoding (1→3,1→4)-β-glucanases in germinated barley. *Planta 173,* 500-508.
Mead, B.A., E. Szczesna-Skorupa & B. Kemper (1986): Single-stranded DNA "blue" T7 promoter plasmids: A versatile tandem promoter system for cloning and protein engineering. *Protein engineering, 1,* 67-74.
Merrifield, R.B. (1963). J. *Am. Chem. Soc. 85,* p. 2149.
Miller, G.L. (1959): Use of dinitrosalicylic acid reagent for determination of reducing sugars. *Analytical Chemistry 31,* 426-428.
Murphy, N., D.J. McConnell & B.A. Cantwell (1984): The DNA sequence of the gene and genetic control sites for the excreted *B. subtilis* enzyme β-glucanase. *Nucleic Acids Res. 12,* 5355-5367.
Querol, E. & A. Parilla (1987): Tentative rules for increasing the thermostability of enzymes by protein engineering. *Enzyme Hicrob. Technol. 9,* 238-244.
Shinnick (1983). *Ann. Rev. Microbiol. 37,* 425-446.
Streuli, M., A. Hall, W. Boll, W.E. Stewart II, S. Nagata & C. Weissmann (1981): Target cell specificity of two species of human interferon-alpha produced in *Escherichia coli* and of hybrid molecules derived from them. *Proc. Natl. Acad. Sci. USA,* 2848-2852.
Thomsen, K.K. (1983): Mouse α-amylase synthesized by *Saccharomyces cerevisiae* is released into the culture medium. *Carlsberg Res. Commun. 48,* 545-555.
Vanish-Perron, C., J. Vieira & J. Messing (1985): Improved M13 phage cloning vectors and host strains: nucleotide sequences of the M13 mp18 and pUC19 vectors. *Gene 33,* 103-119.
Weck, P., T. Apperson, N. Stebbing, H.M. Shephard, D.V. Goeddel (1981): Antiviral activities of hybrids of two major human leukocyte interferons. *Nucleic Acids Res. 9,* 6153-6165.
Yon, J. & M. Fried (1989): Precise gene fusion by PCR. *Nucleic Acid Res. 17,* 4895.
Zhang, H., R. Scholl, J. Browse & C. Sommerville (1988): Double stranded sequencing as a choice for DNA sequencing. *Nucleic Acids Res. 16,* 1220.

## Claims (Claims for the following Contracting State(s): AT, BE, CH, LI, DE, DK, FR, GB, IT, LU, NL, SE)

1. A thermostable hybrid (1,3-1,4)-β-glucanase encoded by a hybrid gene constructed by reciprocal exchange of amino-terminal and carboxy-terminal parts of (1,3-1,4)-β-glucanase genes of Bacillus spp which glucanase retains at least 50% of its activity after 10 minutes or more of incubation in 5-10 mM CaCl₂, 20-40 mM Na-acetate at a pH of 6.0 or lower and at a temperature of 65°C or higher, the incubated solution having an enzyme concentration range from 0.05 mg to 1 mg (1,3-1,4)-β-glucanase per ml, the activity of (1,3-1,4)-β-glucanase being understood as the ability of the enzyme to hydrolyze β-glycosidic linkages in (1,3-1,4)-β-glucans, the hybrid (1,3-1,4)-β-glucanase being more thermostable than any of the glucanases encoded by the parental (1,3-1,4)-β-glucanase genes of Bacillus spp.

2. A thermostable (1,3-1,4)-β-glucanase according to claim 1 wherein the incubation time is 15 minutes.

3. A thermostable (1,3-1,4)-β-glucanase according to claim 1 wherein the incubation time is 18 minutes.

4. A thermostable (1,3-1,4)-β-glucanase according to any of claims 1-3 wherein the incubation temperature is 70°C.

5. A thermostable (1,3-1,4)-β-glucanase according to any of claims 1-4 wherein the solution has a concentration range from 0.3 to 1 mg (1,3-1,4)-β-glucanase per ml.

6. A thermostable (1,3-1,4)-β-glucanase according to claim 1 which retains at least 85% of its activity after 30 minutes or more of incubation.

7. A thermostable (1,3-1,4)-β-glucanase according to claim 6 wherein the incubation time is 60 minutes.

8. A thermostable (1,3-1,4)-β-glucanase according to claim 6 which retains at least 85% of its activity after 30 minutes or more of incubation in 5-10 mM CaCl₂, 20-40 mM Na-acetate at a pH of 4.1 and at a temperature of 65°C, the incubated solution having an enzyme concentration of 0.1 mg (1,3-1,4)-β-glucanase per ml.

9. A thermostable (1,3-1,4)-β-glucanase according to claim 6 which retains at least 85% of its activity after 30 minutes or more of incubation in 5-10 mM CaCl₂, 20-40 mM Na-acetate at a pH of 6.0 and at a temperature of 70°C, the incubated solution having an enzyme concentration of 0.1 mg (1,3-1,4)-β-glucanase per ml.

10. A thermostable (1,3-1,4)-β-glucanase according to claim 8 or 9 wherein the incubation time is 60 minutes.

11. A thermostable (1,3-1,4)-β-glucanase according to any of claims 8-10 or 9 which retains at least 90% of its activity.

12. A thermostable (1,3-1,4)-β-glucanase according to any of claims 1-11 which after 10 minutes of incubation at 65°C and at pH 6.0 in crude cell extracts has a relative β-glucanase activity of at least 100%.

13. A thermostable (1,3-1,4)-β-glucanase according to claim 12 which has a relative β-glucanase activity of 130%.

14. A thermostable (1,3-1,4)-β-glucanase according to any of claims 1-13 comprising an amino acid sequence with the formula
A - M
where A is a polypeptide consisting of 5 to 200 amino acids and being at least 75% identical to a polypeptide of the same total number of amino acid residues from the N-terminal part of the Bacillus amyloliquefaciens or Bacillus macerans (1,3-1,4)-β-glucanase as given in Tables I and II, and M is a polypeptide consisting of 5 to 200 amino acids and being at least 75% identical to a polypeptide of the same total number of amino acid residues from the C-terminal part of the Bacillus macerans or Bacillus amyloliquefaciens (1,3-1,4)-β-glucanase as given in Tables I and II.

15. A thermostable (1,3-1,4)-β-glucanase according to claim 14 comprising an amino acid sequence with the formula
A - M
where A is a polypeptide consisting of 5 to 200 amino acids and being at least 85% identical to a polypeptide of the same total number of amino acid residues from the N-terminal part of the Bacillus amyloliquefaciens or Bacillus macerans (1,3-1,4)-β-glucanase as given in Tables I and II, and M is a polypeptide consisting of 5 to 200 amino acids and being at least 85% identical to a polypeptide of the same total number of amino acid residues from the C-terminal part of the Bacillus macerans or Bacillus amyloliquefaciens (1,3-1,4)-β-glucanase as given in Tables I and II.

16. A thermostable (1,3-1,4)-β-glucanase according to claim 15 comprising an amino acid sequence with the formula
A - M
where A is a polypeptide consisting of 5 to 200 amino acids and being at least 90% identical to a polypeptide of the same total number of amino acid residues from the N-terminal part of the Bacillus amyloliquefaciens or Bacillus macerans (1,3-1,4)-β -glucanase as given in Tables I and II, and M is a polypeptide consisting of 5 to 200 amino acids and being at least 90% identical to a polypeptide of the same total number of amino acid residues from the C-terminal part of the Bacillus macerans or Bacillus amyloliquefaciens (1,3-1,4)-β-glucanase as given in Tables I and II.

17. A thermostable (1,3-1,4)-β-glucanase according to claim 16 comprising an amino acid sequence with the formula
A - M
where A is a polypeptide consisting of 5 to 200 amino acids and being identical to a polypeptide of the same total number of amino acid residues from the N-terminal part of the Bacillus amyloliquefaciens or Bacillus macerans (1,3-1,4)-β-glucanase as given in Tables I and II, and M is a polypeptide consisting of 5 to 200 amino acids and being identical to a polypeptide of the same total number of amino acid residues of the amino acid residues of the C-terminal part of the Bacillus macerans or Bacillus amyloliquefaciens (1,3-1,4)-β-glucanase as given in Tables I and II.

18. A thermostable (1,3-1,4)-β-glucanase according to claim 14 where A is a polypeptide consisting of 16 amino acids which are at least 75%, preferably at least 85%, and in particular at least 90% identical to the amino acid residues of the N-terminal part of the Bacillus amyloliquefaciens (1,3-1,4)-β -glucanase as given in Table I.

19. A thermostable (1,3-1,4)-β-glucanase according to claim 14 where A is a polypeptide consisting of 36 amino acids which are at least 75%, preferably at least 85%, and in particular at least 90% identical to the amino acid residues of the N-terminal part of the Bacillus amyloliquefaciens (1,3-1,4)-β-glucanase as given in Table I.

20. A thermostable (1,3-1,4)-β-glucanase according to claim 14 where A is a polypeptide consisting of 78 amino acids which are at least 75%, preferably at least 85%, and in particular at least 90% identical to the amino acid residues of the N-terminal part of the Bacillus amyloliquefaciens (1,3-1,4)-β-glucanase as given in Table 1.

21. A thermostable (1,3-1,4)-β-glucanase according to claim 14 where A is a polypeptide consisting of 107 amino acids which are at least 75%, preferably at least 85%, and in particular at least 90% identical to the amino acid residues of the N-terminal part of the Bacillus amyloliquefaciens (1,3-1,4)-β-glucanase as given in Table I.

22. A thermostable (1,3-1,4)-β-glucanase according to claim 14 where A is a polypeptide consisting of 152 amino acids which are at least 75%, preferably at least 85%, and in particular at least 90% identical to the amino acid residues of the N-terminal part of the Bacillus amyloliquefaciens (1,3-1,4)-β-glucanase as given in Table I and II.

23. A thermostable (1,3-1,4)-β-glucanase according to any of claims 1 to 22 which has a signal peptide linked to the N-terminal end of the enzyme.

24. A thermostable (1,3-1,4)-β-glucanase according to claim 23 which has a signal peptide derived from a yeast such as a Saccharomyces species.

25. A thermostable (1,3-1,4)-β-glucanase according to claim 24 which has a signal peptide which is the signal peptide from yeast α-factor, yeast acid phosphatase or yeast invertase.

26. A thermostable (1,3-1,4)-β-glucanase according to claim 23 which has a signal peptide which is at least 75%, preferably at least 85%, more preferably at least 90% identical to the signal peptide of Bacillus amyloliquefaciens at the amino acid level.

27. A thermostable (1,3-1,4)-β-glucanase according to claim 1 which comprises the following amino acid sequence: 
Gln-Thr-Gly-Gly-Ser-Phe-Phe-Glu-Pro-Phe-Asn-Ser-Tyr-Asn-Ser-Gly-Leu-
Trp-Gln-Lys-Ala-Asp-Gly-Tyr-Ser-Asn-Gly-Asp-Met-Phe-Asn-Cys-Thr-Trp-
Arg-Ala-Asn-Asn-Val-Ser-Met-Thr-Ser-Leu-Gly-Glu-Met-Arg-Leu-Ala-Leu-
Thr-Ser-Pro-Ser-Tyr-Asn-Lys-Phe-Asp-Cys-Gly-Glu-Asn-Arg-Ser-Val-Gln-
Thr-Tyr-Gly-Tyr-Gly-Leu-Tyr-Glu-Val-Arg-Met-Lys-Pro-Ala-Lys-Asn-Thr-
Gly-Ile-Val-Ser-Ser-Phe-Phe-Thr-Tyr-Thr-Gly-Pro-Thr-Glu-Gly-Thr-Pro-
Trp-Asp-Glu-Ile-Asp-Ile-Glu-Phe-Leu-Gly-Lys-Asp-Thr-Thr-Lys-Val-Gln-
Phe-Asn-Tyr-Tyr-Thr-Asn-Gly-Val-Gly-Gly-His-Glu-Lys-Val-Ile-Ser-Leu-
Gly-Phe-Asp-Ala-Ser-Lys-Gly-Phe-His-Thr-Tyr-Ala-Phe-Asp-Trp-Gln-Pro-
Gly-Tyr-Ile-Lys-Trp-Tyr-Val-Asp-Gly-Val-Leu-Lys-His-Thr-Ala-Thr-Ala-
Asn-Ile-Pro-Ser-Thr-Pro-Gly-Lys-Ile-Met-Met-Asn-Leu-Trp-Asn-Gly-Thr-
Gly-Val-Asp-Asp-Trp-Leu-Gly-Ser-Tyr-Asn-Gly-Ala-Asn-Pro-Leu-Tyr-Ala-
Glu-Tyr-Asp-Trp-Val-Lys-Tyr-Thr-Ser-Asn
 or analogues thereof.

28. A thermostable (1,3-1,4)-β-glucanase according to claim 1 which comprises the following amino acid sequence: 
Met-Lys-Arg-Val-Leu-Leu-Ile-Leu-Val-Thr-Gly-Leu-Phe-Met-Ser-Leu-Cys-
Gly-Ile-Thr-Ser-Ser-Val-Ser-Ala-Gln-Thr-Gly-Gly-Ser-Phe-Phe-Glu-Pro-
Phe-Asn-Ser-Tyr-Asn-Ser-Gly-Leu-Trp-Gln-Lys-Ala-Asp-Gly-Tyr-Ser-Asn-
Gly-Asp-Met-Phe-Asn-Cys-Thr-Trp-Arg-Ala-Asn-Asn-Val-Ser-Met-Thr-Ser-
Leu-Gly-Glu-Met-Arg-Leu-Ala-Leu-Thr-Ser-Pro-Ser-Tyr-Asn-Lys-Phe-Asp-
Cys-Gly-Glu-Asn-Arg-Ser-Val-Gln-Thr-Tyr-Gly-Tyr-Gly-Leu-Tyr-Glu-Val-
Arg-Met-Lys-Pro-Ala-Lys-Asn-Thr-Gly-Ile-Val-Ser-Ser-Phe-Phe-Thr-Tyr-
Thr-Gly-Pro-Thr-Glu-Gly-Thr-Pro-Trp-Asp-Glu-Ile-Asp-Ile-Glu-Phe-Leu-
Gly-Lys-Asp-Thr-Thr-Lys-Val-Gln-Phe-Asn-Tyr-Tyr-Thr-Asn-Gly-Val-Gly-
Gly-His-Glu-Lys-Val-Ile-Ser-Leu-Gly-Phe-Asp-Ala-Ser-Lys-Gly-Phe-His-
Thr-Tyr-Ala-Phe-Asp-Trp-Gln-Pro-Gly-Tyr-Ile-Lys-Trp-Tyr-Val-Asp-Gly-
Val-Leu-Lys-His-Thr-Ala-Thr-Ala-Asn-Ile-Pro-Ser-Thr-Pro-Gly-Lys-Ile-
Met-Met-Asn-Leu-Trp-Asn-Gly-Thr-Gly-Val-Asp-Asp-Trp-Leu-Gly-Ser-Tyr-
Asn-Gly-Ala-Asn-Pro-Leu-Tyr-Ala-Glu-Tyr-Asp-Trp-Val-Lys-Tyr-Thr-Ser-
Asn
 or analogues thereof.

29. A DNA fragment comprising a nucleotide sequence encoding the thermostable (1,3-1,4)-β-glucanase as defined in claim 17.

30. A DNA fragment according to claim 29 which comprises the following nucleotide sequence: 
30 60
GAATTCAACG AAGAATCGCT GCACTATTAT CGATTCGTCA CCCACTTAAA GTTTTTCGAC
90 120
CAGCGTCTTT TTAACGGCAC ACACATGGAA AGCCAGGACG ATTTTTTACT GGAGACAGTG
150 180
AAAGAAAAGT ATCATCAGGC GTATAAATGC ACGAAGAATA TCCATACCTA CATTGAGAAA
210 240
GAGTATGGGC ATAAGCTCAC CAGTGACGAG CTGCTGTATT TAACGATTCA CATAGAAAGG
270 300
ATAGGATTGT TACGGATAAA GCAGGCAAAA GTAGTCAAAC AAGTATAATG AAAGCGCTTT
330 360
CCTCGTATTA ATTGTTTCTT CCATTCATAT CCTATCTGTC TGTGCTGATG GTAGTTTAGG
390 420
TTTGTATTTT TAACAGAAGG ATTATCATTA TTTCGACCGA TGTTCCCTTT GAAAAGGATC
450 480
ATGTATGATC AATAAAGAAA GCGTGTTCAA AAAAGGGGGA ATGCTAACAT GAAACGAGTG
510 540
TTGCTAATTC TTGTCACCGG ATTGTTTATG AGTTTGTGTG GGATCACTTC TAGTGTTTCG
570 600
GCTCAAACAG GCGGATCGTT TTTTGAACCT TTTAACAGCT ATAACTCCGG GTTATGGCAA
630 660
AAAGCTGATG GTTACTCAAA TGGAGATATG TTTAACTGCA CTTGGCGTGC TAATAACGTC
690 720
TCTATGACGT CATTAGGTGA AATGCGTTTG GCGCTGACAA GTCCGTCTTA TAACAAGTTT
750 780
GACTGCGGGG AAAACCGCTG GGTTCAAACA TATGGCTATG GACTTTATGA AGTCAGAATG
810 840
AAACCGGCTA AAAACACAGG GATTGTTTCA TCGTTCTTCA CTTATACAGG TCCAACGGAG
870 900
GGGACTCCTT GGGATGAGAT TGATATCGAA TTTCTAGGAA AAGACACGAC AAAAGTCCAG
930 960
TTTAACTATT ATACCAATGG GGTTGGCGGT CATGAAAAGG TTATCTCTCT TGGCTTTGAT
990 1020
GCATCAAAGG GCTTCCATAC CTATGCTTTC GATTGGCAGC CAGGGTATAT TAAATGGTAT
1050 1080
GTAGACGGTG TTTTGAAACA TACCGCCACC GCGAATATTC CGAGTACGCC AGGCAAAATT
1110 1140
ATGATGAATC TATGGAACGG AACCGGAGTG GATGACTGGT TAGGTTCTTA TAATGGAGCG
1170 1200
AATCCGTTGT ACGCTGAATA TGACTGGGTA AAATATACGA GCAATTAATA TGATTGCAGC
1230
TGGGCATGAG CTTTTTAGTC CACTCCAGGC ATGCAAGCTT
 or an analogue or a subsequence thereof.

31. A method for producing a thermostable (1,3-1,4)-β-glucanase according to claim 1 comprising cultivating a microorganism in which a DNA fragment as defined in claim 29 or 30 has been introduced in such a way that the microorganism is capable of expressing the thermostable (1,3-1,4)-β-glucanase, the cultivation being performed under conditions leading to production of the thermostable (1,3-1,4)-β-glucanase and recovering the (1,3-1,4)-β-glucanase from the culture.

32. A method according to claim 31 wherein the microorganism is a bacterium.

33. A method according to claim 32 wherein the bacterium is a gram-negative bacterium.

34. A method according to claim 33 wherein the gram-negative bacterium is an E. coli strain.

35. A method according to claim 34 wherein the E. coli strain is the E. coli strain harboring plasmid pUC13-H1, deposited in Deutsche sammlung von Mikroorganismen under Accession No. DSM 5461.

36. A method according to claim 34 wherein the E. coli strain is the E. coli strain harboring plasmid pTZ19R-H3, deposited in Deutsche Sammlung von Mikroorganismen under Accession No. DSM 5790.

37. A method according to claim 34 wherein the E. coli strain is the E. coli strain harboring plasmid pTZ19R-H4, deposited in Deutsche Sammlung von Mikroorganismen under Accession No. DSM 5791.

38. A method according to claim 34 wherein the E. coli strain is the E. coli strain harboring plasmid pTZ19R-H5, deposited in Deutsche Sammlung von Mikroorganismen under Accession No. DSM 5792.

39. A method according to claim 34 wherein the E. coli strain is the E. coli strain harboring plasmid pTZ19R-H6, deposited in Deutsche Sammlung von Mikroorganismen under Accession No. DSM 5793.

40. A method according to claim 31 wherein the microorganism is a yeast.

41. A method according to claim 40 wherein the yeast is a Saccharomyces species.

42. A method according to claim 41 wherein the Saccharomyces species is Saccharomyces cerevisiae.

43. A eukaryotic or prokaryotic organism that is transformed with and is capable of expressing the DNA fragment as defined in claim 29 or 30.

44. An organism according to claim 43 which is a microorganism.

45. A microorganism according to claim 44 which is a bacterium.

46. A bacterium according to claim 45 which is a gram-negative bacterium.

47. A bacterium according to claim 46 which is an E. coli strain.

48. The E. coli strain harboring plasmid pUC13-H1 which has been deposited in Deutsche Sammlung von Mikroorganismen under Accession No. DSM 5461 and mutants and variants thereof.

49. The E. coli strain harboring plasmid pTZ19R-H3 which has been deposited in Deutsche Sammlung von Mikroorganismen under Accession No. DSM 5790 and mutants and variants thereof.

50. The E. coli strain harboring plasmid pTZ19R-H4 which has been deposited in Deutsche Sammlung von Mikroorganismen under Accession No. DSM 5791 and mutants and variants thereof.

51. The E. coli strain harboring plasmid pTZ19R-H5 which has been deposited in Deutsche Sammlung von Mikroorganismen under Accession No. DSM 5792 and mutants and variants thereof.

52. The E. coli strain harboring plasmid pTZ19R-H6 which has been deposited in Deutsche Sammlung von Mikroorganismen under Accession No. DSM 5793 and mutants and variants thereof.

53. A microorganism according to claim 44 which is a yeast.

54. A yeast according to claim 53 which is a Saccharomyces species.

55. A Saccharomyces species according to claim 54 which is Saccharomyces cerevisiae.

56. An organism according to claim 43 which is a plant.

57. A plant according to claim 56 which is oat, barley, rye, wheat, rice or maize.

58. A method of degrading (1,3-1,4)-β-glucans in a substrate comprising subjecting the substrate to the action of an effective amount of a thermostable (1,3-1,4)-β-glucanase as defined in any of claims 1-22 for a period of time at a temperature of 65°C or higher, the amount of (1,3-1,4)-β-glucanase being at the most 200 µg, preferably at the most 100 µg, more preferably at the most 50 µg, still more preferably at the most 20 µg, and most preferably 15 µg pr. kg of substrate.

59. A method according to claim 58 wherein the substrate comprises unmodified raw grains or parts thereof from barley or oats or other grains.

60. A method according to claim 58 or 59 in which the substrate comprising (1,3-1,4)-β-glucans is mixed with a second substrate originating from maize, rice or wheat comprising a thermostable (1,3-1,4)-β-glucanase as defined in claim 1.

61. A method for the production of beer, characterized in that the wort is subjected to treatment with a thermostable (1,3-1,4)-β-glucanase according to any of claims 1-22 during mashing at a temperature of 65°C or higher and a pH between 4 and 5.5.

62. A method according to claim 61 in which the temperature is 70°C or higher.

63. A method for the production of animal feed comprising β-glucans, characterized in that the feed is supplemented with a thermostable (1,3-1,4)-β-glucanase according to claim 1 in an amount sufficient to obtain a significant degradation of β-glucans in the gastrointestinal tract of an animal fed with the (1,3-1,4)-β-glucanase supplemented feed.

64. A method according to claim 63 wherein the animal feed is pelletized.

## Claims (Claims for the following Contracting State(s): ES)

1. A process for the production of a thermostable (1,3-1,4)-β -glucanase comprising an amino acid sequence with the formula
A - M
where A is a polypeptide consisting of 5 to 200 amino acids and being at least 75% identical to a polypeptide of the same total number of amino acid residues from the N-terminal part of the Bacillus amyloliquefaciens or Bacillus macerans (1,3-1,4)-β-glucanase as given in Tables I and II, and M is a polypeptide consisting of 5 to 200 amino acids and being at least 75% identical to a polypeptide of the same total number of amino acid residues from the C-terminal part of the Bacillus macerans or Bacillus amyloliquefaciens (1,3-1,4)-β-glucanase as given in Tables I and II.

2. A process according to claim 1 wherein the thermostable (1,3-1,4)-β-glucanase comprises an amino acid sequence with the formula
A - M
where A is a polypeptide consisting of 5 to 200 amino acids and being at least 85% identical to a polypeptide of the same total number of amino acid residues from the N-terminal part of the Bacillus amyloliquefaciens or Bacillus macerans (1,3-1,4)-β-glucanase as given in Tables I and II, and M is a polypeptide consisting of 5 to 200 amino acids and being at least 85% identical to a polypeptide of the same total number of amino acid residues from the C-terminal part of the Bacillus macerans or Bacillus amyloliquefaciens (1,3-1,4)-β-glucanase as given in Tables I and II.

3. A process according to claim 2 wherein the thermostable (1,3-1,4)-β-glucanase comprises an amino acid sequence with the formula
A - M
where A is a polypeptide consisting of 5 to 200 amino acids and being at least 90% identical to a polypeptide of the same total number of amino acid residues from the N-terminal part of the Bacillus amyloliquefaciens or Bacillus macerans (1,3-1,4)-β-glucanase as given in Tables I and II, and M is a polypeptide consisting of 5 to 200 amino acids and being at least 90% identical to a polypeptide of the same total number of amino acid residues from the C-terminal part of the Bacillus macerans or Bacillus amyloliquefaciens (1,3-1,4)-β-glucanase as given in Tables I and II.

4. A process according to claim 3 wherein the thermostable (1,3-1,4)-β-glucanase comprises an amino acid sequence with the formula
A - M
where A is a polypeptide consisting of 5 to 200 amino acids and being identical to a polypeptide of the same total number of amino acid residues from the N-terminal part of the Bacillus amyloliquefaciens or Bacillus macerans (1,3-1,4)-β-glucanase as given in Tables I and II, and M is a polypeptide consisting of 5 to 200 amino acids and being identical to a polypeptide of the same total number of amino acid residues of the amino acid residues of the C-terminal part of the Bacillus macerans or Bacillus amyloliquefaciens (1,3-1,4)-β-glucanase as given in Tables I and II.

5. A process according to claim 1 where A is a polypeptide consisting of 16 amino acids which are at least 75%, preferably at least 85%, and in particular at least 90% identical to the amino acid residues of the N-terminal part of the Bacillus amyloliquefaciens (1,3-1,4)-β-glucanase as given in Table I.

6. A process according to claim 1 where A is a polypeptide consisting of 36 amino acids which are at least 75%, preferably at least 85%, and in particular at least 90% identical to the amino acid residues of the N-terminal part of the Bacillus amyloliquefaciens (1,3-1,4)-β-glucanase as given in Table I.

7. A process according to claim 1 where A is a polypeptide consisting of 78 amino acids which are at least 75%, preferably at least 85%, and in particular at least 90% identical to the amino acid residues of the N-terminal part of the Bacillus amyloliquefaciens (1,3-1,4)-β-glucanase as given in Table I.

8. A process according to claim 1 where A is a polypeptide consisting of 107 amino acids which are at least 75%, preferably at least 85%, and in particular at least 90% identical to the amino acid residues of the N-terminal part of the Bacillus amyloliquefaciens (1,3-1,4)-β-glucanase as given in Table I.

9. A process according to claim 1 where A is a polypeptide consisting of 152 amino acids which are at least 75%, preferably at least 85%, and in particular at least 90% identical to the amino acid residues of the N-terminal part of the Bacillus amyloliquefaciens (1,3-1,4)-β-glucanase as given in Table I and II.

10. A process for the production of a thermostable hybrid (1,3-1,4)-β-glucanase according to any of claims 1 to 9 being encoded by a hybrid gene constructed by reciprocal exchange of amino-terminal and carboxy-terminal parts of (1,3-1,4)-β-glucanase genes of Bacillus spp which glucanase retains at least 50% of its activity after 10 minutes or more of incubation in 5-10 mM CaCl₂, 20-40 mM Na-acetate at a pH of 6.0 or lower and at a temperature of 65°C or higher, the incubated solution having an enzyme concentration range from 0.05 mg to 1 mg (1,3-1,4)-β-glucanase per ml, the activity of (1,3-1,4)-β-glucanase being understood as the ability of the enzyme to hydrolyze β-glycosidic linkages in (1,3-1,4)-β-glucans, the hybrid (1,3-1,4)-β-glucanase being more thermostable than any of the glucanases encoded by the parental (1,3-1,4)-β-glucanase genes of Bacillus spp.

11. A process according to claim 10 wherein the incubation time is 15 minutes.

12. A process according to claim 10 wherein the incubation time is 18 minutes.

13. A process according to any of claims 10-12 wherein the incubation temperature is 70°C.

14. A process according to any of claims 10-13 wherein the solution has a concentration range from 0.3 to 1 mg (1,3-1,4)-β-glucanase per ml.

15. A process according to claim 10 which retains at least 85% of its activity after 30 minutes or more of incubation.

16. A process according to claim 15 wherein the incubation time is 60 minutes.

17. A process according to claim 15 wherein the thermostable (1,3-1,4)-β-glucanase retains at least 85% of its activity after 30 minutes or more of incubation in 5-10 mM CaCl₂, 20-40 mM Na-acetate at a pH of 4.1 and at a temperature of 65°C, the incubated solution having an enzyme concentration of 0.1 mg (1,3-1,4)-β-glucanase per ml.

18. A process according to claim 15 wherein the thermostable (1,3-1,4)-β-glucanase retains at least 85% of its activity after 30 minutes or more of incubation in 5-10 mM CaCl₂, 20-40 mM Na-acetate at a pH of 6.0 and at a temperature of 70°C, the incubated solution having an enzyme concentration of 0.1 mg (1,3-1,4)-β-glucanase per ml.

19. A process according to claim 17 or 18 wherein the incubation time is 60 minutes.

20. A process according to any of claims 17-19 wherein the thermostable (1,3-1,4)-β-glucanase retains at least 90% of its activity.

21. A process according to any of claims 10-20 wherein the thermostable (1,3-1,4)-β-glucanase after 10 minutes of incubation at 65°C and at pH 6.0 in crude cell extracts has a relative β-glucanase activity of at least 100%.

22. A process according to claim 21 wherein the thermostable (1,3-1,4)-β-glucanase has a relative β-glucanase activity of 130%.

23. A process according to any of claims 1-22 wherein the thermostable (1,3-1,4)-β-glucanase has a signal peptide linked to the N-terminal end of the enzyme.

24. A process according to claim 23 wherein the signal peptide is derived from a yeast such as a Saccharomyces species.

25. A process according to claim 24 wherein the signal peptide is the signal peptide from yeast α-factor, yeast acid phosphatase or yeast invertase.

26. A process according to claim 23 wherein the signal peptide is at least 75%, preferably at least 85%, more preferably at least 90% identical to the signal peptide of Bacillus amyloliquefaciens at the amino acid level.

27. A process according to claim 10 wherein the thermostable (1,3-1,4)-β-glucanase comprises the following amino acid sequence: 
Gln-Thr-Gly-Gly-Ser-Phe-Phe-Glu-Pro-Phe-Asn-Ser-Tyr-Asn-Ser-Gly-Leu-
Trp-Gln-Lys-Ala-Asp-Gly-Tyr-Ser-Asn-Gly-Asp-Met-Phe-Asn-Cys-Thr-Trp-
Arg-Ala-Asn-Asn-Val-Ser-Met-Thr-Ser-Leu-Gly-Glu-Met-Arg-Leu-Ala-Leu-
Thr-Ser-Pro-Ser-Tyr-Asn-Lys-Phe-Asp-Cys-Gly-Glu-Asn-Arg-Ser-Val-Gln-
Thr-Tyr-Gly-Tyr-Gly-Leu-Tyr-Glu-Val-Arg-Met-Lys-Pro-Ala-Lys-Asn-Thr-
Gly-Ile-Val-Ser-Ser-Phe-Phe-Thr-Tyr-Thr-Gly-Pro-Thr-Glu-Gly-Thr-Pro-
Trp-Asp-Glu-Ile-Asp-Ile-Glu-Phe-Leu-Gly-Lys-Asp-Thr-Thr-Lys-Val-Gln-
 
Phe-Asn-Tyr-Tyr-Thr-Asn-Gly-Val-Gly-Gly-His-Glu-Lys-Val-Ile-Ser-Leu-
Gly-Phe-Asp-Ala-Ser-Lys-Gly-Phe-His-Thr-Tyr-Ala-Phe-Asp-Trp-Gln-Pro-
Gly-Tyr-Ile-Lys-Trp-Tyr-Val-Asp-Gly-Val-Leu-Lys-His-Thr-Ala-Thr-Ala-
Asn-Ile-Pro-Ser-Thr-Pro-Gly-Lys-Ile-Met-Met-Asn-Leu-Trp-Asn-Gly-Thr-
Gly-Val-Asp-Asp-Trp-Leu-Gly-Ser-Tyr-Asn-Gly-Ala-Asn-Pro-Leu-Tyr-Ala-
Glu-Tyr-Asp-Trp-Val-Lys-Tyr-Thr-Ser-Asn
 or analogues thereof.

28. A process according to claim 10 wherein the thermostable (1,3-1,4)-β-glucanase comprises the following amino acid sequence: 
Met-Lys-Arg-Val-Leu-Leu-Ile-Leu-Val-Thr-Gly-Leu-Phe-Met-Ser-Leu-Cys-
Gly-Ile-Thr-Ser-Ser-Val-Ser-Ala-Gln-Thr-Gly-Gly-Ser-Phe-Phe-Glu-Pro-
Phe-Asn-Ser-Tyr-Asn-Ser-Gly-Leu-Trp-Gln-Lys-Ala-Asp-Gly-Tyr-Ser-Asn-
Gly-Asp-Met-Phe-Asn-Cys-Thr-Trp-Arg-Ala-Asn-Asn-Val-Ser-Met-Thr-Ser-
Leu-Gly-Glu-Met-Arg-Leu-Ala-Leu-Thr-Ser-Pro-Ser-Tyr-Asn-Lys-Phe-Asp-
Cys-Gly-Glu-Asn-Arg-Ser-Val-Gln-Thr-Tyr-Gly-Tyr-Gly-Leu-Tyr-Glu-Val-
Arg-Met-Lys-Pro-Ala-Lys-Asn-Thr-Gly-Ile-Val-Ser-Ser-Phe-Phe-Thr-Tyr-
Thr-Gly-Pro-Thr-Glu-Gly-Thr-Pro-Trp-Asp-Glu-Ile-Asp-Ile-Glu-Phe-Leu-
Gly-Lys-Asp-Thr-Thr-Lys-Val-Gln-Phe-Asn-Tyr-Tyr-Thr-Asn-Gly-Val-Gly-
Gly-His-Glu-Lys-Val-Ile-Ser-Leu-Gly-Phe-Asp-Ala-Ser-Lys-Gly-Phe-His-
Thr-Tyr-Ala-Phe-Asp-Trp-Gln-Pro-Gly-Tyr-Ile-Lys-Trp-Tyr-Val-Asp-Gly-
Val-Leu-Lys-His-Thr-Ala-Thr-Ala-Asn-Ile-Pro-Ser-Thr-Pro-Gly-Lys-Ile-
Met-Met-Asn-Leu-Trp-Asn-Gly-Thr-Gly-Val-Asp-Asp-Trp-Leu-Gly-Ser-Tyr-
Asn-Gly-Ala-Asn-Pro-Leu-Tyr-Ala-Glu-Tyr-Asp-Trp-Val-Lys-Tyr-Thr-Ser-
Asn
 or analogues thereof.

29. A process for the preparation of a thermostable (1,3-1,4)-β-glucanase as defined in claim 4 by the use of a DNA fragment comprising a nucleotide sequence encoding the thermostable (1,3-1,4)-β-glucanase.

30. A process according to claim 29 wherein the DNA fragment comprises the following nucleotide sequence: 
30                                                    60
GAATTCAACG AAGAATCGCT GCACTATTAT CGATTCGTCA CCCACTTAAA GTTTTTCGAC
90                                                    120
CAGCGTCTTT TTAACGGCAC ACACATGGAA AGCCAGGACG ATTTTTTACT GGAGACAGTG
150                                                   180
AAAGAAAAGT ATCATCAGGC GTATAAATGC ACGAAGAATA TCCATACCTA CATTGAGAAA
210                                                   240
 
GAGTATGGGC ATAAGCTCAC CAGTGACGAG CTGCTGTATT TAACGATTCA CATAGAAAGG
270 300
ATAGGATTGT TACGGATAAA GCAGGCAAAA GTAGTCAAAC AAGTATAATG AAAGCGCTTT
330 360
CCTCGTATTA ATTGTTTCTT CCATTCATAT CCTATCTGTC TGTGCTGATG GTAGTTTAGG
390 420
TTTGTATTTT TAACAGAAGG ATTATCATTA TTTCGACCGA TGTTCCCTTT GAAAAGGATC
450 480
ATGTATGATC AATAAAGAAA GCGTGTTCAA AAAAGGGGGA ATGCTAACAT GAAACGAGTG
510 540
TTGCTAATTC TTGTCACCGG ATTGTTTATG AGTTTGTGTG GGATCACTTC TAGTGTTTCG
570 600
GCTCAAACAG GCGGATCGTT TTTTGAACCT TTTAACAGCT ATAACTCCGG GTTATGGCAA
630 660
AAAGCTGATG GTTACTCAAA TGGAGATATG TTTAACTGCA CTTGGCGTGC TAATAACGTC
690 720
TCTATGACGT CATTAGGTGA AATGCGTTTG GCGCTGACAA GTCCGTCTTA TAACAAGTTT
750 780
GACTGCGGGG AAAACCGCTG GGTTCAAACA TATGGCTATG GACTTTATGA AGTCAGAATG
810 840
AAACCGGCTA AAAACACAGG GATTGTTTCA TCGTTCTTCA CTTATACAGG TCCAACGGAG
870 900
GGGACTCCTT GGGATGAGAT TGATATCGAA TTTCTAGGAA AAGACACGAC AAAAGTCCAG
930 960
TTTAACTATT ATACCAATGG GGTTGGCGGT CATGAAAAGG TTATCTCTCT TGGCTTTGAT
990 1020
GCATCAAAGG GCTTCCATAC CTATGCTTTC GATTGGCAGC CAGGGTATAT TAAATGGTAT
1050 1080
GTAGACGGTG TTTTGAAACA TACCGCCACC GCGAATATTC CGAGTACGCC AGGCAAAATT
1110 1140
ATGATGAATC TATGGAACGG AACCGGAGTG GATGACTGGT TAGGTTCTTA TAATGGAGCG
1170 1200
AATCCGTTGT ACGCTGAATA TGACTGGGTA AAATATACGA GCAATTAATA TGATTGCAGC
1230
TGGGCATGAG CTTTTTAGTC CACTCCAGGC ATGCAAGCTT
 or an analogue or a subsequence thereof.

31. A process for producing a thermostable (1,3-1,4)-β-glucanase according to claim 10 comprising cultivating a microorganism in which a DNA fragment as defined in claim 29 or 30 has been introduced in such a way that the microorganism is capable of expressing the thermostable (1,3-1,4)-β-glucanase, the cultivation being performed under conditions leading to production of the thermostable (1,3-1,4)-β-glucanase and recovering the (1,3-1,4)-β-glucanase from the culture.

32. A process according to claim 31 wherein the microorganism is a bacterium.

33. A process according to claim 32 wherein the bacterium is a gram-negative bacterium.

34. A process according to claim 33 wherein the gram-negative bacterium is an E. coli strain.

35. A process according to claim 34 wherein the E. coli strain is the E. coli strain harboring plasmid pUC13-H1, deposited in Deutsche Sammlung von Mikroorganismen under Accession No. DSM 5461.

36. A process according to claim 34 wherein the E. coli strain is the E. coli strain harboring plasmid pTZ19R-H3, deposited in Deutsche Sammlung von Mikroorganismen under Accession No. DSM 5790.

37. A process according to claim 34 wherein the E. coli strain is the E. coli strain harboring plasmid pTZ19R-H4, deposited in Deutsche Sammlung von Mikroorganismen under Accession No. DSM 5791.

38. A process according to claim 34 wherein the E. coli strain is the E. coli strain harboring plasmid pTZ19R-H5, deposited in Deutsche Sammlung von Mikroorganismen under Accession No. DSM 5792.

39. A process according to claim 34 wherein the E. coli strain is the E. coli strain harboring plasmid pTZ19R-H6, deposited in Deutsche Sammlung von Mikroorganismen under Accession No. DSM 5793.

40. A process according to claim 31 wherein the microorganism is a yeast.

41. A process according to claim 40 wherein the yeast is a Saccharomyces species.

42. A process according to claim 41 wherein the Saccharomyces species is Saccharomyces cerevisiae.

43. A process for the production of an eukaryotic or prokaryotic organism capable of expressing the DNA fragment as described in claim 29 or 30, the process comprising transforming said organism with the DNA fragment.

44. A process according to claim 43 wherein the organism is a microorganism.

45. A process according to claim 44 wherein the microorganism is a bacterium.

46. A process according to claim 45 wherein the bacterium is a gram-negative bacterium.

47. A process according to claim 46 wherein the bacterium is an E. coli strain.

48. A process according to claim 47 wherein the E. coli strain is harboring plasmid pUC13-H1 which has been deposited in Deutsche Sammlung von Mikroorganismen under Accession No. DSM 5461 and mutants and variants thereof.

49. A process according to claim 47 wherein the E. coli strain is harboring plasmid pTZ19R-H3 which has been deposited in Deutsche Sammlung von Mikroorganismen under Accession No. DSM 5790 and mutants and variants thereof.

50. A process according to claim 47 wherein the E. coli strain is harboring plasmid pTZ19R-H4 which has been deposited in Deutsche Sammlung von Mikroorganismen under Accession No. DSM 5791 and mutants and variants thereof.

51. A process according to claim 47 wherein the E. coli strain is harboring plasmid pTZ19R-H5 which has been deposited in Deutsche Sammlung von Mikroorganismen under Accession No. DSM 5792 and mutants and variants thereof.

52. A process according to claim 47 wherein the E. coli strain is harboring plasmid pTZ19R-H6 which has been deposited in Deutsche Sammlung von Mikroorganismen under Accession No. DSM 5793 and mutants and variants thereof.

53. A process according to claim 44 wherein the microorganism is a yeast.

54. A process according to claim 53 wherein the yeast is a Saccharomyces species.

55. A process according to claim 54 wherein the Saccharomyces species is Saccharomyces cerevisiae.

56. A process according to claim 43 wherein the organism is a plant.

57. A process according to claim 56 wherein the plant is oat, barley, rye, wheat, rice or maize.

58. A process of degrading (1,3-1,4)-β-glucans in a substrate comprising subjecting the substrate to the action of an effective amount of a thermostable (1,3-1,4)-β-glucanase as defined in any of claims 1-22 for a period of time at a temperature of 65°C or higher, the amount of (1,3-1,4)-β-glucanase being at the most 200 µg, preferably at the most 100 µg, more preferably at the most 50 µg, still more preferably at the most 20 µg, and most preferably 15 µg pr. kg of substrate.

59. A process according to claim 58 wherein the substrate comprises unmodified raw grains or parts thereof from barley or oats or other grains.

60. A process according to claim 58 or 59 in which the substrate comprising (1,3-1,4)-β-glucans is mixed with a second substrate originating from maize, rice or wheat comprising a thermostable (1,3-1,4)-β-glucanase as defined in claim 1.

61. A process for the production of beer, characterized in that the wort is subjected to treatment with a thermostable (1,3-1,4)-β-glucanase according to any of claims 1-22 during mashing at a temperature of 65°C or higher and a pH between 4 and 5.5.

62. A process according to claim 61 in which the temperature is 70°C or higher.

63. A process for the production of animal feed comprising β-glucans, characterized in that the feed is supplemented with a thermostable (1,3-1,4)-β-glucanase according to claim 1 in an amount sufficient to obtain a significant degradation of β-glucans in the gastrointestinal tract of an animal fed with the (1,3-1,4)-β-glucanase supplemented feed.

64. A process according to claim 63 wherein the animal feed is pelletized.

## Patentansprüche (Patentansprüche für folgende(n) Vertragsstaat(en): AT, BE, CH, LI, DE, DK, FR, GB, IT, LU, NL, SE)

1. Thermostabile Hybrid-(1,3-1,4)-β-Glucanase, die von einem durch reziproken Austausch der aminoterminalen und carboxyterminalen Teile der (1,3-1,4)-β-Glucanasegene von Bacillus spp. hergestellten Hybridgen codiert wird, wobei die Glucanase mindestens 50 % ihrer Aktivität nach 10minütiger oder längerer Inkubation in 5 bis 10 mM CaCl₂ und 20 bis 40 mM Na-Acetat bei einem pH-Wert von 6,0 oder niedriger und einer Temperatur von 65°C oder höher behält und dabei die inkubierte Lösung eine Enzymkonzentration im Bereich von 0,05 mg bis 1 mg (1,3-1,4)-β-Glucanase pro ml aufweist, und unter Aktivität der (1,3-1,4)-β-Glucanase die Fähigkeit des Enzyms zur Hydrolyse von β-glycosidischen Bindungen in (1,3-1,4)-β-Glucanen verstanden wird, und wobei die Hybrid-(1,3-1,4)-β-Glucanase thermostabiler als andere Glucanasen ist, die von den ursprünglichen (1,3-1,4)-β-Glucanasegenen von Bacillus spp. codiert werden.

2. Thermostabile (1,3-1,4)-β-Glucanase nach Anspruch 1, wobei die Inkubationszeit 15 Minuten beträgt.

3. Thermostabile (1,3-1,4)-β-Glucanase nach Anspruch 1, wobei die Inkubationszeit 18 Minuten beträgt.

4. Thermostabile (1,3-1,4)-β-Glucanase nach einem der Ansprüche 1 bis 3, wobei die Inkubationstemperatur 70°C beträgt.

5. Thermostabile (1,3-1,4)-β-Glucanase nach einem der Ansprüche 1 bis 4, wobei die Lösung eine Konzentration im Bereich von 0,3 bis 1 mg (1,3-1,4)-β-Glucanase pro ml aufweist.

6. Thermostabile (1,3-1,4)-β-Glucanase nach Anspruch 1, die mindestens 85 % ihrer Aktivität nach 30minütiger oder längerer Inkubation behält.

7. Thermostabile (1,3-1,4)-β-Glucanase nach Anspruch 6, wobei die Inkubationszeit 60 Minuten beträgt.

8. Thermostabile (1,3-1,4)-β-Glucanase nach Anspruch 6, die mindestens 85 % ihrer Aktivität nach 30minütiger oder längerer Inkubation in 5 bis 10 mM CaCl₂ und 20 bis 40 mM Na-Acetat bei einem pH-Wert von 4,1 und einer Temperatur von 65°C behält, wobei die inkubierte Lösung eine Enzymkonzentration von 0,1 mg (1,3-1,4)-β-Glucanase pro ml aufweist.

9. Thermostabile (1,3-1,4)-β-Glucanase nach Anspruch 6, die mindestens 85 % ihrer Aktivität nach 30minütiger oder längerer Inkubation in 5 bis 10 mM CaCl₂ und 20 bis 40 mM Na-Acetat bei einem pH-Wert von 6,0 und einer Temperatur von 70°C behält, wobei die inkubierte Lösung eine Enzymkonzentration von 0,1 mg (1,3-1,4)-β-Glucanase pro ml aufweist.

10. Thermostabile (1,3-1,4)-β-Glucanase nach Anspruch 8 oder 9, wobei die Inkubationszeit 60 Minuten beträgt.

11. Thermostabile (1,3-1,4)-β-Glucanase nach einem der Ansprüche 8 bis 10, die mindestens 90 % ihrer Aktivität behält.

12. Thermostabile (1,3-1,4)-β-Glucanase nach einem der Ansprüche 1 bis 11, die nach 10minütiger Inkubation bei 65°C und einem pH-Wert von 6,0 in Rohzellextrakten eine relative β-Glucanaseaktivität von mindestens 100 % hat.

13. Thermostabile (1,3-1,4)-β-Glucanase nach Anspruch 12, die eine relative β-Glucanaseaktivität von 130 % hat.

14. Thermostabile (1,3-1,4)-β-Glucanase nach einem der Ansprüche 1 bis 13, umfassend eine Aminosäuresequenz mit der Formel
A - M
wobei A ein Polypeptid ist, das aus 5 bis 200 Aminosäuren besteht und zu mindestens 75 % identisch ist mit einem Polypeptid mit der gleichen Gesamtzahl an Aminosäureresten im N-terminalen Teil der (1,3-1,4)-β-Glucanase von Bacillus amyloliquefaciens oder Bacillus macerans, wie es in den Tabellen I und II dargestellt ist, und M ein Polypeptid ist, das aus 5 bis 200 Aminosäuren besteht und zu mindestens 75 % identisch ist mit einem Polypeptid mit der gleichen Gesamtzahl an Aminosäureresten im C-terminalen Teil der (1,3-1,4)-β-Glucanase von Bacillus amyloliquefaciens oder Bacillus macerans, wie es in den Tabellen I und II dargestellt ist.

15. Thermostabile (1,3-1,4)-β-Glucanase nach Anspruch 14, umfassend eine Aminosäuresequenz mit der Formel
A - M
wobei A ein Polypeptid ist, das aus 5 bis 200 Aminosäuren besteht und zu mindestens 85 % identisch ist mit einem Polypeptid mit der gleichen Gesamtzahl an Aminosäureresten im N-terminalen Teil der (1,3-1,4)-β-Glucanase von Bacillus amyloliquefaciens oder Bacillus macerans, wie es in den Tabellen I und II dargestellt ist, und M ein Polypeptid ist, das aus 5 bis 200 Aminosäuren besteht und zu mindestens 85 % identisch ist mit einem Polypeptid mit der gleichen Gesamtzahl an Aminosäureresten im C-terminalen Teil der (1,3-1,4)-β-Glucanase von Bacillus amyloliquefaciens oder Bacillus macerans, wie es in den Tabellen I und II dargestellt ist.

16. Thermostabile (1,3-1,4)-β-Glucanase nach Anspruch 15, umfassend eine Aminosäuresequenz mit der Formel
A - M
wobei A ein Polypeptid ist, das aus 5 bis 200 Aminosäuren besteht und zu mindestens 90 % identisch ist mit einem Polypeptid mit der gleichen Gesamtzahl an Aminosäureresten im N-terminalen Teil der (1,3-1,4)-β-Glucanase von Bacillus amyloliquefaciens oder Bacillus macerans, wie es in den Tabellen I und II dargestellt ist, und M ein Polypeptid ist, das aus 5 bis 200 Aminosäuren besteht und zu mindestens 90 % identisch ist mit einem Polypeptid mit der gleichen Gesamtzahl an Aminosäureresten im C-terminalen Teil der (1,3-1,4)-β-Glucanase von Bacillus amyloliquefaciens oder Bacillus macerans, wie es in den Tabellen I und II dargestellt ist.

17. Thermostabile (1,3-1,4)-β-Glucanase nach Anspruch 16, umfassend eine Aminosäuresequenz mit der Formel
A - M
wobei A ein Polypeptid ist, das aus 5 bis 200 Aminosäuren besteht und identisch ist mit einem Polypeptid mit der gleichen Gesamtzahl an Aminosäureresten im N-terminalen Teil der (1,3-1,4)-β-Glucanase von Bacillus amyloliquefaciens oder Bacillus macerans, wie es in den Tabellen I und II dargestellt ist, und M ein Polypeptid ist, das aus 5 bis 200 Aminosäuren besteht und identisch ist mit einem Polypeptid mit der gleichen Gesamtzahl an Aminosäureresten des C-terminalen Teils der (1,3-1,4)-β-Glucanase von Bacillus amyloliquefaciens oder Bacillus macerans, wie es in den Tabellen I und II dargestellt ist.

18. Thermostabile (1,3-1,4)-β-Glucanase nach Anspruch 14, wobei A ein Polypeptid ist, das aus 16 Aminosäuren besteht, die zu mindestens 75 %, vorzugsweise zu mindestens 85 % und insbesondere zu mindestens 90 % identisch mit den Aminosäureresten des N-terminalen Teils der (1,3-1,4)-β-Glucanase von Bacillus amyloliquefaciens sind, wie es in Tabelle I dargestellt ist.

19. Thermostabile (1,3-1,4)-β-Glucanase nach Anspruch 14, wobei A ein Polypeptid ist, das aus 36 Aminosäuren besteht, die zu mindestens 75 %, vorzugsweise zu mindestens 85 % und insbesondere zu mindestens 90 % identisch mit den Aminosäureresten des N-terminalen Teils der (1,3-1,4)-β-Glucanase von Bacillus amyloliquefaciens sind, wie es in Tabelle I dargestellt ist.

20. Thermostabile (1,3-1,4)-β-Glucanase nach Anspruch 14, wobei A ein Polypeptid ist, das aus 78 Aminosäuren besteht, die zu mindestens 75 %, vorzugsweise zu mindestens 85 % und insbesondere zu mindestens 90 % identisch mit den Aminosäureresten des N-terminalen Teils der (1,3-1,4)-β-Glucanase von Bacillus amyloliquefaciens sind, wie es in Tabelle I dargestellt ist.

21. Thermostabile (1,3-1,4)-β-Glucanase nach Anspruch 14, wobei A ein Polypeptid ist, das aus 107 Aminosäuren besteht, die zu mindestens 75 %, vorzugsweise zu mindestens 85 % und insbesondere zu mindestens 90 % identisch mit den Aminosäureresten des N-terminalen Teils der (1,3-1,4)-β-Glucanase von Bacillus amyloliquefaciens sind, wie es in Tabelle I dargestellt ist.

22. Thermostabile (1,3-1,4)-β-Glucanase nach Anspruch 14, wobei A ein Polypeptid ist, das aus 152 Aminosäuren besteht, die zu mindestens 75 %, vorzugsweise zu mindestens 85 % und insbesondere zu mindestens 90 % identisch mit den Aminosäureresten des N-terminalen Teils der (1,3-1,4)-β-Glucanase von Bacillus amyloliquefaciens sind, wie es in Tabellen I und II dargestellt ist.

23. Thermostabile (1,3-1,4)-β-Glucanase nach einem der Ansprüche 1 bis 22, die ein an das N-terminale Ende des Enzyms gebundenes Signalpeptid besitzt.

24. Thermostabile (1,3-1,4)-β-Glucanase nach Anspruch 23, die ein von einer Hefe, wie einer Saccharomyces-Art, stammendes Signalpeptid besitzt.

25. Thermostabile (1,3-1,4)-β-Glucanase nach Anspruch 24, die ein Signalpeptid besitzt, das das Signalpeptid vom α-Faktor von Hefe, von saurer Phosphatase von Hefe oder von Invertase von Hefe ist.

26. Thermostabile (1,3-1,4)-β-Glucanase nach Anspruch 23, die ein Signalpeptid besitzt, das auf Aminosäureebene zu mindestens 75 %, vorzugsweise zu mindestens 85 % und stärker bevorzugt zu mindestens 90 % identisch mit dem Signalpeptid von Bacillus amyloliquefaciens ist.

27. Thermostabile (1,3-1,4)-β-Glucanase nach Anspruch 1, die die folgende Aminosäuresequenz umfaßt: 
Gln-Thr-Gly-Gly-Ser-Phe-Phe-Glu-Pro-Phe-Asn-Ser-Tyr-Asn-Ser-Gly-Leu-
Trp-Gln-Lys-Ala-Asp-Gly-Tyr-Ser-Asn-Gly-Asp-Met-Phe-Asn-Cys-Thr-Trp-
Arg-Ala-Asn-Asn-Val-Ser-Met-Thr-Ser-Leu-Gly-Glu-Met-Arg-Leu-Ala-Leu-
Thr-Ser-Pro-Ser-Tyr-Asn-Lys-Phe-Asp-Cys-Gly-Glu-Asn-Arg-Ser-Val-Gln-
Thr-Tyr-Gly-Tyr-Gly-Leu-Tyr-Glu-Val-Arg-Met-Lys-Pro-Ala-Lys-Asn-Thr-
Gly-Ile-Val-Ser-Ser-Phe-Phe-Thr-Tyr-Thr-Gly-Pro-Thr-Glu-Gly-Thr-Pro-
Trp-Asp-Glu-Ile-Asp-Ile-Glu-Phe-Leu-Gly-Lys-Asp-Thr-Thr-Lys-Val-Gln-
Phe-Asn-Tyr-Tyr-Thr-Asn-Gly-Val-Gly-Gly-His-Glu-Lys-Val-Ile-Ser-Leu-
Gly-Phe-Asp-Ala-Ser-Lys-Gly-Phe-His-Thr-Tyr-Ala-Phe-Asp-Trp-Gln-Pro-
Gly-Tyr-Ile-Lys-Trp-Tyr-Val-Asp-Gly-Val-Leu-Lys-His-Thr-Ala-Thr-Ala-
Asn-Ile-Pro-Ser-Thr-Pro-Gly-Lys-Ile-Met-Met-Asn-Leu-Trp-Asn-Gly-Thr-
Gly-Val-Asp-Asp-Trp-Leu-Gly-Ser-Tyr-Asn-Gly-Ala-Asn-Pro-Leu-Tyr-Ala-
Glu-Tyr-Asp-Trp-Val-Lys-Tyr-Thr-Ser-Asn
 oder Analoge davon.

28. Thermostabile (1,3-1,4)-β-Glucanase nach Anspruch 1, die die folgende Aminosäuresequenz umfaßt: 
Met-Lys-Arg-Val-Leu-Leu-Ile-Leu-Val-Thr-Gly-Leu-Phe-Met-Ser-Leu-Cys-
Gly-Ile-Thr-Ser-Ser-Val-Ser-Ala-Gln-Thr-Gly-Gly-Ser-Phe-Phe-Glu-Pro-
Phe-Asn-Ser-Tyr-Asn-Ser-Gly-Leu-Trp-Gln-Lys-Ala-Asp-Gly-Tyr-Ser-Asn-
Gly-Asp-Met-Phe-Asn-Cys-Thr-Trp-Arg-Ala-Asn-Asn-Val-Ser-Met-Thr-Ser-
Leu-Gly-Glu-Met-Arg-Leu-Ala-Leu-Thr-Ser-Pro-Ser-Tyr-Asn-Lys-Phe-Asp-
Cys-Gly-Glu-Asn-Arg-Ser-Val-Gln-Thr-Tyr-Gly-Tyr-Gly-Leu-Tyr-Glu-Val-
Arg-Met-Lys-Pro-Ala-Lys-Asn-Thr-Gly-Ile-Val-Ser-Ser-Phe-Phe-Thr-Tyr-
Thr-Gly-Pro-Thr-Glu-Gly-Thr-Pro-Trp-Asp-Glu-Ile-Asp-Ile-Glu-Phe-Leu-
Gly-Lys-Asp-Thr-Thr-Lys-Val-Gln-Phe-Asn-Tyr-Tyr-Thr-Asn-Gly-Val-Gly-
Gly-His-Glu-Lys-Val-Ile-Ser-Leu-Gly-Phe-Asp-Ala-Ser-Lys-Gly-Phe-His-
Thr-Tyr-Ala-Phe-Asp-Trp-Gln-Pro-Gly-Tyr-Ile-Lys-Trp-Tyr-Val-Asp-Gly-
Val-Leu-Lys-His-Thr-Ala-Thr-Ala-Asn-Ile-Pro-Ser-Thr-Pro-Gly-Lys-Ile-
Met-Met-Asn-Leu-Trp-Asn-Gly-Thr-Gly-Val-Asp-Asp-Trp-Leu-Gly-Ser-Tyr-
Asn-Gly-Ala-Asn-Pro-Leu-Tyr-Ala-Glu-Tyr-Asp-Trp-Val-Lys-Tyr-Thr-Ser-
Asn
 oder Analoge davon.

29. DNA-Fragment, umfassend eine Nucleotidsequenz, die die thermostabile (1,3-1,4)-β-Glucanase nach Anspruch 17 codiert.

30. DNA-Fragment nach Anspruch 29, die die folgende Nucleotidsequenz umfaßt: 
30 60
GAATTCAACG AAGAATCGCT GCACTATTAT CGATTCGTCA CCCACTTAAA GTTTTTCGAC
90 120
CAGCGTCTTT TTAACGGCAC ACACATGGAA AGCCAGGACG ATTTTTTACT GGAGACAGTG
150 180
AAAGAAAAGT ATCATCAGGC GTATAAATGC ACGAAGAATA TCCATACCTA CATTGAGAAA
210 240
GAGTATGGGC ATAAGCTCAC CAGTGACGAG CTGCTGTATT TAACGATTCA CATAGAAAGG
270 300
ATAGGATTGT TACGGATAAA GCAGGCAAAA GTAGTCAAAC AAGTATAATG AAAGCGCTTT
330 360
CCTCGTATTA ATTGTTTCTT CCATTCATAT CCTATCTGTC TGTGCTGATG GTAGTTTAGG
390 420
TTTGTATTTT TAACAGAAGG ATTATCATTA TTTCGACCGA TGTTCCCTTT GAAAAGGATC
450 480
ATGTATGATC AATAAAGAAA GCGTGTTCAA AAAAGGGGGA ATGCTAACAT GAAACGAGTG
510 540
TTGCTAATTC TTGTCACCGG ATTGTTTATG AGTTTGTGTG GGATCACTTC TAGTGTTTCG
570 600
GCTCAAACAG GCGGATCGTT TTTTGAACCT TTTAACAGCT ATAACTCCGG GTTATGGCAA
630 660
AAAGCTGATG GTTACTCAAA TGGAGATATG TTTAACTGCA CTTGGCGTGC TAATAACGTC
 
690 720
TCTATGACGT CATTAGGTGA AATGCGTTTG GCGCTGACAA GTCCGTCTTA TAACAAGTTT
750 780
GACTGCGGGG AAAACCGCTG GGTTCAAACA TATGGCTATG GACTTTATGA AGTCAGAATG
810 840
AAACCGGCTA AAAACACAGG GATTGTTTCA TCGTTCTTCA CTTATACAGG TCCAACGGAG
870 900
GGGACTCCTT GGGATGAGAT TGATATCGAA TTTCTAGGAA AAGACACGAC AAAAGTCCAG
930 960
TTTAACTATT ATACCAATGG GGTTGGCGGT CATGAAAAGG TTATCTCTCT TGGCTTTGAT
990 1020
GCATCAAAGG GCTTCCATAC CTATGCTTTC GATTGGCAGC CAGGGTATAT TAAATGGTAT
1050 1080
GTAGACGGTG TTTTGAAACA TACCGCCACC GCGAATATTC CGAGTACGCC AGGCAAAATT
1110 1140
ATGATGAATC TATGGAACGG AACCGGAGTG GATGACTGGT TAGGTTCTTA TAATGGAGCG
1170 1200
AATCCGTTGT ACGCTGAATA TGACTGGGTA AAATATACGA GCAATTAATA TGATTGCAGC
1230
TGGGCATGAG CTTTTTAGTC CACTCCAGGC ATGCAAGCTT
 oder ein Analog oder eine Subsequenz davon.

31. Verfahren zur Herstellung einer thermostabilen (1,3-1,4)-β-Glucanase nach Anspruch 1, umfassend die Züchtung eines Mikroorganismus, in den ein DNA-Fragment nach Anspruch 29 oder 30 so eingeführt wurde, daß der Mikroorganismus in der Lage ist, die thermostabile (1,3-1,4)-β-Glucanase zu exprimieren, wobei die Züchtung unter Bedingungen durchgeführt wird, die zur Produktion der thermostabilen (1,3-1,4)-β-Glucanase und Gewinnung der (1,3-1,4)-β-Glucanase aus der Kultur führt.

32. Verfahren nach Anspruch 31, wobei der Mikroorganismus ein Bakterium ist.

33. Verfahren nach Anspruch 32, wobei das Bakterium ein gram-negatives Bakterium ist.

34. Verfahren nach Anspruch 33, wobei das gram-negative Bakterium ein E. coli-Stamm ist.

35. Verfahren nach Anspruch 34, wobei der E. coli-Stamm ein das Plasmid pUC13-H1 enthaltender E. coli-Stamm ist und das Plasmid bei der Deutschen Sammlung von Mikroorganismen unter der Hinterlegungsnummer DSM 5461 hinterlegt ist.

36. Verfahren nach Anspruch 34, wobei der E. coli-Stamm ein das Plasmid pTZ19R-H3 enthaltender E. coli-Stamm ist und das Plasmid bei der Deutschen Sammlung von Mikroorganismen unter der Hinterlegungsnummer DSM 5790 hinterlegt ist.

37. Verfahren nach Anspruch 34, wobei der E. coli-Stamm ein das Plasmid pTZ19R-H4 enthaltender E. coli-Stamm ist und das Plasmid bei der Deutschen Sammlung von Mikroorganismen unter der Hinterlegungsnummer DSM 5791 hinterlegt ist.

38. Verfahren nach Anspruch 34, wobei der E. coli-Stamm ein das Plasmid pTZ19R-H5 enthaltender E. coli-Stamm ist und das Plasmid bei der Deutschen Sammlung von Mikroorganismen unter der Hinterlegungsnummer DSM 5792 hinterlegt ist.

39. Verfahren nach Anspruch 34, wobei der E. coli-Stamm ein das Plasmid pTZ19R-H6 enthaltender E. coli-Stamm ist und das Plasmid bei der Deutschen Sammlung von Mikroorganismen unter der Hinterlegungsnummer DSM 5793 hinterlegt ist.

40. Verfahren nach Anspruch 31, wobei der Mikroorganismus eine Hefe ist.

41. Verfahren nach Anspruch 40, wobei die Hefe eine Saccharomyces-Art ist.

42. Verfahren nach Anspruch 41, wobei die Saccharomyces-Art Saccharomyces cerevisiae ist.

43. Eukaryontischer oder prokaryontischer Organismus, der mit dem DNA-Fragment nach Anspruch 29 oder 30 transformiert ist und dieses exprimieren kann.

44. Organismus nach Anspruch 43, der ein Mikroorganismus ist.

45. Mikroorganismus nach Anspruch 44, der ein Bakterium ist.

46. Bakterium nach Anspruch 45, das ein gram-negatives Bakterium ist.

47. Bakterium nach Anspruch 46, das ein E. coli-Stamm ist.

48. E. coli-Stamm, der das Plasmid pUC13-H1 enthält, das bei der Deutschen Sammlung von Mikroorganismen unter der Hinterlegungsnummer DSM 5461 hinterlegt ist, und Mutanten und Varianten davon.

49. E. coli-Stamm, der das Plasmid pTZ19R-H3 enthält, das bei der Deutschen Sammlung von Mikroorganismen unter der Hinterlegungsnummer DSM 5790 hinterlegt ist, und Mutanten und Varianten davon.

50. E. coli-Stamm, der das Plasmid pTZ19R-H4 enthält, das bei der Deutschen Sammlung von Mikroorganismen unter der Hinterlegungsnummer DSM 5791 hinterlegt ist, und Mutanten und Varianten davon.

51. E. coli-Stamm, der das Plasmid pTZ19R-H5 enthält, das bei der Deutschen Sammlung von Mikroorganismen unter der Hinterlegungsnummer DSM 5792 hinterlegt ist, und Mutanten und Varianten davon.

52. E. coli-Stamm, der das Plasmid pTZ19R-H6 enthält, das bei der Deutschen Sammlung von Mikroorganismen unter der Hinterlegungsnummer DSM 5793 hinterlegt ist, und Mutanten und Varianten davon.

53. Mikroorganismus nach Anspruch 44, der eine Hefe ist.

54. Hefe nach Anspruch 53, die eine Saccharomyces-Art ist.

55. Saccharomyces-Art nach Anspruch 54, die Saccharomyces cerevisiae ist.

56. Organismus nach Anspruch 43, der eine Pflanze ist.

57. Pflanze nach Anspruch 56, die Hafer, Gerste, Roggen, Weizen, Reis oder Mais ist.

58. Verfahren zum Abbau von (1,3-1,4)-β-Glucanen in einem Substrat, umfassend das Einwirken einer wirksamen Menge an thermostabiler (1,3-1,4)-β-Glucanase nach einem der Ansprüche 1 bis 22 für einen Zeitraum bei einer Temperatur von 65°C oder höher auf das Substrat, wobei die Menge an (1,3-1,4)-β-Glucanase höchstens 200 µg, bevorzugt höchstens 100 µg, stärker bevorzugt höchstens 50 µg, noch stärker bevorzugt höchstens 20 µg und am stärksten bevorzugt 15 µg pro kg Substrat beträgt.

59. Verfahren nach Anspruch 58, wobei das Substrat nicht-modifizierte, unbehandelte Körner oder Teile davon von Gerste oder Hafer oder anderem Getreide umfaßt.

60. Verfahren nach Anspruch 58 oder 59, wobei das (1,3-1,4)-β-Glucane enthaltende Substrat mit einem zweiten Substrat gemischt wird, das aus eine thermostabile (1,3-1,4)-β-Glucanase nach Anspruch 1 enthaltendem Mais, Reis oder Weizen stammt.

61. Verfahren zur Herstellung von Bier, dadurch gekennzeichnet, daß die Würze der Behandlung mit einer thermostabilen (1,3-1,4)-β-Glucanase nach einem der Ansprüche 1 bis 22 während des Zerstampfens bei einer Temperatur von 65°C oder höher und einem pH-Wert zwischen 4 und 5,5 unterzogen wird.

62. Verfahren nach Anspruch 61, bei dem die Temperatur 70°C oder mehr beträgt.

63. Verfahren zur Herstellung von β-Glucane enthaltender Tiernahrung, dadurch gekennzeichnet, daß die Tiernahrung mit einer thermostabilen (1,3-1,4)-β-Glucanase nach Anspruch 1 in einer Menge versetzt wird, die zum Erhalt eines signifikanten Abbaus von β-Glucanen im Gastrointestinaltrakt eines Tieres geeignet ist, das mit mit (1,3-1,4)-β-Glucanase versetzter Tiernahrung gefüttert wurde.

64. Verfahren nach Anspruch 63, wobei die Tiernahrung zu Pellets geformt ist.

## Patentansprüche (Patentansprüche für folgende(n) Vertragsstaat(en): ES)

1. Verfahren zur Herstellung einer thermostabilen (1,3-1,4)-β-Glucanase, umfassend eine Aminosäuresequenz mit der Formel
A - M
in der A ein Polypeptid ist, das aus 5 bis 200 Aminosäuren besteht und zu mindestens 75 % identisch ist mit einem Polypeptid mit der gleichen Gesamtzahl an Aminosäureresten im N-terminalen Teil der (1,3-1,4)-β-Glucanase von Bacillus amyloliquefaciens oder Bacillus macerans, wie es in den Tabellen I und II dargestellt ist, und M ein Polypeptid ist, das aus 5 bis 200 Aminosäuren besteht und zu mindestens 75 % identisch ist mit einem Polypeptid mit der gleichen Gesamtzahl an Aminosäureresten im C-terminalen Teil der (1,3-1,4)-β-Glucanase von Bacillus amyloliquefaciens oder Bacillus macerans, wie es in den Tabellen I und II dargestellt ist.

2. Verfahren nach Anspruch 1, wobei die thermostabile (1,3-1,4)-β-Glucanase eine Aminosäuresequenz mit der Formel
A - M
umfaßt, in der A ein Polypeptid ist, das aus 5 bis 200 Aminosäuren besteht und zu mindestens 85 % identisch ist mit einem Polypeptid mit der gleichen Gesamtzahl an Aminosäureresten im N-terminalen Teil der (1,3-1,4)-β-Glucanase von Bacillus amyloliquefaciens oder Bacillus macerans, wie es in den Tabellen I und II dargestellt ist, und M ein Polypeptid ist, das aus 5 bis 200 Aminosäuren besteht, und zu mindestens 85 % identisch ist mit einem Polypeptid mit der gleichen Gesamtzahl an Aminosäureresten im C-terminalen Teil der (1,3-1,4)-β-Glucanase von Bacillus amyloliquefaciens oder Bacillus macerans, wie es in den Tabellen I und II dargestellt ist.

3. Verfahren nach Anspruch 2, wobei die thermostabile (1,3-1,4)-β-Glucanase eine Aminosäuresequenz mit der Formel
A - M
umfaßt, in der A ein Polypeptid ist, das aus 5 bis 200 Aminosäuren besteht und zu mindestens 90 % identisch ist mit einem Polypeptid mit der gleichen Gesamtzahl an Aminosäureresten im N-terminalen Teil der (1,3-1,4)-β-Glucanase von Bacillus amyloliquefaciens oder Bacillus macerans, wie es in den Tabellen I und II dargestellt ist, und M ein Polypeptid ist, das aus 5 bis 200 Aminosäuren besteht, und zu mindestens 90 % identisch ist mit einem Polypeptid mit der gleichen Gesamtzahl an Aminosäureresten im C-terminalen Teil der (1,3-1,4)-β-Glucanase von Bacillus amyloliquefaciens oder Bacillus macerans, wie es in den Tabellen I und II dargestellt ist.

4. Verfahren nach Anspruch 3, wobei die thermostabile (1,3-1,4)-β-Glucanase eine Aminosäuresequenz mit der Formel
A - M
umfaßt, in der A ein Polypeptid ist, das aus 5 bis 200 Aminosäuren besteht und identisch ist mit einem Polypeptid mit der gleichen Gesamtzahl an Aminosäureresten im N-terminalen Teil der (1,3-1,4)-β-Glucanase von Bacillus amyloliquefaciens oder Bacillus macerans, wie es in den Tabellen I und II dargestellt ist, und M ein Polypeptid ist, das aus 5 bis 200 Aminosäuren besteht und identisch ist mit einem Polypeptid mit der gleichen Gesamtzahl an Aminosäureresten des C-terminalen Teils der (1,3-1,4)-**β**-Glucanase von Bacillus amyloliquefaciens oder Bacillus macerans, wie es in den Tabellen I und II dargestellt ist.

5. Verfahren nach Anspruch 1, wobei A ein Polypeptid ist, das aus 16 Aminosäuren besteht, die zu mindestens 75 %, vorzugsweise zu mindestens 85 % und insbesondere zu mindestens 90 % identisch mit den Aminosäureresten des N-terminalen Teils der (1,3-1,4)-β-Glucanase von Bacillus amyloliquefaciens sind, wie es in Tabelle I dargestellt ist.

6. Verfahren nach Anspruch 1, wobei A ein Polypeptid ist, das aus 36 Aminosäuren besteht, die zu mindestens 75 %, vorzugsweise zu mindestens 85 % und insbesondere zu mindestens 90 % identisch mit den Aminosäureresten des N-terminalen Teils der (1,3-1,4)-β-Glucanase von Bacillus amyloliquefaciens sind, wie es in Tabelle I dargestellt ist.

7. Verfahren nach Anspruch 1, wobei A ein Polypeptid ist, das aus 78 Aminosäuren besteht, die zu mindestens 75 %, vorzugsweise zu mindestens 85 % und insbesondere zu mindestens 90 % identisch mit den Aminosäureresten des N-terminalen Teils der (1,3-1,4)-β-Glucanase von Bacillus amyloliquefaciens sind, wie es in Tabelle I dargestellt ist.

8. Verfahren nach Anspruch 1, wobei A ein Polypeptid ist, das aus 107 Aminosäuren besteht, die zu mindestens 75 %, vorzugsweise zu mindestens 85 % und insbesondere zu mindestens 90 % identisch mit den Aminosäureresten des N-terminalen Teils der (1,3-1,4)-β-Glucanase von Bacillus amyloliquefaciens sind, wie es in Tabelle I dargestellt ist.

9. Verfahren nach Anspruch 1, wobei A ein Polypeptid ist, das aus 152 Aminosäuren besteht, die zu mindestens 75 %, vorzugsweise zu mindestens 85 % und insbesondere zu mindestens 90 % identisch mit den Aminosäureresten des N-terminalen Teils der (1,3-1,4)-β-Glucanase von Bacillus amyloliquefaciens sind, wie es in Tabellen I und II dargestellt ist.

10. Verfahren zur Herstellung einer thermostabilen Hybrid-(1,3-1,4)-β-Glucanase nach einem der Ansprüche 1 bis 9, die von einem durch reziproken Austausch der aminoterminalen und carboxyterminalen Teile der (1,3-1,4)-β-Glucanasegene von Bacillus spp. hergestellten Hybridgen codiert wird, wobei die Glucanase mindestens 50 % ihrer Aktivität nach 10minütiger oder längerer Inkubation in 5 bis 10 mM CaCl₂ und 20 bis 40 mM Na-Acetat bei einem pH-Wert von 6,0 oder niedriger und einer Temperatur von 65°C oder höher behält und dabei die inkubierte Lösung eine Enzymkonzentration im Bereich von 0,05 mg bis 1 mg (1,3-1,4)-β-Glucanase pro ml aufweist, und unter Aktivität der (1,3-1,4)-β-Glucanase die Fähigkeit des Enzyms zur Hydrolyse von β-glycosidischen Bindungen in (1,3-1,4)-β-Glucanen verstanden wird, und wobei die Hybrid-(1,3-1,4)-β-Glucanase thermostabiler als andere Glucanasen ist, die von den ursprünglichen (1,3-1,4)-β-Glucanasegenen von Bacillus spp. codiert werden.

11. Verfahren nach Anspruch 10, wobei die Inkubationszeit 15 Minuten beträgt.

12. Verfahren nach Anspruch 10, wobei die Inkubationszeit 18 Minuten beträgt.

13. Verfahren nach einem der Ansprüche 10 bis 12, wobei die Inkubationstemperatur 70°C beträgt.

14. Verfahren nach einem der Ansprüche 10 bis 13, wobei die Lösung eine Konzentration im Bereich von 0,3 bis 1 mg (1,3-1,4)-β-Glucanase pro ml aufweist.

15. Verfahren nach Anspruch 10, wobei mindestens 85 % der Aktivität nach 30minütiger oder längerer Inkubation erhalten bleiben.

16. Verfahren nach Anspruch 15, wobei die Inkubationszeit 60 Minuten beträgt.

17. Verfahren nach Anspruch 15, wobei die thermostabile (1,3-1,4)-β-Glucanase mindestens 85 % ihrer Aktivität nach 30minütiger oder längerer Inkubation in 5 bis 10 mM CaCl₂ und 20 bis 40 mM Na-Acetat bei einem pH-Wert von 4,1 und einer Temperatur von 65°C behält, wobei die inkubierte Lösung eine Enzymkonzentration von 0,1 mg (1,3-1,4)-β-Glucanase pro ml aufweist.

18. Verfahren nach Anspruch 15, wobei die thermostabile (1,3-1,4)-β-Glucanase mindestens 85 % ihrer Aktivität nach 30minütiger oder längerer Inkubation in 5 bis 10 mM CaCl₂ und 20 bis 40 mM Na-Acetat bei einem pH-Wert von 6,0 und einer Temperatur von 70°C behält, wobei die inkubierte Lösung eine Enzymkonzentration von 0,1 mg (1,3-1,4)-β-Glucanase pro ml aufweist.

19. Verfahren nach Anspruch 17 oder 18, wobei die Inkubationszeit 60 Minuten beträgt.

20. Verfahren nach einem der Ansprüche 17 bis 19, wobei die thermostabile (1,3-1,4)-β-Glucanase mindestens 90 % ihrer Aktivität behält.

21. Verfahren nach einem der Ansprüche 10 bis 20, wobei die thermostabile (1,3-1,4)-β-Glucanase nach 10minütiger Inkubation bei 65°C und einem pH-Wert von 6,0 in Rohzellextrakten eine relative β-Glucanaseaktivität von mindestens 100 % hat.

22. Verfahren nach Anspruch 21, wobei die thermostabile (1,3-1,4)-β-Glucanase eine relative β-Glucanaseaktivität von 130 % hat.

23. Verfahren nach einem der Anspruche 1 bis 22, wobei die thermostabile (1,3-1,4)-β-Glucanase ein an das N-terminale Ende des Enzyms gebundenes Signalpeptid besitzt.

24. Verfahren nach Anspruch 23, wobei das Signalpeptid von einer Hefe, wie einer Saccharomyces-Art, stammt.

25. Verfahren nach Anspruch 24, wobei das Signalpeptid das Signalpeptid vom α-Faktor von Hefe, von saurer Phosphatase von Hefe oder von Invertase von Hefe ist.

26. Verfahren nach Anspruch 23, wobei das Signalpeptid auf Aminosäureebene zu mindestens 75 %, vorzugsweise zu mindestens 85 % und stärker bevorzugt zu mindestens 90 % identisch mit dem Signalpeptid von Bacillus amyloliquefaciens ist.

27. Verfahren nach Anspruch 10, wobei die thermostabile (1,3-1,4)-β-Glucanase die folgende Aminosäuresequenz umfaßt: 
Gln-Thr-Gly-Gly-Ser-Phe-Phe-Glu-Pro-Phe-Asn-Ser-Tyr-Asn-Ser-Gly-Leu-
Trp-Gln-Lys-Ala-Asp-Gly-Tyr-Ser-Asn-Gly-Asp-Met-Phe-Asn-Cys-Thr-Trp-
Arg-Ala-Asn-Asn-Val-Ser-Met-Thr-Ser-Leu-Gly-Glu-Met-Arg-Leu-Ala-Leu-
Thr-Ser-Pro-Ser-Tyr-Asn-Lys-Phe-Asp-Cys-Gly-Glu-Asn-Arg-Ser-Val-Gln-
Thr-Tyr-Gly-Tyr-Gly-Leu-Tyr-Glu-Val-Arg-Met-Lys-Pro-Ala-Lys-Asn-Thr-
Gly-Ile-Val-Ser-Ser-Phe-Phe-Thr-Tyr-Thr-Gly-Pro-Thr-Glu-Gly-Thr-Pro-
Trp-Asp-Glu-Ile-Asp-Ile-Glu-Phe-Leu-Gly-Lys-Asp-Thr-Thr-Lys-Val-Gln-
Phe-Asn-Tyr-Tyr-Thr-Asn-Gly-Val-Gly-Gly-His-Glu-Lys-Val-Ile-Ser-Leu-
Gly-Phe-Asp-Ala-Ser-Lys-Gly-Phe-His-Thr-Tyr-Ala-Phe-Asp-Trp-Gln-Pro-
Gly-Tyr-Ile-Lys-Trp-Tyr-Val-Asp-Gly-Val-Leu-Lys-His-Thr-Ala-Thr-Ala-
Asn-Ile-Pro-Ser-Thr-Pro-Gly-Lys-Ile-Met-Met-Asn-Leu-Trp-Asn-Gly-Thr-
Gly-Val-Asp-Asp-Trp-Leu-Gly-Ser-Tyr-Asn-Gly-Ala-Asn-Pro-Leu-Tyr-Ala-
Glu-Tyr-Asp-Trp-Val-Lys-Tyr-Thr-Ser-Asn
 oder Analoge davon.

28. Verfahren nach Anspruch 10, wobei die thermostabile (1,3-1,4)-β-Glucanase die folgende Aminosäuresequenz umfaßt: 
Met-Lys-Arg-Val-Leu-Leu-Ile-Leu-Val-Thr-Gly-Leu-Phe-Met-Ser-Leu-Cys-
Gly-Ile-Thr-Ser-Ser-Val-Ser-Ala-Gln-Thr-Gly-Gly-Ser-Phe-Phe-Glu-Pro-
Phe-Asn-Ser-Tyr-Asn-Ser-Gly-Leu-Trp-Gln-Lys-Ala-Asp-Gly-Tyr-Ser-Asn-
Gly-Asp-Met-Phe-Asn-Cys-Thr-Trp-Arg-Ala-Asn-Asn-Val-Ser-Met-Thr-Ser-
Leu-Gly-Glu-Met-Arg-Leu-Ala-Leu-Thr-Ser-Pro-Ser-Tyr-Asn-Lys-Phe-Asp-
Cys-Gly-Glu-Asn-Arg-Ser-Val-Gln-Thr-Tyr-Gly-Tyr-Gly-Leu-Tyr-Glu-Val-
Arg-Met-Lys-Pro-Ala-Lys-Asn-Thr-Gly-Ile-Val-Ser-Ser-Phe-Phe-Thr-Tyr-
Thr-Gly-Pro-Thr-Glu-Gly-Thr-Pro-Trp-Asp-Glu-Ile-Asp-Ile-Glu-Phe-Leu-
Gly-Lys-Asp-Thr-Thr-Lys-Val-Gln-Phe-Asn-Tyr-Tyr-Thr-Asn-Gly-Val-Gly-
Gly-His-Glu-Lys-Val-Ile-Ser-Leu-Gly-Phe-Asp-Ala-Ser-Lys-Gly-Phe-His-
Thr-Tyr-Ala-Phe-Asp-Trp-Gln-Pro-Gly-Tyr-Ile-Lys-Trp-Tyr-Val-Asp-Gly-
Val-Leu-Lys-His-Thr-Ala-Thr-Ala-Asn-Ile-Pro-Ser-Thr-Pro-Gly-Lys-Ile-
Met-Met-Asn-Leu-Trp-Asn-Gly-Thr-Gly-Val-Asp-Asp-Trp-Leu-Gly-Ser-Tyr-
Asn-Gly-Ala-Asn-Pro-Leu-Tyr-Ala-Glu-Tyr-Asp-Trp-Val-Lys-Tyr-Thr-Ser-
Asn
 oder Analoge davon.

29. Verfahren zur Herstellung einer thermostabilen (1,31,4)-β-Glucanase gemäß der Definition in Anspruch 4 durch die Verwendung eines DNA-Fragments, umfassend eine Nucleotidsequenz, die die thermostabile (1,3-1,4)-β-Glucanase codiert.

30. Verfahren nach Anspruch 29, wobei das DNA-Fragment die folgende Nucleotidsequenz umfaßt: 
30 60
GAATTCAACG AAGAATCGCT GCACTATTAT CGATTCGTCA CCCACTTAAA GTTTTTCGAC
90 120
CAGCGTCTTT TTAACGGCAC ACACATGGAA AGCCAGGACG ATTTTTTACT GGAGACAGTG
150 180
AAAGAAAAGT ATCATCAGGC GTATAAATGC ACGAAGAATA TCCATACCTA CATTGAGAAA
210 240
GAGTATGGGC ATAAGCTCAC CAGTGACGAG CTGCTGTATT TAACGATTCA CATAGAAAGG
270 300
ATAGGATTGT TACGGATAAA GCAGGCAAAA GTAGTCAAAC AAGTATAATG AAAGCGCTTT
330 360
CCTCGTATTA ATTGTTTCTT CCATTCATAT CCTATCTGTC TGTGCTGATG GTAGTTTAGG
390 420
TTTGTATTTT TAACAGAAGG ATTATCATTA TTTCGACCGA TGTTCCCTTT GAAAAGGATC
450 480
ATGTATGATC AATAAAGAAA GCGTGTTCAA AAAAGGGGGA ATGCTAACAT GAAACGAGTG
 
510 540
TTGCTAATTC TTGTCACCGG ATTGTTTATG AGTTTGTGTG GGATCACTTC TAGTGTTTCG
570 600
GCTCAAACAG GCGGATCGTT TTTTGAACCT TTTAACAGCT ATAACTCCGG GTTATGGCAA
630 660
AAAGCTGATG GTTACTCAAA TGGAGATATG TTTAACTGCA CTTGGCGTGC TAATAACGTC
690 720
TCTATGACGT CATTAGGTGA AATGCGTTTG GCGCTGACAA GTCCGTCTTA TAACAAGTTT
750 780
GACTGCGGGG AAAACCGCTG GGTTCAAACA TATGGCTATG GACTTTATGA AGTCAGAATG
810 840
AAACCGGCTA AAAACACAGG GATTGTTTCA TCGTTCTTCA CTTATACAGG TCCAACGGAG
870 900
GGGACTCCTT GGGATGAGAT TGATATCGAA TTTCTAGGAA AAGACACGAC AAAAGTCCAG
930 960
TTTAACTATT ATACCAATGG GGTTGGCGGT CATGAAAAGG TTATCTCTCT TGGCTTTGAT
990 1020
GCATCAAAGG GCTTCCATAC CTATGCTTTC GATTGGCAGC CAGGGTATAT TAAATGGTAT
1050 1080
GTAGACGGTG TTTTGAAACA TACCGCCACC GCGAATATTC CGAGTACGCC AGGCAAAATT
1110 1140
ATGATGAATC TATGGAACGG AACCGGAGTG GATGACTGGT TAGGTTCTTA TAATGGAGCG
1170 1200
AATCCGTTGT ACGCTGAATA TGACTGGGTA AAATATACGA GCAATTAATA TGATTGCAGC
1230
TGGGCATGAG CTTTTTAGTC CACTCCAGGC ATGCAAGCTT
 oder ein Analog oder eine Subsequenz davon.

31. Verfahren zur Herstellung einer thermostabilen (1,31,4)-β-Glucanase nach Anspruch 10, umfassend die Züchtung eines Mikroorganismus, in den ein DNA-Fragment nach Anspruch 29 oder 30 so eingeführt wurde, daß der Mikroorganismus in der Lage ist, die thermostabile (1,3-1,4)-β-Glucanase zu exprimieren, wobei die Züchtung unter Bedingungen durchgeführt wird, die zur Produktion der thermostabilen (1,3-1,4)-β-Glucanase und Gewinnung der (1,3-1,4)-β-Glucanase aus der Kultur führt.

32. Verfahren nach Anspruch 31, wobei der Mikroorganismus ein Bakterium ist.

33. Verfahren nach Anspruch 32, wobei das Bakterium ein gram-negatives Bakterium ist.

34. Verfahren nach Anspruch 33, wobei das gram-negative Bakterium ein E. coli-Stamm ist.

35. Verfahren nach Anspruch 34, wobei der E. coli-Stamm ein das Plasmid pUC13-H1 enthaltender E. coli-Stamm ist und das Plasmid bei der Deutschen Sammlung von Mikroorganismen unter der Hinterlegungsnummer DSM 5461 hinterlegt ist.

36. Verfahren nach Anspruch 34, wobei der E. coli-Stamm ein das Plasmid pTZ19R-H3 enthaltender E. coli-Stamm ist und das Plasmid bei der Deutschen Sammlung von Mikroorganismen unter der Hinterlegungsnummer DSM 5790 hinterlegt ist.

37. Verfahren nach Anspruch 34, wobei der E. coli-Stamm ein das Plasmid pTZ19R-H4 enthaltender E. coli-Stamm ist und das Plasmid bei der Deutschen Sammlung von Mikroorganismen unter der Hinterlegungsnummer DSM 5791 hinterlegt ist.

38. Verfahren nach Anspruch 34, wobei der E. coli-Stamm ein das Plasmid pTZ19R-H5 enthaltender E. coli-Stamm ist und das Plasmid bei der Deutschen Sammlung von Mikroorganismen unter der Hinterlegungsnummer DSM 5792 hinterlegt ist.

39. Verfahren nach Anspruch 34, wobei der E. coli-Stamm ein das Plasmid pTZ19R-H6 enthaltender E. coli-Stamm ist und das Plasmid bei der Deutschen Sammlung von Mikroorganismen unter der Hinterlegungsnummer DSM 5793 hinterlegt ist.

40. Verfahren nach Anspruch 31, wobei der Mikroorganismus eine Hefe ist.

41. Verfahren nach Anspruch 40, wobei die Hefe eine Saccharomyces-Art ist.

42. Verfahren nach Anspruch 41, wobei die Saccharomyces-Art Saccharomyces cerevisiae ist.

43. Verfahren zur Herstellung eines eukaryontischen oder prokaryontischen Organismus, der das DNA-Fragment nach Anspruch 29 oder 30 exprimieren kann, umfassend die Transformation des Organismus mit dem DNA-Fragment.

44. Verfahren nach Anspruch 43, wobei der Organismus ein Mikroorganismus ist.

45. Verfahren nach Anspruch 44, wobei der Mikroorganismus ein Bakterium ist.

46. Verfahren nach Anspruch 45, wobei das Bakterium ein gram-negatives Bakterium ist.

47. Verfahren nach Anspruch 46, wobei das Bakterium ein E. coli-Stamm ist.

48. Verfahren nach Anspruch 47, wobei der E. coli-Stamm das Plasmid pUC13-H1 enthält, das bei der Deutschen Sammlung von Mikroorganismen unter der Hinterlegungsnummer DSM 5461 hinterlegt ist, und Mutanten und Varianten davon.

49. Verfahren nach Anspruch 47, wobei der E. coli-Stamm das Plasmid pTZ19R-H3 enthält, das bei der Deutschen Sammlung von Mikroorganismen unter der Hinterlegungsnummer DSM 5790 hinterlegt ist, und Mutanten und Varianten davon.

50. Verfahren nach Anspruch 47, wobei der E. coli-Stamm das Plasmid pTZ19R-H4 enthält, das bei der Deutschen Sammlung von Mikroorganismen unter der Hinterlegungsnummer DSM 5791 hinterlegt ist, und Mutanten und Varianten davon.

51. Verfahren nach Anspruch 47, wobei der E. coli-Stamm das Plasmid pTz19R-H5 enthält, das bei der Deutschen Sammlung von Mikroorganismen unter der Hinterlegungsnummer DSM 5792 hinterlegt ist, und Mutanten und Varianten davon.

52. Verfahren nach Anspruch 47, wobei der E. coli-Stamm das Plasmid pTZ19R-H6 enthält, das bei der Deutschen Sammlung von Mikroorganismen unter der Hinterlegungsnummer DSM 5793 hinterlegt ist, und Mutanten und Varianten davon.

53. Verfahren nach Anspruch 44, wobei der Mikroorganismus eine Hefe ist.

54. Verfahren nach Anspruch 53, wobei die Hefe eine Saccharomyces-Art ist.

55. Verfahren nach Anspruch 54, wobei die Saccharomyces-Art Saccharomyces cerevisiae ist.

56. Verfahren nach Anspruch 43, wobei der Organismus eine Pflanze ist.

57. Verfahren nach Anspruch 56, wobei die Pflanze Hafer, Gerste, Roggen, Weizen, Reis oder Mais ist.

58. Verfahren zum Abbau von (1,3-1,4)-β-Glucanen in einem Substrat, umfassend das Einwirken einer wirksamen Menge an thermostabiler (1,3-1,4)-β-Glucanase nach einem der Ansprüche 1 bis 22 für einen Zeitraum bei einer Temperatur von 65°C oder höher auf das Substrat, wobei die Menge an (1,3-1,4)-β-Glucanase höchstens 200 µg, bevorzugt höchstens 100 µg, stärker bevorzugt höchstens 50 µg, noch stärker bevorzugt höchstens 20 µg und am stärksten bevorzugt 15 µg pro kg Substrat beträgt.

59. Verfahren nach Anspruch 58, wobei das Substrat nicht-modifizierte, unbehandelte Körner oder Teile davon von Gerste oder Hafer oder anderem Getreide umfaßt.

60. Verfahren nach Anspruch 58 oder 59, wobei das (1,3-1,4)-β-Glucane enthaltende Substrat mit einem zweiten Substrat gemischt wird, das aus eine thermostabile (1,3-1,4)-β-Glucanase nach Anspruch 1 enthaltendem Mais, Reis oder Weizen stammt.

61. Verfahren zur Herstellung von Bier, dadurch gekennzeichnet, daß die Würze der Behandlung mit einer thermostabilen (1,3-1,4)-β-Glucanase nach einem der Ansprüche 1 bis 22 während des Zerstampfens bei einer Temperatur von 65°C oder höher und einem pH-Wert zwischen 4 und 5,5 unterzogen wird.

62. Verfahren nach Anspruch 61, bei dem die Temperatur 70°C oder mehr beträgt.

63. Verfahren zur Herstellung von β-Glucane enthaltender Tiernahrung, dadurch gekennzeichnet, daß die Tiernahrung mit einer thermostabilen (1,3-1,4)-β-Glucanase nach Anspruch 1 in einer Menge versetzt wird, die zum Erhalt eines signifikanten Abbaus von β-Glucanen im Gastrointestinaltrakt eines Tieres geeignet ist, das mit mit (1,3-1,4)-β-Glucanase versetzter Tiernahrung gefüttert wurde.

64. Verfahren nach Anspruch 63, wobei die Tiernahrung zu Pellets geformt ist.

## Revendications (Revendications pour l'(les) Etat(s) contractant(s) suivant(s): AT, BE, CH, LI, DE, DK, FR, GB, IT, LU, NL, SE)

1. (1,3-1,4)-β-glucanase hybride thermostable encodée par un gène hybride construit par échange réciproque de régions amino-terminales et carboxy-terminales de gènes d'une (1,3-1,4)-β-glucanase du genre Bacillus, laquelle glucanase conserve au moins 50% de son activité au bout de 10 minutes d'incubation ou plus dans du CaCl₂ 5-10 mM, acétate de Na 2040 mM à pH inférieur ou égal à 6,0 et à température supérieure ou égale à 65°C, la concentration en enzyme de la solution mise en incubation étant comprise entre 0,05 mg et 1 mg de (1,3-1,4)-β-glucanase par ml, l'activité de la (1,3-1,4)-β-glucanase étant assimilée à la capacité de l'enzyme à hydrolyser les liaisons β-glycosidiques des (1,3-1,4)-β-glucanes, la (1,3-1,4)-β-glucanase hybride étant plus thermostable qu'une quelconque glucanase codée par les gênes de la (1,3-1,4)-β-glucanase parentaux du genre Bacillus.

2. (1,3-1,4)-β-glucanase thermostable selon la revendication 1, dans laquelle la durée d'incubation est de 15 minutes.

3. (1,3-1,4)-β-glucanase thermostable selon la revendication 1, dans laquelle la durée d'incubation est de 18 minutes.

4. (1,3-1,4)-β-glucanase thermostable selon l'une quelconque des revendications 1 à 3, dans laquelle la température d'incubation est de 70°C.

5. (1,3-1,4)-β-glucanase thermostable selon l'une quelconque des revendications 1 à 4, dans laquelle la concentration de la solution s'échelonne de 0,3 mg à 1 mg de (1,3-1,4)-β-glucanase par ml.

6. (1,3-1,4)-β-glucanase thermostable selon la revendication 1, qui conserve au moins 85% de son activité au bout de 30 minutes d'incubation ou plus.

7. (1,3-1,4)-β-glucanase thermostable selon la revendication 6, dans laquelle la durée d'incubation est de 60 minutes.

8. (1,3-1,4)-β-glucanase thermostable selon la revendication 6, qui conserve au moins 85% de son activité au bout de 30 minutes d'incubation ou plus dans du CaCl₂ 5-10 mM, acétate de Na 20-40 mM à un *p*H de 4,1 et à une température de 65°C, la concentration en enzyme de la solution mise en incubation étant de 0,1 mg de (1,3-1,4)-β-glucanase par ml.

9. (1,3-1,4)-β-glucanase thermostable selon la revendication 6, qui conserve au moins 85% de son activité au bout de 30 minutes d'incubation ou plus dans du CaCl₂ 5-10 mM, acétate de Na 20-40 mM à un pH de 6,0 et à une température de 70°C, la concentration en enzyme de la solution mise en incubation étant de 0,1 mg de (1,3-1,4)-β-glucanase par ml.

10. (1,3-1,4)-β-glucanase thermostable selon la revendication 8 ou 9, dans laquelle la durée d'incubation est de 60 minutes.

11. (1,3-1,4)-β-glucanase thermostable selon l'une quelconque des revendications 8-10 ou 9, qui conserve au moins 90% de son activité.

12. (1,3-1,4)-β-glucanase thermostable selon l'une quelconque des revendications 1 à 11, qui au bout de 10 minutes d'incubation à 65°C et à *p*H 6,0 dans des extraits cellulaires bruts présente une activité β-glucanase relative d'au moins 100%.

13. (1,3-1,4)-β-glucanase thermostable selon la revendication 12, qui présente une activité β-glucanase relative de 130%.

14. (1,3-1,4)-β-glucanase thermostable selon l'une quelconque des revendications 1 à 13, comprenant une séquence d'acides aminés de formule
A - M
où A est un polypeptide constitué de 5 à 200 acides aminés présentant un degré d'homologie d'au moins 75% à un polypeptide comportant au total le même nombre de résidus d'acides aminés provenant de la région N-terminale de la (1,3-1,4)-β-glucanase de Bacillus amyloliquefaciens ou de Bacillus macerans tel qu'il est indiqué aux Tableaux I et II, et M est un polypeptide constitué de 5 à 200 acides aminés présentant un degré d'homologie d'au moins 75% à un polypeptide comportant au total le même nombre de résidus d'acides aminés provenant de la région C-terminale de la (1,3-1,4)-β-glucanase de Bacillus macerans ou de Bacillus amyloliquefaciens tel qu'il est indiqué aux Tableaux I et II.

15. (1,3-1,4)-β-glucanase thermostable selon la revendication 14, comprenant une séquence d'acides aminés de formule
A - M
où A est un polypeptide constitué de 5 à 200 acides aminés présentant un degré d'homologie d'au moins 85% à un polypeptide comportant au total le même nombre de résidus d'acides aminés provenant de la région N-terminale de la (1,3-1,4)-β-glucanase de Bacillus amyloliquefaciens ou de Bacillus macerans tel qu'il est indiqué aux Tableaux I et II, et M est un polypeptide constitué de 5 à 200 acides aminés présentant un degré d'homologie d'au moins 85% à un polypeptide comportant au total le même nombre de résidus d'acides aminés provenant de la région C-terminale de la (1,3-1,4)-β-glucanase de Bacillus macerans ou de Bacillus amyloliquefaciens tel qu'il est indiqué aux Tableaux I et II.

16. (1,3-1,4)-β-glucanase thermostable selon la revendication 15, comprenant une séquence d'acides aminés de formule
A - M
où A est un polypeptide constitué de 5 à 200 acides aminés présentant un degré d'homologie d'au moins 90% à un polypeptide comportant au total le même nombre de résidus d'acides aminés provenant de la région N-terminale de la (1,3-1,4)-β-glucanase de Bacillus amyloliquefaciens ou de Bacillus macerans (1,3-1,4)- tel qu'il est indiqué aux Tableaux I et II, et M est un polypeptide constitué de 5 à 200 acides aminés présentant un degré d'homologie d'au moins 90% à un polypeptide comportant au total le même nombre de résidus d'acides aminés provenant de la région C-terminale de la (1,3-1,4)-β-glucanase de Bacillus macerans ou de Bacillus amyloliquefaciens tel qu'il est indiqué aux Tableaux I et II.

17. (1,3-1,4)-β-glucanase thermostable selon la revendication 16, comprenant une séquence d'acides aminés de formule
A - M
où A est un polypeptide constitué de 5 à 200 acides aminés identique à un polypeptide comportant au total le même nombre de résidus d'acides aminés provenant de la région N-terminale de la (1,3-1,4)-β-glucanase de Bacillus amyloliquefaciens ou de Bacillus macerans tel qu'il est indiqué aux Tableaux I et II, et M est un polypeptide constitué de 5 à 200 acides aminés identique à un polypeptide comportant au total le même nombre de résidus d'acides aminés de la région C-terminale de la (1,3-1,4)-β-glucanase de Bacillus macerans ou de Bacillus amyloliquefaciens tel qu'il est indiqué aux Tableaux I et II.

18. (1,3-1,4)-β-glucanase thermostable selon la revendication 14, où A est un polypeptide constitué de 16 acides aminés qui présentent un degré d'homologie d'au moins 75%, de préférence d'au moins 85%, et tout spécialement d'au moins 90% avec les résidus d'acides aminés de la région N-terminale de la (1,3-1,4)-β-glucanase de Bacillus amyloliquefaciens tel qu'il est indiqué au Tableau I.

19. (1,3-1,4)-β-glucanase thermostable selon la revendication 14, où A est un polypeptide constitué de 36 acides aminés qui présentent un degré d'homologie d'au moins 75%, de préférence d'au moins 85%, et tout spécialement d'au moins 90% avec les résidus d'acides aminés de la région N-terminale de la (1,3-1,4)-β-glucanase de Bacillus amyloliquefaciens tel qu'il est indiqué au Tableau I.

20. (1,3-1,4)-β-glucanase thermostable selon la revendication 14, où A est un polypeptide constitué de 78 acides aminés qui présentent un degré d'homologie d'au moins 75%, de préférence d'au moins 85%, et tout spécialement d'au moins 90% avec les résidus d'acides aminés de la région N-terminale de la (1,3-1,4)-β-glucanase de Bacillus amyloliquefaciens tel qu'il est indiqué au Tableau I.

21. (1,3-1,4)-β-glucanase thermostable selon la revendication 14, où A est un polypeptide constitué de 107 acides aminés qui présentent un degré d'homologie d'au moins 75%, de préférence d'au moins 85%, et tout spécialement d'au moins 90% avec les résidus d'acides aminés de la région N-terminale de la (1,3-1,4)-β-glucanase de Bacillus amyloliquefaciens tel qu'il est indiqué au Tableau I.

22. (1,3-1,4)-β-glucanase thermostable selon la revendication 14, où A est un polypeptide constitué de 152 acides aminés qui présentent un degré d'homologie d'au moins 75%, de préférence d'au moins 85%, et tout spécialement d'au moins 90% avec les résidus d'acides aminés de la région N-terminale de la (1,3-1,4)-β-glucanase de Bacillus amyloliquefaciens tel qu'il est indiqué aux Tableaux I et II.

23. (1,3-1,4)-β-glucanase thermostable selon l'une quelconque des revendications 1 à 22, qui possède un peptide signal lié à l'extrémité N-terminale de l'enzyme.

24. (1,3-1,4)-β-glucanase thermostable selon la revendication 23 qui possède un peptide signal dérivé d'une levure comme celle du genre Saccharomyces.

25. (1,3-1,4)-β-glucanase thermostable selon la revendication 24, dont le peptide signal est le peptide signal du facteur α de levure, de la phosphatase acide de levure ou de l'invertase de levure.

26. (1,3-1,4)-β-glucanase thermostable selon la revendication 23, qui possède un peptide signal présentant un degré d'homologie d'au moins 75%, de préférence d'au moins 85%, et tout spécialement d'au moins 90% au peptide signal de Bacillus amyloliquefaciens au niveau des acides aminés.

27. (1,3-1,4)-β-glucanase thermostable selon la revendication 1, qui comprend la séquence d'acides aminés suivante : 
Gln-Thr-Gly-Gly-Ser-Phe-Phe-Glu-Pro-Phe-Asn-Ser-Tyr-Asn-Ser-Gly-Leu-
Trp-Gln-Lys-Ala-Asp-Gly-Tyr-Ser-Asn-Gly-Asp-Met-Phe-Asn-Cys-Thr-Trp-
Arg-Ala-Asn-Asn-Val-Ser-Met-Thr-Ser-Leu-Gly-Glu-Met-Arg-Leu-Ala-Leu-
Thr-Ser-Pro-Ser-Tyr-Asn-Lys-Phe-Asp-Cys-Gly-Glu-Asn-Arg-Ser-Val-Gln-
Thr-Tyr-Gly-Tyr-Gly-Leu-Tyr-Glu-Val-Arg-Met-Lys-Pro-Ala-Lys-Asn-Thr-
Gly-Ile-Val-Ser-Ser-Phe-Phe-Thr-Tyr-Thr-Gly-Pro-Thr-Glu-Gly-Thr-Pro-
Trp-Asp-Glu-Ile-Asp-Ile-Glu-Phe-Leu-Gly-Lys-Asp-Thr-Thr-Lys-Val-Gln-
Phe-Asn-Tyr-Tyr-Thr-Asn-Gly-Val-Gly-Gly-His-Glu-Lys-Val-Ile-Ser-Leu-
Gly-Phe-Asp-Ala-Ser-Lys-Gly-Phe-His-Thr-Tyr-Ala-Phe-Asp-Trp-Gln-Pro-
Gly-Tyr-Ile-Lys-Trp-Tyr-Val-Asp-Gly-Val-Leu-Lys-His-Thr-Ala-Thr-Ala-
Asn-Ile-Pro-Ser-Thr-Pro-Gly-Lys-Ile-Met-Met-Asn-Leu-Trp-Asn-Gly-Thr-
Gly-Val-Asp-Asp-Trp-Leu-Gly-Ser-Tyr-Asn-Gly-Ala-Asn-Pro-Leu-Tyr-Ala-
Glu-Tyr-Asp-Trp-Val-Lys-Tyr-Thr-Ser-Asn
 ou les analogues de celle-ci.

28. (1,3-1,4)-β-glucanase thermostable selon la revendication 1, qui comprend la séquence d'acides aminés suivante : 
Met-Lys-Arg-Val-Leu-Leu-Ile-Leu-Val-Thr-Gly-Leu-Phe-Met-Ser-Leu-Cys-
Gly-Ile-Thr-Ser-Ser-Val-Ser-Ala-Gln-Thr-Gly-Gly-Ser-Phe-Phe-Glu-Pro-
Phe-Asn-Ser-Tyr-Asn-Ser-Gly-Leu-Trp-Gln-Lys-Ala-Asp-Gly-Tyr-Ser-Asn-
Gly-Asp-Met-Phe-Asn-Cys-Thr-Trp-Arg-Ala-Asn-Asn-Val-Ser-Met-Thr-Ser-
Leu-Gly-Glu-Met-Arg-Leu-Ala-Leu-Thr-Ser-Pro-Ser-Tyr-Asn-Lys-Phe-Asp-
Cys-Gly-Glu-Asn-Arg-Ser-Val-Gln-Thr-Tyr-Gly-Tyr-Gly-Leu-Tyr-Glu-Val-
Arg-Met-Lys-Pro-Ala-Lys-Asn-Thr-Gly-Ile-Val-Ser-Ser-Phe-Phe-Thr-Tyr-
Thr-Gly-Pro-Thr-Glu-Gly-Thr-Pro-Trp-Asp-Glu-Ile-Asp-Ile-Glu-Phe-Leu-
Gly-Lys-Asp-Thr-Thr-Lys-Val-Gln-Phe-Asn-Tyr-Tyr-Thr-Asn-Gly-Val-Gly-
Gly-His-Glu-Lys-Val-Ile-Ser-Leu-Gly-Phe-Asp-Ala-Ser-Lys-Gly-Phe-His-
Thr-Tyr-Ala-Phe-Asp-Trp-Gln-Pro-Gly-Tyr-Ile-Lys-Trp-Tyr-Val-Asp-Gly-
Val-Leu-Lys-His-Thr-Ala-Thr-Ala-Asn-Ile-Pro-Ser-Thr-Pro-Gly-Lys-Ile-
Met-Met-Asn-Leu-Trp-Asn-Gly-Thr-Gly-Val-Asp-Asp-Trp-Leu-Gly-Ser-Tyr-
Asn-Gly-Ala-Asn-Pro-Leu-Tyr-Ala-Glu-Tyr-Asp-Trp-Val-Lys-Tyr-Thr-Ser-
Asn
 ou les analogues de celle-ci.

29. Fragment d'ADN comprenant une séquence nucléotidique codant pour la (1,3-1,4)-β-glucanase thermostable telle que définie dans la revendication 17.

30. Fragment d'ADN selon la revendication 29, qui comprend la séquence nucléotidique suivante : 
30 60
GAATTCAACG AAGAATCGCT GCACTATTAT CGATTCGTCA CCCACTTAAA GTTTTTCGAC
90 120
CAGCGTCTTT TTAACGGCAC ACACATGGAA AGCCAGGACG ATTTTTTACT GGAGACAGTG
150 180
AAAGAAAAGT ATCATCAGGC GTATAAATGC ACGAAGAATA TCCATACCTA CATTGAGAAA
210 240
GAGTATGGGC ATAAGCTCAC CAGTGACGAG CTGCTGTATT TAACGATTCA CATAGAAAGG
270 300
GTAGTCAAAC AAGTATAATG AAAGCGCTTT CCTCGTATTA ATTGTTTCTT CCATTCATAT
330 360
ATACCATTGT TACGGATAAA GCAGGCAAAA CCTATCTGTC TGTGCTGATG GTAGTTTAGG
390 420
TTTGTATTTT TAACAGAAGG ATTATCATTA TTTCGACCGA TGTTCCCTTT GAAAAGGATC
450 480
ATGTATGATC AATAAAGAAA GCGTGTTCAA AAAAGGGGGA ATGCTAACAT GAAACGAGTG
510 540
TTGCTAATTC TTGTCACCGG ATTGTTTATG AGTTTGTGTG GGATCACTTC TAGTGTTTCG
570 600
GCTCAAACAG GCGGATCGTT TTTTGAACCT TTTAACAGCT ATAACTCCGG GTTATGGCAA
630 660
AAAGCTGATG GTTACTCAAA TGGAGATATG TTTAACTGCA CTTGGCGTGC TAATAACGTC
690 720
TCTATGACGT CATTAGGTGA AATGCGTTTG GCGCTGACAA GTCCGTCTTA TAACAAGTTT
750 780
GACTGCGGGG AAAACCGCTG GGTTCAAACA TATGGCTATG GACTTTATGA AGTCAGAATG
810 840
AAACCGGCTA AAAACACAGG GATTGTTTCA TCGTTCTTCA CTTATACAGG TCCAACGGAG
870 900
GGGACTCCTT GGGATGAGAT TGATATCGAA TTTCTAGGAA AAGACACGAC AAAAGTCCAG
930 960
TTTAACTATT ATACCAATGG GGTTGGCGGT CATGAAAAGG TTATCTCTCT TGGCTTTGAT
990 1020
GCATCAAAGG GCTTCCATAC CTATGCTTTC GATTGGCAGC CAGGGTATAT TAAATGGTAT
1050 1080
GTAGACGGTG TTTTGAAACA TACCGCCACC GCGAATATTC CGAGTACGCC AGGCAAAATT
1110 1140
ATGATGAATC TATGGAACGG AACCGGAGTG GATGACTGGT TAGGTTCTTA TAATGGAGCG
1170 1200
AATCCGTTGT ACGCTGAATA TGACTGGGTA AAATATACGA GCAATTAATA TGATTGCAGC
1230
TGGGCATGAG CTTTTTAGTC CACTCCAGGC ATGCAAGCTT
 ou un analogue ou une sous-séquence de celle-ci.

31. Procédé de production d'une (1,3-1,4)-β-glucanase thermostable selon la revendication 1, comprenant la mise en culture d'un micro-organisme dans lequel un fragment d'ADN tel que défini dans la revendication 29 ou 30 a été introduit de manière à ce que le micro-organisme puisse exprimer la (1,3-1,4)-β-glucanase thermostable, la mise en culture s'effectuant dans des conditions conduisant à la production de (1,3-1,4)-β-glucanase thermostable et l'isolement de la (1,3-1,4)-β-glucanase de la culture.

32. Procédé selon la revendication 31, dans lequel le micro-organisme est une bactérie.

33. Procédé selon la revendication 32, dans lequel la bactérie est une bactérie gram négative.

34. Procédé selon la revendication 33, dans lequel la bactérie gram négative est une souche d'E.coli.

35. Procédé selon la revendication 34, dans lequel la souche d'E. coli. est la souche d'E. coli hébergeant le plasmide pUC13-H1, déposée à Deutsche Sammlung von Mikroorganismen sous la référence N° DSM 5461.

36. Procédé selon la revendication 34, dans lequel la souche d'E. coli. est la souche d'E. coli hébergeant le plasmide pTZ19R-H3, déposée à Deutsche Sammlung von Mikroorganismen sous la référence N° DSM 5790.

37. Procédé selon la revendication 34, dans lequel la souche d'E. coli. est la souche d'E. coli hébergeant le plasmide pTZ19R-H4, déposée à Deutsche Sammlung von Mikroorganismen sous la référence N° DSM 5791.

38. Procédé selon la revendication 34, dans lequel la souche d'E. coli. est la souche d'E. coli hébergeant le plasmide pTZ19R-H5, déposée à Deutsche Sammlung von Mikroorganismen sous la référence N° DSM 5792.

39. Procédé selon la revendication 34, dans lequel la souche d'E. coli. est la souche d'E. coli hébergeant le plasmide pTZ19R-H6, déposée à Deutsche Sammlung von Mikroorganismen sous la référence N' DSM 5793.

40. Procédé selon la revendication 31, dans lequel le micro-organisme est une levure.

41. Procédé selon la revendication 40, dans lequel la levure est du genre Saccharomyces

42. Procédé selon la revendication 41, dans lequel l'espèce du genre Saccharomyces est Saccharomyces cerevisiae.

43. Organisme eucaryote ou procaryote qui est transformé avec et est capable d'exprimer le fragment d'ADN tel que défini dans la revendication 29 ou 30.

44. Organisme selon la revendication 43, qui est un micro-organisme.

45. Micro-organisme selon la revendication 44, qui est une bactérie.

46. Bactérie selon la revendication 45, qui est une bactérie gram négative.

47. Bactérie selon la revendication 46, qui est une souche d'E. coli.

48. Souche d'E. coli hébérgeant le plasmide pUC13-H1 ayant fait l'objet de dépôt à Deutsche Sammlung von Mikroorganismen sous la référence DSM 5461 ainsi que les mutants et les variants de celle-ci.

49. Souche d'E. coli hébergeant le plasmide pTE19R-H3 ayant fait l'objet de dépôt à Deutsche Sammlung von Mikroorganismen sous la référence DSM 5790 ainsi que les mutants et les variants de celle-ci.

50. Souche d'E. coli hébérgeant le plasmide pTZ19R-H4 ayant fait l'objet de dépôt à Deutsche Sammlung von Mikroorganismen sous la référence DSM 5791 ainsi que les mutants et les variants de celle-ci.

51. Souche d'E. coli hébérgeant le plasmide pTZ19R-H5 ayant fait l'objet de dépôt à Deutsche Sammlung von Mikroorganismen sous la référence DSM 5792 ainsi que les mutants et les variants de celle-ci.

52. Souche d'E. coli hébergeant le plasmide pTZ19R-H6 ayant fait l'objet de dépôt à Deutsche Sammlung von Mikroorganismen sous la référence DSM 5793 ainsi que les mutants et les variants de celle-ci.

53. Micro-organisme selon la revendication 44, qui est une levure.

54. Levure selon la revendication 53, qui est une espèce du genre Saccharomyces.

55. Espèce du genre Saccharomyces selon la revendication 54, qui est Saccharomyces cerevisiae.

56. Organisme selon la revendication 43, qui est une plante.

57. Plante selon la revendication 56, qui est l'avoine, l'orge, le seigle, le blé, le riz ou le maïs.

58. Procédé de dégradation des (1,3-1,4)-β-glucanes contenus dans un substrat comprenant la soumission du substrat à l'action d'une quantité efficace d'une (1,3-1,4)-β-glucanase thermostable telle que définie dans l'une quelconque des revendications 1 à 22 pour une durée donnée à une température supérieure ou égale à 65°C, la quantité de (1,3-1,4)-β-glucanase étant d'au plus 200 µg, de préférence d'au plus 100 µg, mieux encore d'au plus 50 µg, en particulier d'au plus 20 µg, et tout spécialement de 15 µg par kg de substrat.

59. Procédé selon la revendication 58, dans lequel le substrat comprend des céréales non modifiées ou des fragments de celles-ci issues d'orge ou d'avoine ou d'autres céréales.

60. Procédé selon la revendication 58 ou 59, dans lequel le substrat comprenant des (1,3-1,4)-β-glucanes est mélangé à un deuxième substrat issu du maïs, du riz ou du blé comprenant une (1,3-1,4)-β-glucanase thermostable telle que définie dans la revendication 1.

61. Procédé de production de bière, caractérisé en que le moût est soumis à un traitement par une (1,3-1,4)-β-glucanase thermostable selon l'une quelconque des revendications 1 à 22 lors du trempage à une température supérieure ou égale à 65°C et à pH compris entre 4 et 5,5.

62. Procédé selon la revendication 61, dans lequel la température est supérieure ou égale à 70°C.

63. Procédé de production de fourrage comprenant des β-glucanes, caractérisé en ce que le fourrage est supplémenté en une (1,3-1,4)-β-glucanase thermostable selon la revendication 1 en quantité suffisante pour obtenir une dégradation significative des β-glucanes dans le tube gastro-intestinal d'un animal alimenté en fourrage supplémenté en (1,3-1,4)-β-glucanase.

64. Procédé selon la revendication 63, dans lequel le fourrage est en granulés.

## Revendications (Revendications pour l'(les) Etat(s) contractant(s) suivant(s): ES)

1. Procédé de production d'une (1,3-1,4)-β-glucanase thermostable comprenant une séquence d'acides aminés de formule
A - M
où A est un polypeptide constitué de 5 à 200 acides aminés présentant un degré d'homologie d'au moins 75% à un polypeptide comportant au total le même nombre de résidus d'acides aminés provenant de la région N-terminale de la (1,3-1,4)-β-glucanase de Bacillus amyloliquefaciens ou de Bacillus macerans tel qu'il est indiqué aux Tableaux I et II, et M est un polypeptide constitué de 5 à 200 acides aminés présentant un degré d'homologie d'au moins 75% à un polypeptide comportant au total le même nombre de résidus d'acides aminés provenant de la région C-terminale de la (1,3-1,4)-β-glucanase de Bacillus macerans ou de Bacillus amyloliquefaciens tel qu'il est indiqué aux Tableaux I et II.

2. Procédé selon la revendication 1, dans lequel la (1,3-1,4)-β-glucanase thermostable comprend une séquence d'acides aminés de formule
A - M
où A est un polypeptide constitué de 5 à 200 acides aminés présentant un degré d'homologie d'au moins 85% à un polypeptide comportant au total le même nombre de résidus d'acides aminés provenant de la région N-terminale de la (1,3-1,4)-β-glucanase de Bacillus amyloliquefaciens ou de Bacillus macerans tel qu'il est indiqué aux Tableaux I et II, et M est un polypeptide constitué de 5 à 200 acides aminés présentant un degré d'homologie d'au moins 85% à un polypeptide comportant au total le même nombre de résidus d'acides aminés provenant de la région C-terminale de la (1,3-1,4)-β-glucanase de Bacillus macerans ou de Bacillus amyloliquefaciens tel qu'il est indiqué aux Tableaux I et II.

3. Procédé selon la revendication 2, dans lequel la (1,3-1,4)-β-glucanase thermostable comprend une séquence d'acides aminés de formule
A - M
où A est un polypeptide constitué de 5 à 200 acides aminés présentant un degré d'homologie d'au moins 90% à un polypeptide comportant au total le même nombre de résidus d'acides aminés provenant de la région N-terminale de la (1,3-1,4)-β-glucanase de Bacillus amyloliquefaciens ou de Bacillus macerans (1,3-1,4)- tel qu'il est indiqué aux Tableaux I et II, et M est un polypeptide constitué de 5 à 200 acides aminés présentant un degré d'homologie d'au moins 90% à un polypeptide comportant au total le même nombre de résidus d'acides aminés provenant de la région C-terminale de la (1,3-1,4)-β-glucanase de Bacillus macerans ou de Bacillus amyloliquefaciens tel qu'il est indiqué aux Tableaux I et II.

4. Procédé selon la revendication 3, dans lequel la (1,3-1,4)-β-glucanase thermostable comprend une séquence d'acides aminés de formule
A - M
où A est un polypeptide constitué de 5 à 200 acides aminés parfaitement homologue à un polypeptide comportant au total le même nombre de résidus d'acides aminés provenant de la région N-terminale de la (1,3-1,4)-β-glucanase de Bacillus amyloliquefaciens ou de Bacillus macerans tel qu'il est indiqué aux Tableaux I et II, et M est un polypeptide constitué de 5 à 200 acides aminés parfaitement homologue à un polypeptide comportant au total le même nombre de résidus d'acides aminés de la région C-terminale de la (1,3-1,4)-β-glucanase de Bacillus macerans ou de Bacillus amyloliquefaciens tel qu'il est indiqué aux Tableaux I et II.

5. Procédé selon la revendication 1, où A est un polypeptide constitué de 16 acides aminés qui présentent un degré d'homologie d'au moins 75%, de préférence d'au moins 85%, et tout spécialement d'au moins 90% avec les résidus d'acides aminés de la région N-terminale de la (1,3-1,4)-β-glucanase de Bacillus amyloliquefaciens tel qu'il est indiqué au Tableau I.

6. Procédé selon la revendication 1, où A est un polypeptide constitué de 36 acides aminés qui présentent un degré d'homologie d'au moins 75%, de préférence d'au moins 85%, et tout spécialement d'au moins 90% avec les résidus d'acides aminés de la région N-terminale de la (1,3-1,4)-β-glucanase de Bacillus amyloliquefaciens tel qu'il est indiqué au Tableau I.

7. Procédé selon la revendication 1, où A est un polypeptide constitué de 78 acides aminés qui présentent un degré d'homologie d'au moins 75%, de préférence d'au moins 85%, et tout spécialement d'au moins 90% avec les résidus d'acides aminés de la région N-terminale de la (1,3-1,4)-β-glucanase de Bacillus amyloliquefaciens tel qu'il est indiqué au Tableau I.

8. Procédé selon la revendication 1, où A est un polypeptide constitué de 107 acides aminés qui présentent un degré d'homologie d'au moins 75%, de préférence d'au moins 85%, et tout spécialement d'au moins 90% avec les résidus d'acides aminés de la région N-terminale de la (1,3-1,4)-β-glucanase de Bacillus amyloliquefaciens tel qu'il est indiqué au Tableau I.

9. Procédé selon la revendication 1, où A est un polypeptide constitué de 152 acides aminés qui présentent un degré d'homologie d'au moins 75%, de préférence d'au moins 85%, et tout spécialement d'au moins 90% avec les résidus d'acides aminés de la région N-terminale de (1,3-1,4)-β-glucanase de Bacillus amyloliquefaciens tel qu'il est indiqué aux Tableaux I et II.

10. Procédé de production d'une (1,3-1,4)-β-glucanase hybride thermostable selon l'une quelconque des revendications 1 à 9, codée par un gène hybride construit par échange réciproque des régions amino-terminales et carboxy-terminales des gènes de la (1,3-1,4)-β-glucanase du genre Bacillus, laquelle glucanase conserve au moins 50% de son activité au bout de 10 minutes d'incubation ou plus dans du CaCl₂ 5-10 mM, acétate de Na 20-40 mM à pH inférieur ou égal à 6,0 et à température supérieure ou égale à 65°C, la concentration en enzyme de la solution mise en incubation étant comprise entre 0,05 mg et 1 mg de (1,3-1,4)-β-glucanase par ml, l'activité de la (1,3-1,4)-β-glucanase étant assimilée à la capacité de l'enzyme à hydrolyser les liaisons β-glycosidiques des (1,3-1,4)-β-glucanes, la (1,3-1,4)-β-glucanase hybride étant plus thermostable qu'une quelconque glucanase encodée par les gènes de la (1,3-1,4)-β-glucanase parentaux du genre Bacillus.

11. Procédé selon la revendication 10, dans lequel la durée d'incubation est de 15 minutes.

12. Procédé selon la revendication 10, dans lequel la durée d'incubation est de 18 minutes.

13. Procédé selon l'une quelconque des revendications 10 à 12 , dans lequel la température d'incubation est de 70°C.

14. Procédé selon l'une quelconque des revendications 10 à 13, dans lequel la concentration de la solution s'échelonne de 0,3 mg à 1 mg de (1,3-1,4)-β-glucanase par ml.

15. Procédé selon la revendication 10, dans lequel la (1,3-1,4)-β-glucanase thermostable conserve au moins 85% de son activité au bout de 30 minutes d'incubation ou plus.

16. Procédé selon la revendication 15, dans lequel la durée d'incubation est de 60 minutes.

17. Procédé selon la revendication 15, dans lequel la (1,3-1,4)-β-glucanase thermostable conserve au moins 85% de son activité au bout de 30 minutes d'incubation ou plus dans du CaCl₂ 5-10 mM, acétate de Na 20-40 mM à un pH de 4,1 et à une température de 65°C, la concentration en enzyme de la solution mise en incubation étant de 0,1 mg de (1,3-1,4)-β-glucanase par ml.

18. Procédé selon la revendication 15, dans lequel la (1,3-1,4)-β-glucanase thermostable conserve au moins 85% de son activité au bout de 30 minutes d'incubation ou plus dans du CaCl₂ 5-10 mM, acétate de Na 20-40 mM à un *p*H de 6,0 et à une température de 70°C, la concentration en enzyme de la solution mise en incubation étant de 0,1 mg de (1,3-1,4)-β-glucanase par ml.

19. Procédé selon la revendication 17 ou 18, dans lequel la durée d'incubation est de 60 minutes.

20. Procédé selon l'une quelconque des revendications 17 à 19, dans lequel la (1,3-1,4)-β-glucanase thermostable conserve au moins 90% de son activité.

21. Procédé selon l'une quelconque des revendications 10 à 20, dans lequel la (1,3-1,4)-β-glucanase thermostable présente une activité β-glucanase relative d'au moins 100% au bout de 10 minutes d'incubation à 65°C et à pH 6,0 dans des extraits cellulaires bruts.

22. Procédé selon la revendication 21, dans lequel la (1,3-1,4)-β-glucanase thermostable présente une activité β-glucanase relative de 130%.

23. Procédé selon l'une quelconque des revendications 1 à 22, dans lequel la (1,3-1,4)-β-glucanase thermostable possède un peptide signal lié à l'extrémité N-terminale de l'enzyme.

24. Procédé selon la revendication 23, dans lequel le peptide signal dérive d'une levure comme celle du genre Saccharomyces.

25. Procédé selon la revendication 24, dans lequel le peptide signal est le peptide signal du facteur α de levure, de la phosphatase acide de levure ou de l'invertase de levure.

26. Procédé selon la revendication 24, dans lequel le peptide signal présente un degré d'homologie d'au moins 75%, de préférence d'au moins 85%, et tout spécialement d'au moins 90% au peptide signal de Bacillus amyloliquefaciens au niveau des acides aminés.

27. Procédé selon la revendication 10, dans lequel la (1,3-1,4)-β-glucanase thermostable comprend la séquence d'acides aminés suivante: 
Gln-Thr-Gly-Gly-Ser-Phe-Phe-Glu-Pro-Phe-Asn-Ser-Tyr-Asn-Ser-Gly-Leu-
Trp-Gln-Lys-Ala-Asp-Gly-Tyr-Ser-Asn-Gly-Asp-Met-Phe-Asn-Cys-Thr-Trp-
Arg-Ala-Asn-Asn-Val-Ser-Met-Thr-Ser-Leu-Gly-Glu-Met-Arg-Leu-Ala-Leu-
Thr-Ser-Pro-Ser-Tyr-Asn-Lys-Phe-Asp-Cys-Gly-Glu-Asn-Arg-Ser-Val-Gln-
Thr-Tyr-Gly-Tyr-Gly-Leu-Tyr-Glu-Val-Arg-Met-Lys-Pro-Ala-Lys-Asn-Thr-
Gly-Ile-Val-Ser-Ser-Phe-Phe-Thr-Tyr-Thr-Gly-Pro-Thr-Glu-Gly-Thr-Pro-
Trp-Asp-Glu-Ile-Asp-Ile-Glu-Phe-Leu-Gly-Lys-Asp-Thr-Thr-Lys-Val-Gln-
Phe-Asn-Tyr-Tyr-Thr-Asn-Gly-Val-Gly-Gly-His-Glu-Lys-Val-Ile-Ser-Leu-
Gly-Phe-Asp-Ala-Ser-Lys-Gly-Phe-His-Thr-Tyr-Ala-Phe-Asp-Trp-Gln-Pro-
Gly-Tyr-Ile-Lys-Trp-Tyr-Val-Asp-Gly-Val-Leu-Lys-His-Thr-Ala-Thr-Ala-
Asn-Ile-Pro-Ser-Thr-Pro-Gly-Lys-Ile-Met-Met-Asn-Leu-Trp-Asn-Gly-Thr-
Gly-Val-Asp-Asp-Trp-Leu-Gly-Ser-Tyr-Asn-Gly-Ala-Asn-Pro-Leu-Tyr-Ala-
Glu-Tyr-Asp-Trp-Val-Lys-Tyr-Thr-Ser-Asn
 ou les analogues de celle-ci.

28. Procédé selon la revendication 10, dans lequel la (1,3-1,4)-β-glucanase thermostable comprend la séquence d'acides aminés suivante: 
Met-Lys-Arg-Val-Leu-Leu-Ile-Leu-Val-Thr-Gly-Leu-Phe-Met-Ser-Leu-Cys-
Gly-Ile-Thr-Ser-Ser-Val-Ser-Ala-Gln-Thr-Gly-Gly-Ser-Phe-Phe-Glu-Pro-
Phe-Asn-Ser-Tyr-Asn-Ser-Gly-Leu-Trp-Gln-Lys-Ala-Asp-Gly-Tyr-Ser-Asn-
Gly-Asp-Met-Phe-Asn-Cys-Thr-Trp-Arg-Ala-Asn-Asn-Val-Ser-Met-Thr-Ser-
Leu-Gly-Glu-Met-Arg-Leu-Ala-Leu-Thr-Ser-Pro-Ser-Tyr-Asn-Lys-Phe-Asp-
Cys-Gly-Glu-Asn-Arg-Ser-Val-Gln-Thr-Tyr-Gly-Tyr-Gly-Leu-Tyr-Glu-Val-
Arg-Met-Lys-Pro-Ala-Lys-Asn-Thr-Gly-Ile-Val-Ser-Ser-Phe-Phe-Thr-Tyr-
Thr-Gly-Pro-Thr-Glu-Gly-Thr-Pro-Trp-Asp-Glu-Ile-Asp-Ile-Glu-Phe-Leu-
Gly-Lys-Asp-Thr-Thr-Lys-Val-Gln-Phe-Asn-Tyr-Tyr-Thr-Asn-Gly-Val-Gly-
Gly-His-Glu-Lys-Val-Ile-Ser-Leu-Gly-Phe-Asp-Ala-Ser-Lys-Gly-Phe-His-
Thr-Tyr-Ala-Phe-Asp-Trp-Gln-Pro-Gly-Tyr-Ile-Lys-Trp-Tyr-Val-Asp-Gly-
Val-Leu-Lys-His-Thr-Ala-Thr-Ala-Asn-Ile-Pro-Ser-Thr-Pro-Gly-Lys-Ile-
Met-Met-Asn-Leu-Trp-Asn-Gly-Thr-Gly-Val-Asp-Asp-Trp-Leu-Gly-Ser-Tyr-
Asn-Gly-Ala-Asn-Pro-Leu-Tyr-Ala-Glu-Tyr-Asp-Trp-Val-Lys-Tyr-Thr-Ser-
Asn
 ou les analogues de celle-ci.

29. Procédé de préparation d'une (1,3-1,4)-β-glucanase thermostable telle que définie dans la revendication 4 au moyen d'un fragment d'ADN comprenant une séquence nucléotidique codant pour la (1,3-1,4)-β-glucanase thermostable.

30. Procédé selon la revendication 29, dans lequel le fragment d'ADN comprend la séquence nucléotidique suivante : 
30 60
GAATTCAACG AAGAATCGCT GCACTATTAT CGATTCGTCA CCCACTTAAA GTTTTTCGAC
90 120
CAGCGTCTTT TTAACGGCAC ACACATGGAA AGCCAGGACG ATTTTTTACT GGAGACAGTG
150 180
AAAGAAAAGT ATCATCAGGC GTATAAATGC ACGAAGAATA TCCATACCTA CATTGAGAAA
210 240
GAGTATGGGC ATAAGCTCAC CAGTGACGAG CTGCTGTATT TAACGATTCA CATAGAAAGG
270 300
ATAGGATTGT TACGGATAAA GCAGGCAAAA GTAGTCAAAC AAGTATAATG AAAGCGCTTT
330 360
CCTCGTATTA ATTGTTTCTT CCATTCATAT CCTATCTGTC TGTGCTGATG GTAGTTTAGG
390 420
TTTGTATTTT TAACAGAAGG ATTATCATTA TTTCGACCGA TGTTCCCTTT GAAAAGGATC
450 480
ATGTATGATC AATAAAGAAA GCGTGTTCAA AAAAGGGGGA ATGCTAACAT GAAACGAGTG
510 540
TTGCTAATTC TTGTCACCGG ATTGTTTATG AGTTTGTGTG GGATCACTTC TAGTGTTTCG
570 600
GCTCAAACAG GCGGATCGTT TTTTGAACCT TTTAACAGCT ATAACTCCGG GTTATGGCAA
630 660
AAAGCTGATG GTTACTCAAA TGGAGATATG TTTAACTGCA CTTGGCGTGC TAATAACGTC
690 720
TCTATGACGT CATTAGGTGA AATGCGTTTG GCGCTGACAA GTCCGTCTTA TAACAAGTTT
750 780
GACTGCGGGG AAAACCGCTG GGTTCAAACA TATGGCTATG GACTTTATGA AGTCAGAATG
810 840
AAACCGGCTA AAAACACAGG GATTGTTTCA TCGTTCTTCA CTTATACAGG TCCAACGGAG
870 900
GGGACTCCTT GGGATGAGAT TGATATCGAA TTTCTAGGAA AAGACACGAC AAAAGTCCAG
930 960
TTTAACTATT ATACCAATGG GGTTGGCGGT CATGAAAAGG TTATCTCTCT TGGCTTTGAT
990 1020
GCATCAAAGG GCTTCCATAC CTATGCTTTC GATTGGCAGC CAGGGTATAT TAAATGGTAT
1050 1080
GTAGACGGTG TTTTGAAACA TACCGCCACC GCGAATATTC CGAGTACGCC AGGCAAAATT
1110 1140
ATGATGAATC TATGGAACGG AACCGGAGTG GATGACTGGT TAGGTTCTTA TAATGGAGCG
1170 1200
AATCCGTTGT ACGCTGAATA TGACTGGGTA AAATATACGA GCAATTAATA TGATTGCAGC
1230
TGGGCATGAG CTTTTTAGTC CACTCCAGGC ATGCAAGCTT
 ou un analogue ou une sous-séquence de celle-ci.

31. Procédé de production d'une (1,3-1,4)-β-glucanase thermostable selon la revendication 10, comprenant la mise en culture d'un micro-organisme dans lequel un fragment d'ADN tel que défini dans la revendication 29 ou 30 a été introduit de manière à ce que le micro-organisme puisse exprimer la (1,3-1,4)-β-glucanase thermostable, la mise en culture s'effectuant dans des conditions conduisant à la production de (1,3-1,4)-β-glucanase thermostable et l'isolement de la (1,3-1,4)-β-glucanase de la culture.

32. Procédé selon la revendication 31, dans lequel le micro-organisme est une bactérie.

33. Procédé selon la revendication 32, dans lequel la bactérie est une bactérie gram négative.

34. Procédé selon la revendication 33, dans lequel la bactérie gram négative est une souche d'E. coli.

35. Procédé selon la revendication 34, dans lequel la souche d'E. coli est la souche d'E. coli hébergeant le plasmide pUC13-H1, déposée à Deutsche sammlung von Mikroorganismen sous la référence N' DSM 5461.

36. Procédé selon la revendication 34, dans lequel la souche d'E. coli est la souche d'E. coli hébergeant le plasmide pTZ19R-H3, déposée à Deutsche Sammlung von Mikroorganismen sous la référence N° DSM 5790.

37. Procédé selon la revendication 34, dans lequel la souche d'E. coli est la souche d'E. coli hébergeant le plasmide pTZ19R-H4, déposée à Deutsche Sammlung von Mikroorganismen sous la référence N° DSM 5791.

38. Procédé selon la revendication 34, dans lequel la souche d'E. coli est la souche d'E. coli hébergeant le plasmide pTZ19R-H5, déposée à Deutsche Sammlung von Mikroorganismen sous la référence N° DSM 5792.

39. Procédé selon la revendication 34, dans lequel la souche d'E. coli est la souche d'E. coli hébergeant le plasmide pTZ19R-H6, déposée à Deutsche Sammlung von Mikroorganismen sous la référence N° DSM 5793.

40. Procédé selon la revendication 31, dans lequel le micro-organisme est une levure.

41. Procédé selon la revendication 40, dans lequel la levure est du genre Saccharomyces.

42. Procédé selon la revendication 41, dans lequel l'espèce du genre Saccharomyces est Saccharomyces cerevisiae.

43. Procédé de production d'un organisme eucaryote ou procaryote capable d'exprimer le fragment d'ADN tel que décrit dans la revendication 29 ou 30, procédé comprenant la transformation dudit organisme avec le fragment d'ADN.

44. Procédé selon la revendication 43, dans lequel l'organisme est un micro-organisme.

45. Procédé selon la revendication 44, dans lequel le micro-organisme est une bactérie.

46. Procédé selon la revendication 45, dans lequel la bactérie est une bactérie gram négative.

47. Procédé selon la revendication 46, dans lequel la bactérie est une souche d'E. coli.

48. Procédé selon la revendication 47, dans lequel la souche d'E. coli héberge le plasmide pUC13-H1 ayant fait l'objet de dépôt à Deutsche Sammlung von Mikroorganismen sous la référence DSM 5461 ainsi que les mutants et les variants de celle-ci.

49. Procédé selon la revendication 47, dans lequel la souche d'E. coli héberge le plasmide pTZ19R-H3 ayant fait l'objet de dépôt à Deutsche Sammlung von Mikroorganismen sous la référence DSM 5790 ainsi que les mutants et les variants de celle-ci.

50. Procédé selon la revendication 47, dans lequel la souche d'E. coli héberge le plasmide pTZ19R-H4 ayant fait l'objet de dépôt à Deutsche Sammlung von Mikroorganismen sous la référence DSM 5791 ainsi que les mutants et les variants de celle-ci.

51. Procédé selon la revendication 47, dans lequel la souche d'E. coli héberge le plasmide pTZ19R-H5 ayant fait l'objet de dépôt à Deutsche Sammlung von Mikroorganismen sous la référence DSM 5792 ainsi que les mutants et les variants de celle-ci.

52. Procédé selon la revendication 47, dans lequel la souche d'E. coli héberge le plasmide pTZ19R-H6 ayant fait l'objet de dépôt à Deutsche Sammlung von Mikroorganismen sous la référence DSM 5793 ainsi que les mutants et les variants de celle-ci.

53. Procédé selon la revendication 44, dans lequel le micro-organisme est une levure.

54. Procédé selon la revendication 47, dans lequel la levure est une espèce du genre Saccharomyces.

55. Procédé selon la revendication 54, dans lequel l'espèce du genre Saccharomyces est Saccharomyces cerevisiae.

56. Procédé selon la revendication 43, dans lequel l'organisme est une plante.

57. Procédé selon la revendication 56, dans lequel la plante est l'avoine, l'orge, le seigle, le blé, le riz ou le maïs.

58. Procédé de dégradation des (1,3-1,4)-β-glucanes contenus dans un substrat comprenant la soumission du substrat à l'action d'une quantité efficace d'une (1,3-1,4)-β-glucanase thermostable telle que définie dans l'une quelconque des revendications 1 à 22 pour une durée donnée à une température supérieure ou égale à 65'C, la quantité de (1,3-1,4)-β-glucanase étant d'au plus 200 µg, de préférence d'au plus 100 µg, mieux encore d'au plus 50 µg, en particulier d'au plus 20 µg, et tout spécialement de 15 µg par kg de substrat.

59. Procédé selon la revendication 58, dans lequel le substrat comprend des céréales brutes non modifiées ou des fragments de celles-ci issues d'orge ou d'avoine ou d'autres céréales.

60. Procédé selon la revendication 58 ou 59, dans lequel le substrat comprenant des (1,3-1,4)-β-glucanes est mélangé à un deuxième substrat issu du maïs, du riz ou du blé comprenant une (1,3-1,4)-β-glucanase thermostable telle que définie dans la revendication 1.

61. Procédé de production de bière, caractérisé en que le moût est soumis à un traitement par une (1,3-1,4)-β-glucanase thermostable selon l'une quelconque des revendications 1 à 22 lors du trempage à une température supérieure ou égale à 65'C et à pH compris entre 4 et 5,5.

62. Procédé selon la revendication 61, dans lequel la température est supérieure ou égale à 70°C.

63. Procédé de production de fourrage comprenant des β-glucanes, caractérisé en ce que le fourrage est supplémenté en une (1,3-1,4)-β-glucanase thermostable selon la revendication 1 en quantité suffisante pour obtenir une dégradation significative des β-glucanes dans le tube gastro-intestinal d'un animal alimenté en fourrage supplémenté en (1,3-1,4)-β-glucanase.

64. Procédé selon la revendication 63, dans lequel le fourrage est en granulés.
